(19) Europäisches Patentamt
European Patent Office
Office européen des brevets

(11) EP 4 385 976 A1

(12) **EUROPEAN PATENT APPLICATION**
published in accordance with Art. 153(4) EPC

(43) Date of publication:
**19.06.2024 Bulletin 2024/25**

(21) Application number: **22854947.3**

(22) Date of filing: **24.04.2022**

(51) International Patent Classification (IPC):
***C07D 209/48*** *(2006.01)* ***C07D 209/49*** *(2006.01)*
***A61P 11/06*** *(2006.01)* ***A61P 29/00*** *(2006.01)*
***A61P 11/00*** *(2006.01)* ***A61P 17/00*** *(2006.01)*
***A61P 17/08*** *(2006.01)* ***A61P 17/06*** *(2006.01)*
***A61P 19/02*** *(2006.01)* ***A61P 1/00*** *(2006.01)*
***A61K 31/4035*** *(2006.01)*

(86) International application number:
**PCT/CN2022/088776**

(87) International publication number:
**WO 2023/015944 (16.02.2023 Gazette 2023/07)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **13.08.2021 CN 202110932229**

(71) Applicant: **Suzhou Intragrand Pharma Co., Ltd**
**Suzhou, Jiangsu 215000 (CN)**

(72) Inventors:
- **ZHU, Jiawang**
  **Suzhou, Jiangsu 215000 (CN)**
- **YAO, Yao**
  **Suzhou, Jiangsu 215000 (CN)**

(74) Representative: **Zaboliene, Reda**
**Metida**
**Business center Vertas**
**Gyneju str. 16**
**01109 Vilnius (LT)**

# (54) SUBSTITUTED ISOINDOLIN-1,3-DIONE PDE4 INHIBITOR AND PHARMACEUTICAL USE THEREOF

(57) The present disclosure relates to a compound as shown in formula I and its racemates, stereoisomers, tautomers, isotopic labels, solvates, polymorphs, esters, prodrugs or pharmaceutically acceptable salts as well as a pharmaceutical composition containing same, and preparation methods and medical uses thereof. The structure of formula I is as follows:

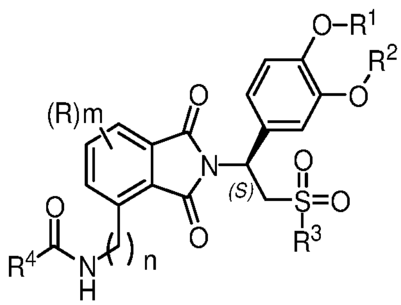

Formula I

EP 4 385 976 A1

## Description

### Technical Field

The present disclosure belongs to the field of medicine, and in particular relates to a substituted isoindoline-1, 3-diketone PDE4 inhibitor as well as a preparation method and pharmaceutical application thereof.

### Background

Cyclic adenosine monophosphate (cAMP) as a second messenger plays a fairly significant role in biological processes. Studies have found that the deficiency or inactivation of the cyclic adenosine monophosphate has contributed to diseases such as asthma, pulmonary obstructive disease, and inflammation (Lowe and Cheng, Drugs of the Future, 17(9): 799-807, 1992), while an increase in cAMP level of inflammatory leukocytes inhibits the release of inflammatory mediators including TNF-$\alpha$ and NF-$\kappa$B; and furthermore, the increase in the cAMP level will also lead to the relaxation of airway smooth muscle (ASM).

The main biological mechanism for inactivation of cyclic adenosine monophosphate is due to the destruction of cAMP level by cyclic nucleotide phosphodiesterase (PDE) family (Beavo and Reitsnyder, Trends in Pharm., 11: 150-155, 1990). It is known that there are currently 11 family enzymes of PDE members, and inhibition of type 4 PDE (type IV PDE) has a significant effect on the promotion of cAMP and the release of inflammatory mediators (Verghes et al., Journal of Pharmacology and Experimental Therapeutics, 272(3): 1313-1320, 1995). Thus, the organic compounds that selectively inhibiting of PDE4 has the potential to inhibit airway inflammation, promote the relaxation of ASM, and treat skin inflammation.

Inhibition of the PDE4 enzyme may block the activity or production of certain cytokines including alpha-tumor necrosis factor (TNF-$\alpha$). Alpha-tumor necrosis factor is a cytokine that is released primarily by mononuclear phagocytes in response to immune stimuli. TNF-$\alpha$ is capable of promoting most cellular processes such as differentiation, recruitment, proliferation and protein degradation. At low levels, TNF-$\alpha$ has a protective effect against infectious agents, tumors and tissue damage, but TNF-$\alpha$ plays an inducing and exacerbating role in many diseases. When administered to mammals or humans, TNF-$\alpha$ causes or exacerbates inflammation, fever, cardiovascular effects, hemorrhage, and acute reactions similar to those occurring during acute infection and shock.

Arthritis, arthritic conditions (such as osteoarthritis and rheumatoid arthritis), enteritis (such as segmental ileitis and ulcerative colitis), sepsis, psoriasis, atopic dermatitis (AD), contact dermatitis and chronic obstructive pulmonary disease (COPD), chronic pneumonia, acute respiratory distress syndrome (ARDS), vitiligo, prurigo nodularis, vulvodynia, fibrotic disease, cachexia, autoimmune disease, ankylosing spondylitis, osteoporosis, segmental ileitis, ulcerative colitis, enteritis, multiple sclerosis (MS), discoid lupus erythematosus, systemic lupus erythematosus, radiation injury, hyperoxia alveolar damage (Tracey et al., 1987, Nature, 330: 662-664 and Hinshaw et al., 1990, Circle. Shock, 30: 279-292 (endotoxin shock); Millar et al., 1989, Lancet, 2: 712-714 and Ferrai-Baliviera et al., 1989, Arch Surg., 124: 1400-1405 (Adult Respiratory Distress Syndrome); Bertolini et al., 1986, Nature, 319: 516-518; Pignet et al., 1990, Nature, 344: 245-247, Bissonnette et al., 1989, Inflammation, 13: 329-339, and Baughman et al., 1990, J Lab. Lin. Med., 115: 36-42 (Chronic Pneumonia); Elliot et al., 1995, Int. J. Pharmac., 17: 141-145 (Rheumatoid Arthritis); Von Dullemen et al., 1995, Gastroenterology, 109: 129-135 (Segmental Ileitis)) and other inflammatory diseases are common intractable diseases, and in these inflammatory reactions, alpha-tumor necrosis factor plays a crucial role. The inhibition of the alpha-tumor necrosis factor can be seen to show an effective blockage effect on chronic and acute inflammatory reactions in animal models of inflammatory diseases. A number of small-molecule inhibitors have been found to be effective in inflammatory diseases involving alpha-tumor necrosis factor (see Review by Lowe, 1998, Exp. Opin. Ther. Patents, 8: 1309-1332). Such molecules are the substituted phenethyl sulfones described in US patents US6020358, US6962940, as well as WO0134606A1, WO0025777, WO2012083153, WO2018157779A1, WO0134604, WO2012083153, and WO2016169533. Apremilast is disclosed in patent US2003187052, and Chinese patents of the same family are CN1652772, CN1965823, CN101683334, and CN03811093.8.

However, the biological activity of isoindoline-1, 3-diketone PDE4 inhibitors in the prior art is relatively general, and thus the efficacy thereof is not high. Therefore, it is necessary to provide an isoindoline-1, 3-diketone PDE4 inhibitor with a novel structure and good efficacy to treat many diseases.

### Summary

In order to solve the problems existing in the prior art and improve the efficacy, the present disclosure provides a compound as shown in formula I and its racemates, stereoisomers, tautomers, isotopic labels, solvates, polymorphs, esters, prodrugs or pharmaceutically acceptable salts:

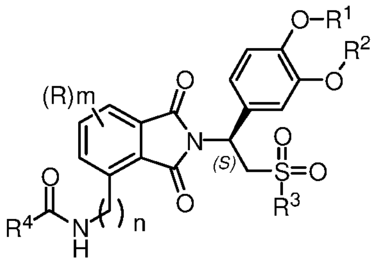

I

in the formula, each R may independently be H, deuterium, halogen, amino, hydroxyl, cyano, nitro, and the following groups unsubstituted or optionally substituted with one, two or more Ra: alkyl groups of C1-C16, heteroalkyl groups of C1-C16, cycloalkyl groups of C3-C12, R'$SO_2$NH-, alkyl- of R'$SO_2$NH-C1-C16, alkyl- of R'$SO_2$-C1-C16, R'$SO_2$-, 3-12 membered heterocyclic groups, aryl groups of C6-C14, and 5-14 membered heteroaryl groups; or cyclic moiety may be formed between two Rs at different positions independently.

Ra is independently selected from deuterium, halogen, amino, hydroxyl, cyano, nitro, oxo (=O), and the following groups unsubstituted or optionally substituted with one, two or more Rb: hydrocarbyl groups of C1-C16, heteroalkyl groups of C1-C16, cycloalkyl groups of C3-C12, 3-12 membered heterocyclic groups, aryl groups of C6-C14, and 5-14 membered heteroaryl groups;
R' is independently selected from the following groups unsubstituted or optionally substituted with one, two or more Rb: hydrocarbyl groups of C1-C16, heteroalkyl groups of C1-C16, cycloalkyl groups of C3-C12, 3-12 membered heterocyclic groups, aryl groups of C6-C14, and 5-14 membered heteroaryl groups;
Rb is selected from deuterium, halogen, amino, hydroxyl, cyano, nitro, oxo (=O), as well as hydrocarbyl groups of C1-C16, heteroalkyl groups of C1-C16, cycloalkyl groups of C3-C12, 3-12 membered heterocyclic groups, aryl groups of C6-C14, and 5-14 membered heteroaryl groups;
m is 1, 2, or 3; n is 0 or 1;
$R^1$ refers to the following groups unsubstituted or optionally substituted with one, two or more R1a: hydrocarbyl groups of C1-C16, heteroalkyl groups of C1-C16, and cycloalkyl groups of C3-C12;
$R^2$ refers to the following groups unsubstituted or optionally substituted with one, two or more R2a: hydrocarbyl groups of C1-C16, heteroalkyl groups of C1-C16, and cycloalkyl groups of C3-C12;
alternatively, $R^1$ and $R^2$ may form cyclic moiety;
$R^3$ refers to the following groups unsubstituted or optionally substituted with one, two or more R3a: hydrocarbyl groups of C1-C16, heteroalkyl groups of C1-C16, and cycloalkyl groups of C3-C12;
$R^4$ refers to the following groups unsubstituted or optionally substituted with one, two or more R4a: hydrocarbyl groups of C1-C16, heteroalkyl groups of C1-C16, and cycloalkyl groups of C3-C12;
R1a is independently selected from deuterium, halogen, amino, hydroxyl, cyano, nitro, oxo (=O), and the following groups unsubstituted or optionally substituted with one, two or more R1b: hydrocarbyl groups of C1-C16, heteroalkyl groups of C1-C16, and cycloalkyl groups of C3-C12;
R2a is independently selected from deuterium, halogen, amino, hydroxyl, cyano, nitro, oxo (=O), and the following groups unsubstituted or optionally substituted with one, two or more R2b: hydrocarbyl groups of C1-C16, heteroalkyl groups of C1-C16, and cycloalkyl groups of C3-C12;
R3a is independently selected from deuterium, halogen, amino, hydroxyl, cyano, nitro, oxo (=O), and the following groups unsubstituted or optionally substituted with one, two or more R3b: hydrocarbyl groups of C1-C16, heteroalkyl groups of C1-C16, and cycloalkyl groups of C3-C12;
R4a is independently selected from deuterium, halogen, amino, hydroxyl, cyano, nitro, oxo (=O), and the following groups unsubstituted or optionally substituted with one, two or more R4b: hydrocarbyl groups of C1-C16, heteroalkyl groups of C1-C16, and cycloalkyl groups of C3-C12;
R1b is selected from deuterium, halogen, amino, hydroxyl, cyano, nitro, oxo (=O), as well as hydrocarbyl groups of C1-C16, and heteroalkyl groups of C1-C 16;
R2b is selected from deuterium, halogen, amino, hydroxyl, cyano, nitro, oxo (=O), as well as hydrocarbyl groups of C1-C16, and heteroalkyl groups of C1-C16;
R3b is selected from deuterium, halogen, amino, hydroxyl, cyano, nitro, oxo (=O), as well as hydrocarbyl groups of C1-C16, and heteroalkyl groups of C1-C16; and
R4b is selected from deuterium, halogen, amino, hydroxyl, cyano, nitro, oxo (=O), as well as hydrocarbyl groups of C1-C16, and heteroalkyl groups of C1-C16.

According to some embodiments of the present disclosure, $R^1$ and $R^2$ can form 5-, 6-, and 7-membered cyclic mioety.

According to some embodiments of the present disclosure, the R may independently be a 5, 6, or 7 substituents.

According to some embodiments of the present disclosure, the hydrocarbyl groups of C1-C16 are alkyl groups of C1-C16, alkenyl groups of C2-C16, and alkynyl groups of C2-C16.

According to some embodiments of the present disclosure, each of the heteroalkyl groups of C1-C16 is an alkyl containing one, two or more heteroatoms selected from N, O, S; specifically, the heteroalkyl groups of C1-C16 may be selected from C1-C16 alkyloxy, C1-C8-alkyl OC1-C8 alkyl-, C1-C8-alkyl-O-C1-C8 alkyl-NH-, C1-C16 alkylthio-, C1-C8-alkyl-S-C1-C8 alkyl-, C1-C8-alkyl-S-C1-C8 alkyl-NH-, C1-C16 alkyl-NH-, C1-C8-alkyl-NH-C1-C8 alkyl-, $NH_2$-C1-C16 alkyl-, -C1-C8-alkyl-NH-C1-C8 alkyl-$NH_2$; and the number of carbon atoms in the heteroalkyl groups of C1-C16 may be further preferably C1-C12, more preferably C5-8.

According to some embodiments of the present disclosure, the two Rs together with the carbon atom to which they are attached form C5-6 membered cycloalkyl groups.

According to some embodiments of the present disclosure, the R is a 5, 6, or 7 substituent, and may independently be H, deuterium, halogen, amino, hydroxyl, cyano, nitro, and the following groups unsubstituted or optionally substituted with one, two or more Ra: alkyl groups of C1-C12, alkenyl groups of C2-C12, alkynyl groups of C2-C12, heteroalkyl groups of C1-C12, cycloalkyl groups of C3-C8, 3-10 membered heterocyclic groups, aryl groups of C6-C10, and 5-10 membered heteroaryl groups.

According to the exemplary embodiment of the present disclosure, the R is a 5, 6, or 7 substituent, and may independently be H, deuterium, halogen, amino, hydroxyl, cyano, nitro, and the following groups unsubstituted or optionally substituted with one, two or more Ra: alkyl groups of C5-C8, alkenyl groups of C5-C8, alkynyl groups of C5-C8, heteroalkyl groups of C5-C8, cycloalkyl groups of C3-C6, 3-6 membered heterocyclic groups, an aryl group of C6, and 5-6 membered heteroaryl groups.

According to some embodiments of the present disclosure, the Ra is independently selected from deuterium, halogen, amino, hydroxyl, cyano, nitro, oxo (=O), and the following groups unsubstituted or optionally substituted with one, two or more Rb: alkyl groups of C1-C12, alkenyl groups of C2-C12, alkynyl groups of C2-C12, heteroalkyl groups of C1-C12, cycloalkyl groups of C3-C8, 3-12 membered heterocyclic groups, aryl groups of C6-C14, and 5-10 membered heteroaryl groups.

According to the exemplary embodiment of the present disclosure, the Ra is independently selected from deuterium, halogen, amino, hydroxyl, cyano, nitro, oxo (=O), and the following groups unsubstituted or optionally substituted with one, two or more Rb: alkyl groups of C5-C8, alkenyl groups of C5-C8, alkynyl groups of C5-C8, heteroalkyl groups of C5-C8, cycloalkyl groups of C3-C6, 3-6 membered heterocyclic groups, an aryl group of C6, and 5-6 membered heteroaryl groups.

According to some embodiments of the present disclosure, R' is independently selected from the following groups unsubstituted or optionally substituted with one, two or more Rb: alkyl groups of C1-C12, alkenyl groups of C2-C12, alkynyl groups of C2-C12, heteroalkyl groups of C1-C12, cycloalkyl groups of C3-C8, 3-12 membered heterocyclic groups, aryl groups of C6-C14, and 5-10 membered heteroaryl groups.

According to the exemplary embodiment of the present disclosure, the R' is independently selected from deuterium, halogen, amino, hydroxyl, cyano, nitro, oxo (=O), and the following groups unsubstituted or optionally substituted with one, two or more Rb: alkyl groups of C5-C8, alkenyl groups of C5-C8, alkynyl groups of C5-C8, heteroalkyl groups of C5-C8, cycloalkyl groups of C3-C6, 3-6 membered heterocyclic groups, an aryl group of C6, and 5-6 membered heteroaryl groups.

According to some embodiments of the present disclosure, Rb is selected from deuterium, halogen, amino, hydroxyl, cyano, nitro, oxo (=O), as well as alkyl groups of C1-C12, alkenyl groups of C2-C12, alkynyl groups of C2-C12, heteroalkyl groups of C1-C12, cycloalkyl groups of C3-C8, 3-12 membered heterocyclic groups, aryl groups of C6-C14, and 5-10 membered heteroaryl groups.

According to the exemplary embodiment of the present disclosure, the Rb is selected from deuterium, halogen, amino, hydroxyl, cyano, nitro, oxo (=O), as well as alkyl groups of C5-C8, alkenyl groups of C5-C8, alkynyl groups of C5-C8, heteroalkyl groups of C5-C8, cycloalkyl groups of C3-C6, 3-6 membered heterocyclic groups, an aryl group of C6, and 5-6 membered heteroaryl groups.

According to some embodiments of the present disclosure,

$R^1$ refers to the following groups unsubstituted or optionally substituted with one, two or more R1a: alkyl groups of C1-C12, alkenyl groups of C2-C12, alkynyl groups of C2-C12, heteroalkyl groups of C1-C12, and cycloalkyl groups of C3-C8; the R1a is independently selected from deuterium, halogen, amino, hydroxyl, cyano, nitro, oxo (=O), and the following groups unsubstituted or optionally substituted with one, two or more R1b: alkyl groups of C1-C12, alkenyl groups of C2-C12, alkynyl groups of C2-C12, heteroalkyl groups of C1-C12, and cycloalkyl groups of C3-C12; the R1b is selected from deuterium, halogen, amino, hydroxyl, cyano, nitro, oxo (=O);

preferably, $R^1$ refers to the following groups unsubstituted or optionally substituted with one, two or more R1a: alkyl groups of C1-C6, alkenyl groups of C2-C6, alkynyl groups of C2-C6, heteroalkyl groups of C1-C6, and cycloalkyl groups of C3-C6; the R1a is independently selected from deuterium, halogen, amino, hydroxyl, cyano, nitro, oxo (=O), and the following groups unsubstituted or optionally substituted with one, two or more R1b: alkyl groups of C1-C6, alkenyl groups of C2-C6, alkynyl groups of C2-C6, heteroalkyl groups of C1-C6, and cycloalkyl groups of C3-C6; the R1b is selected from deuterium, halogen, amino, hydroxyl, cyano, nitro, oxo (=O);

more preferably, $R^1$ refers to the following groups unsubstituted or optionally substituted with one, two or more R1a: alkyl groups of C1-C3, alkenyl groups of C2-C3, alkynyl groups of C2-C3, heteroalkyl groups of C1-C3, and cycloalkyl groups of C3-C6; the R1a is independently selected from deuterium, halogen, amino, hydroxyl, cyano, nitro, oxo (=O), and the following groups unsubstituted or optionally substituted with one, two or more R1b: alkyl groups of C1-C3, alkenyl groups of C2-C3, alkynyl groups of C2-C3, heteroalkyl groups of C1-C3, and cycloalkyl groups of C3-C6; the R1b is selected from deuterium, halogen, amino, hydroxyl, cyano, nitro, oxo (=O);

$R^2$ refers to the following groups unsubstituted or optionally substituted with one, two or more R2a: alkyl groups of C1-C12, alkenyl groups of C2-C12, alkynyl groups of C2-C12, heteroalkyl groups of C1-C12, and cycloalkyl groups of C3-C8; the R2a is independently selected from deuterium, halogen, amino, hydroxyl, cyano, nitro, oxo (=O), and the following groups unsubstituted or optionally substituted with one, two or more R2b: alkyl groups of C1-C12, alkenyl groups of C2-C12, alkynyl groups of C2-C12, heteroalkyl groups of C1-C12, and cycloalkyl groups of C3-C12; the R2b is selected from deuterium, halogen, amino, hydroxyl, cyano, nitro, oxo (=O);

preferably, $R^2$ refers to the following groups unsubstituted or optionally substituted with one, two or more R2a: alkyl groups of C1-C6, alkenyl groups of C2-C6, alkynyl groups of C2-C6, heteroalkyl groups of C1-C6, and cycloalkyl groups of C3-C6; the R2a is independently selected from deuterium, halogen, amino, hydroxyl, cyano, nitro, oxo (=O), and the following groups unsubstituted or optionally substituted with one, two or more R2b: alkyl groups of C1-C6, alkenyl groups of C2-C6, alkynyl groups of C2-C6, heteroalkyl groups of C1-C6, and cycloalkyl groups of C3-C6; the R2b is selected from deuterium, halogen, amino, hydroxyl, cyano, nitro, oxo (=O);

more preferably, $R^2$ refers to the following groups unsubstituted or optionally substituted with one, two or more R2a: alkyl groups of C1-C5, alkenyl groups of C2-C5, alkynyl groups of C2-C5, heteroalkyl groups of C1-C5, and cycloalkyl groups of C3-C6; the R2a is independently selected from deuterium, halogen, amino, hydroxyl, cyano, nitro, oxo (=O), and the following groups unsubstituted or optionally substituted with one, two or more R2b: alkyl groups of C1-C5, alkenyl groups of C2-C5, alkynyl groups of C2-C5, heteroalkyl groups of C1-C5, and cycloalkyl groups of C3-C6; the R2b is selected from deuterium, halogen, amino, hydroxyl, cyano, nitro, oxo (=O);

$R^3$ refers to the following groups unsubstituted or optionally substituted with one, two or more R3a: alkyl groups of C1-C12, alkenyl groups of C2-C12, alkynyl groups of C2-C12, heteroalkyl groups of C1-C12, and cycloalkyl groups of C3-C8; the R3a is independently selected from deuterium, halogen, amino, hydroxyl, cyano, nitro, oxo (=O), and the following groups unsubstituted or optionally substituted with one, two or more R3b: alkyl groups of C1-C12, alkenyl groups of C2-C12, alkynyl groups of C2-C12, heteroalkyl groups of C1-C12, and cycloalkyl groups of C3-C12; the R3b is selected from deuterium, halogen, amino, hydroxyl, cyano, nitro, oxo (=O);

preferably, $R^3$ refers to the following groups unsubstituted or optionally substituted with one, two or more R3a: alkyl groups of C1-C6, alkenyl groups of C2-C6, alkynyl groups of C2-C6, heteroalkyl groups of C1-C6, and cycloalkyl groups of C3-C6; the R3a is independently selected from deuterium, halogen, amino, hydroxyl, cyano, nitro, oxo (=O), and the following groups unsubstituted or optionally substituted with one, two or more R3b: alkyl groups of C1-C6, alkenyl groups of C2-C6, alkynyl groups of C2-C6, heteroalkyl groups of C1-C6, and cycloalkyl groups of C3-C6; the R3b is selected from deuterium, halogen, amino, hydroxyl, cyano, nitro, oxo (=O);

more preferably, $R^3$ refers to the following groups unsubstituted or optionally substituted with one, two or more R3a: alkyl groups of C1-C5, alkenyl groups of C2-C5, alkynyl groups of C2-C5, heteroalkyl groups of C1-C5, and cycloalkyl groups of C3-C6; the R3a is independently selected from deuterium, halogen, amino, hydroxyl, cyano, nitro, oxo (=O), and the following groups unsubstituted or optionally substituted with one, two or more R3b: alkyl groups of C1-C5, alkenyl groups of C2-C5, alkynyl groups of C2-C5, heteroalkyl groups of C1-C5, and cycloalkyl groups of C3-C6; the R3b is selected from deuterium, halogen, amino, hydroxyl, cyano, nitro, oxo (=O);

$R^4$ refers to the following groups unsubstituted or optionally substituted with one, two or more R4a: alkyl groups of C1-C12, alkenyl groups of C2-C12, alkynyl groups of C2-C12, heteroalkyl groups of C1-C12, and cycloalkyl groups of C3-C8; the R4a is independently selected from deuterium, halogen, amino, hydroxyl, cyano, nitro, oxo (=O), and the following groups unsubstituted or optionally substituted with one, two or more R4b: alkyl groups of C1-C12, alkenyl groups of C2-C12, alkynyl groups of C2-C12, heteroalkyl groups of C1-C12, and cycloalkyl groups of C3-C12; the R4b is selected from deuterium, halogen, amino, hydroxyl, cyano, nitro, oxo (=O);

preferably, $R^4$ refers to the following groups unsubstituted or optionally substituted with one, two or more R4a: alkyl groups of C1-C6, alkenyl groups of C2-C6, alkynyl groups of C2-C6, heteroalkyl groups of C1-C6, and cycloalkyl groups of C3-C6; the R4a is independently selected from deuterium, halogen, amino, hydroxyl, cyano, nitro, oxo (=O), and the following groups unsubstituted or optionally substituted with one, two or more R4b: alkyl groups of

C1-C6, alkenyl groups of C2-C6, alkynyl groups of C2-C6, heteroalkyl groups of C1-C6, and cycloalkyl groups of C3-C6; and the R4b is selected from deuterium, halogen, amino, hydroxyl, cyano, nitro, oxo (=O).

According to the embodiments of the present disclosure, the compound as shown in formula I is further selected from the following formula II:

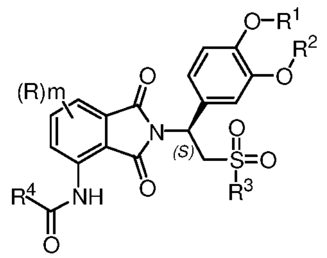

II

in formula II, the R, $R^1$, $R^2$, $R^3$, $R^4$, and m are as defined above.

According to the embodiments of the present disclosure, the compound as shown in formula I is further selected from the following formula III:

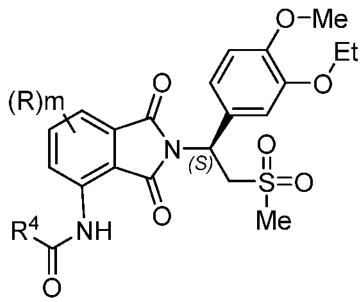

III

in formula III, the R, $R^4$, and m are as defined above.

According to the embodiments of the present disclosure, the compound as shown in formula I is further selected from the following formula IV:

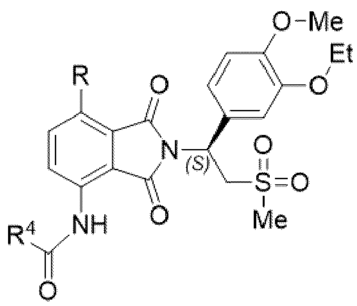

Formula IV

in formula IV, the R, m, and $R^4$ are as defined above.

According to the embodiments of the present disclosure, in the compound as shown in formula I (including formula II-III) and its racemates, stereoisomers, tautomers, isotopic labels, solvates, polymorphs, esters, prodrugs or pharmaceutically acceptable salts, illustrative non-restrictive specific examples of the compound as shown in formula I are as follows:

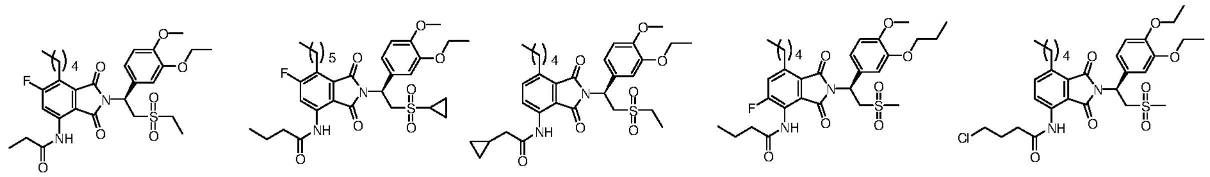

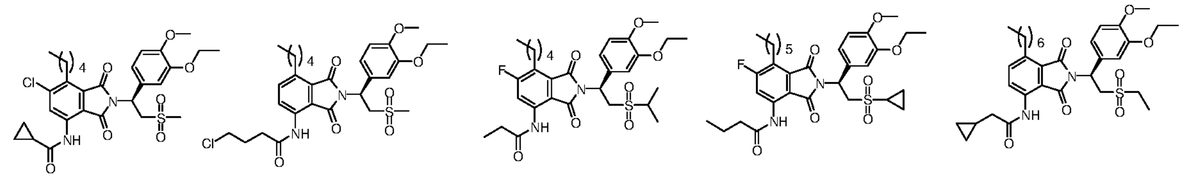

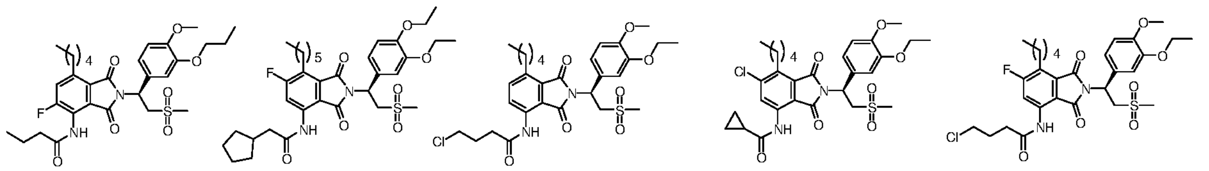

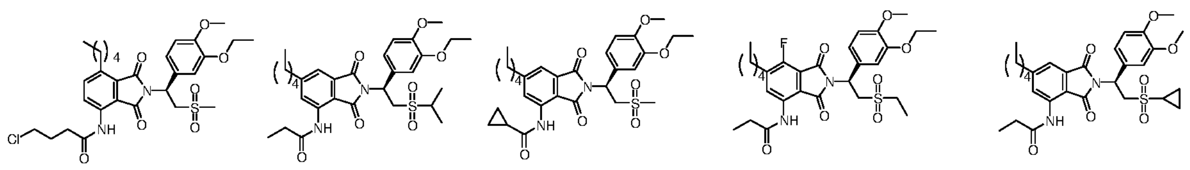

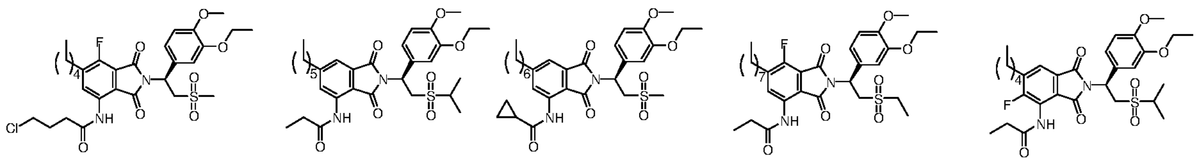

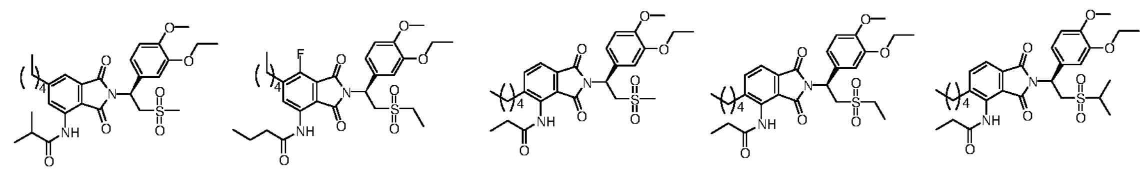

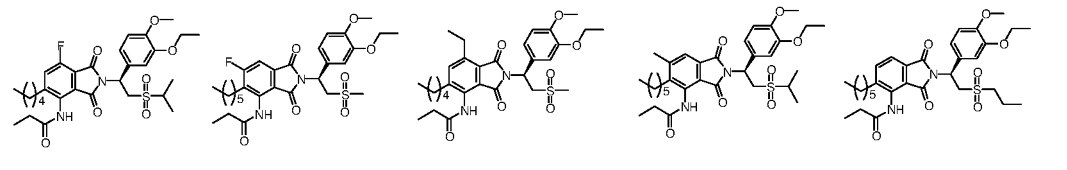

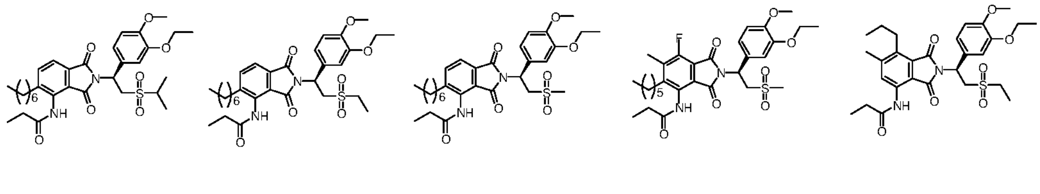

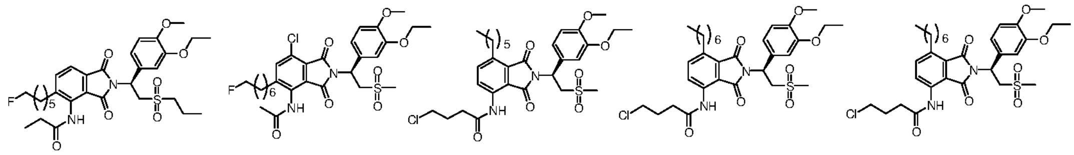

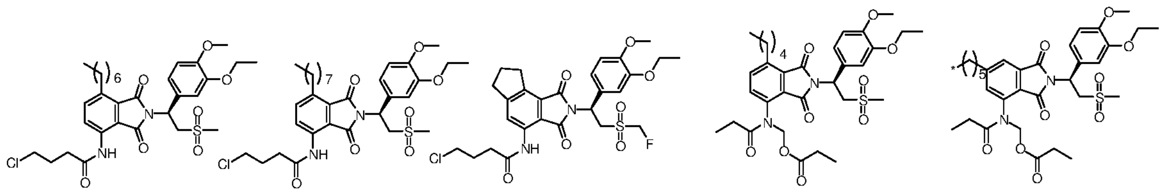

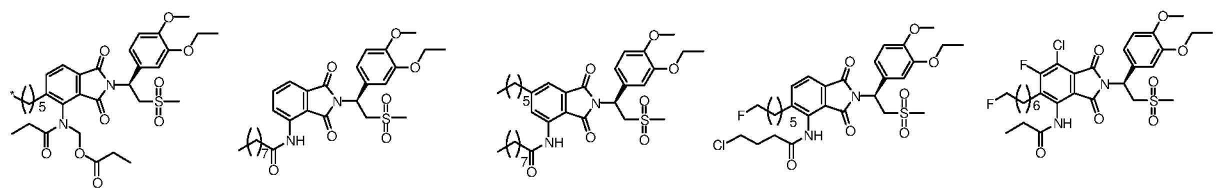

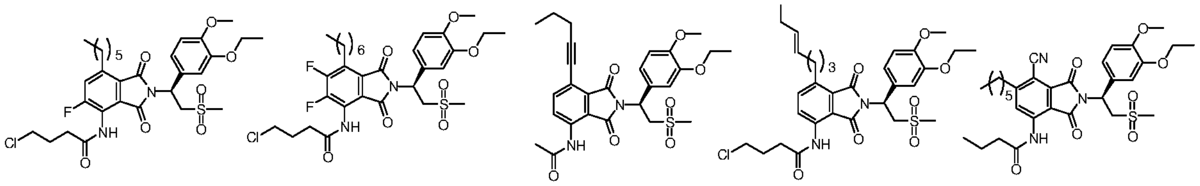

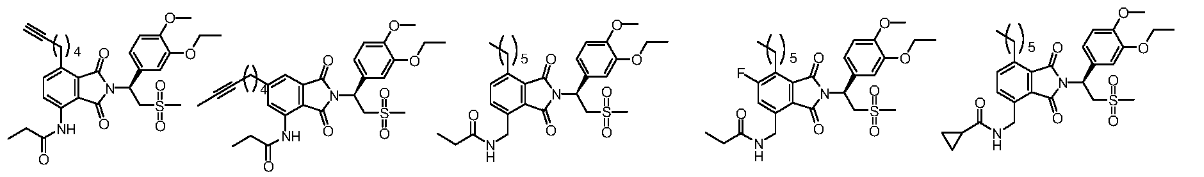

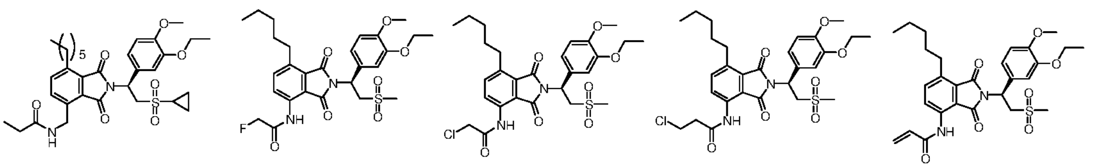

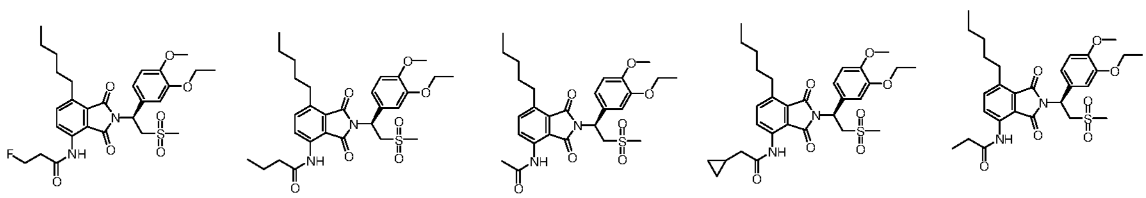

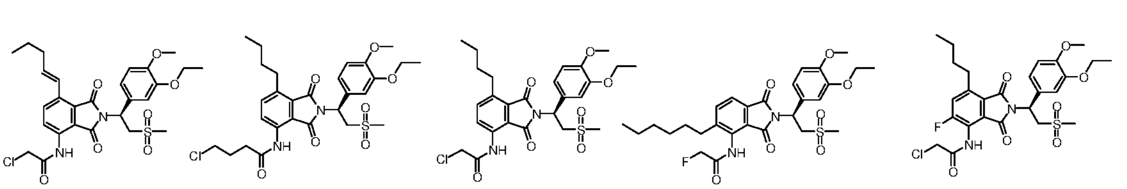

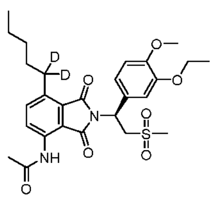
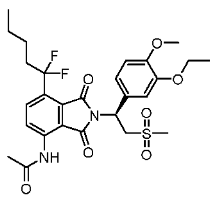
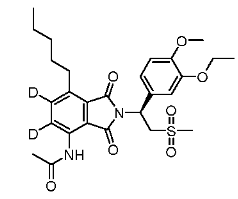
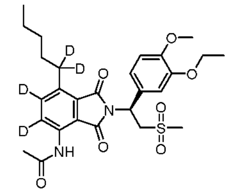
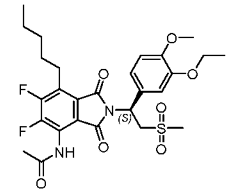
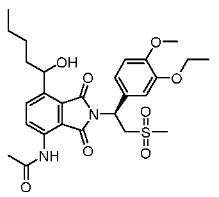
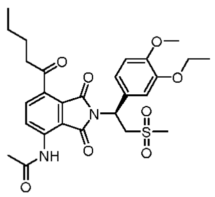
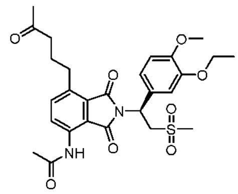
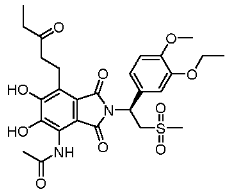
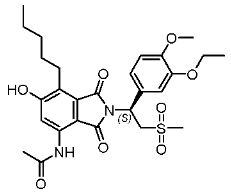
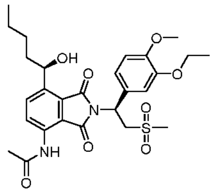
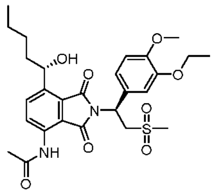
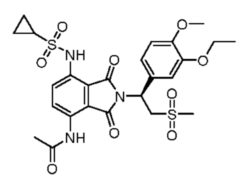
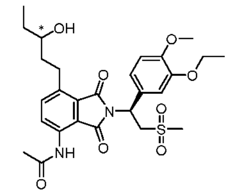
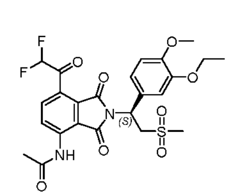
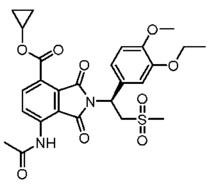
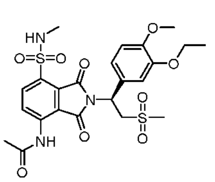
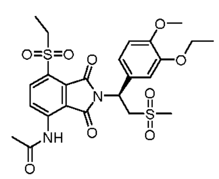
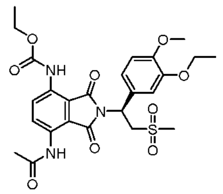
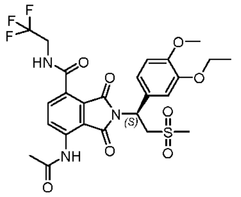
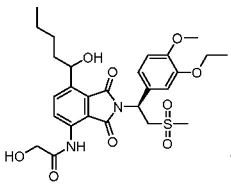
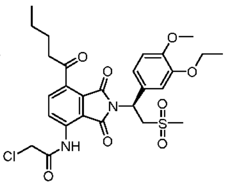
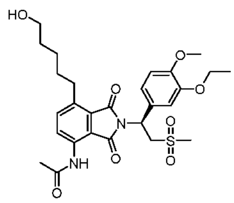
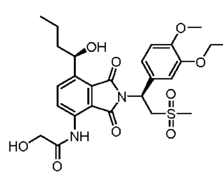
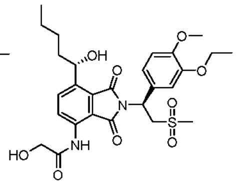
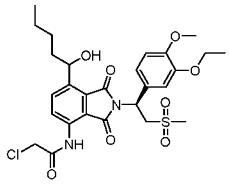
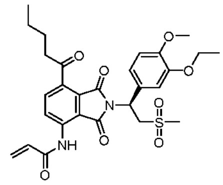
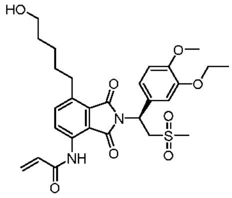
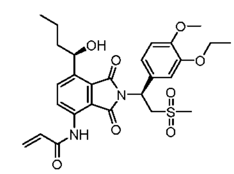
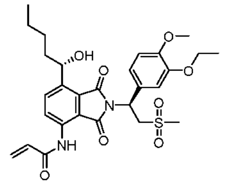

The present disclosure also provides preparation methods the compound as shown in formula I (including formula II-III) and its racemates, stereoisomers, tautomers, isotopic labels, solvates, polymorphs, esters, prodrugs or pharmaceutically acceptable salts, but not limited to the methods described below. All raw materials are prepared based on the group characteristics of target molecules that conform to the general formula law, and through solutions in these routes and methods well known to the those having ordinary skill in the art of organic chemistry, or are purchased directly. The compound provided by the present disclosure can be synthesized using the methods described below in combination with synthetic methods known in the art of synthetic organic chemistry, or with relevant modifications recognized by those skilled in the art. Those skilled in the art know that, according to a specific target structure, one or more of the following solutions may be optionally combined, or any steps in one or more of the solutions may be combined, so as to obtain a synthetic solution.

The preparation method of the compound as shown in formula I of the present disclosure includes: under suitable conditions, a substituted benzoic acid raw material I-1 (with R' being halogen, alkane, carboxyl, cyano, amino or nitro, and t being an integer ranging from 1 to 5) is converted into acid anhydride I-2 through synthesis, and then further reacts with an amine intermediate I-3 to generate substituted isoindoline-1, 3-diketone I-4; and under suitable conditions, the compound as shown in formula I is obtained through the steps of adding protecting groups, removing protecting groups, substituting, condensing, reductive amination or hydrolysis. Specifically, the compound can be synthesized with reference to the following further solution.

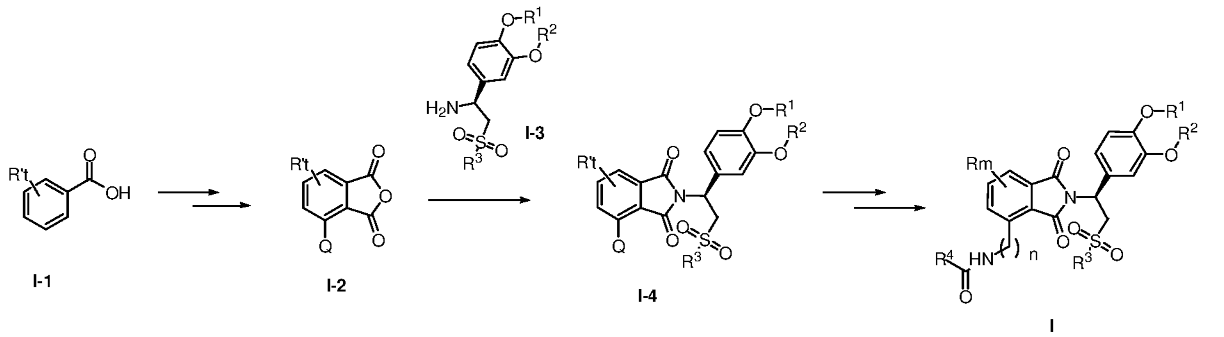

I-1 I-2 I-3 I-4 I

The preparation of the compound provided by the present disclosure may include one or more of the following general steps according to the known synthesis method (such as WO2016169533). Further synthetic route of intermediate sulfonyl ethylamine I-3 (11):

A raw material substituted benzoate 1 is subjected to para-phenolic group protection to obtain compound 2, subjected to meta-etherification to obtain compound 3, and subjected to para-phenolic group deprotection to obtain compound 4. Similar etherification is performed on para-substituted compound 5, ester is reduced to alcohol 6, and oxidation is carried out to obtain intermediate aldehyde 7. Further reaction is carried out to successfully obtain methylsulfonyl styrene 8, which is further alkylated to obtain alkylsulfonyl styrene 9, and subjected to an amination reaction to obtain a product 10. Through chiral resolution or separation of phenyl ketone, (S)-1-(3-alkoxy-4-alkoxyphenyl)-2-alkyl sulfonyl ethylamine 11 can be obtained.

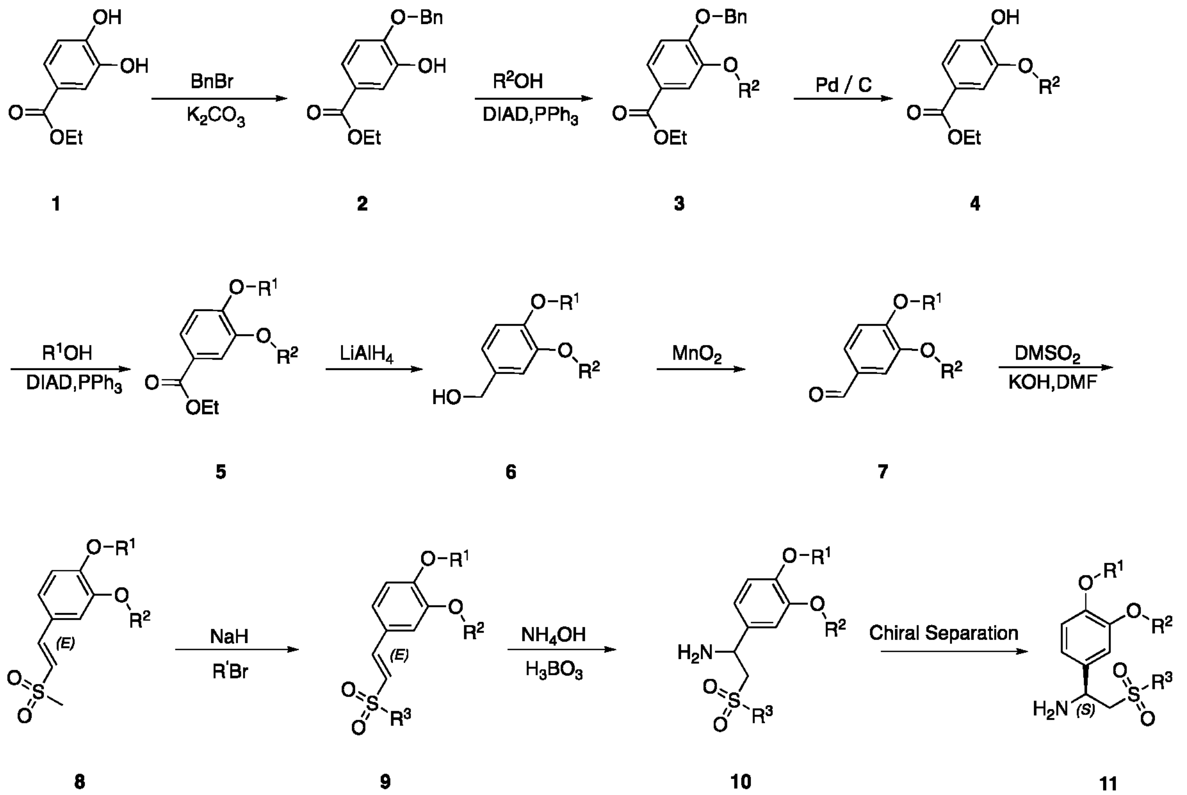


1 2 3 4

5 6 7

8 9 10 11

In addition, a raw material phenyl cyanide 12 may also be converted into phenyl ketone 14 intermediate through an intermediate 13, reduced to alcohol 15, and dehydrated to obtain methylsulfonyl styrene 8, so that chiral 1-(3-alkoxy-4-alkoxyphenyl)-2-alkyl sulfonyl ethylamine 11 is obtained by further chemical conversion as above.

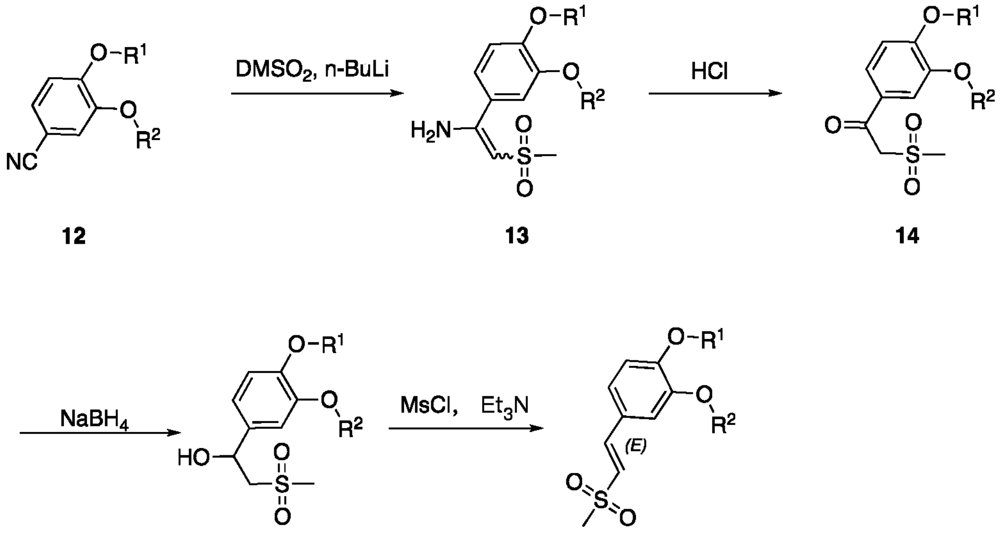


12 13 14 15 8

Bihydroxy-substituted benzophenone 24 is para-alkylated to obtain 17, further alkylated to produce bisalkoxy benzophenone 18, subjected to a bromination reaction to obtain bromobenzophenone 19, and subjected to thioetherification to obtain an intermediate 20. The intermediate 11 is synthesized through two pathways in 21, 22, and 23.

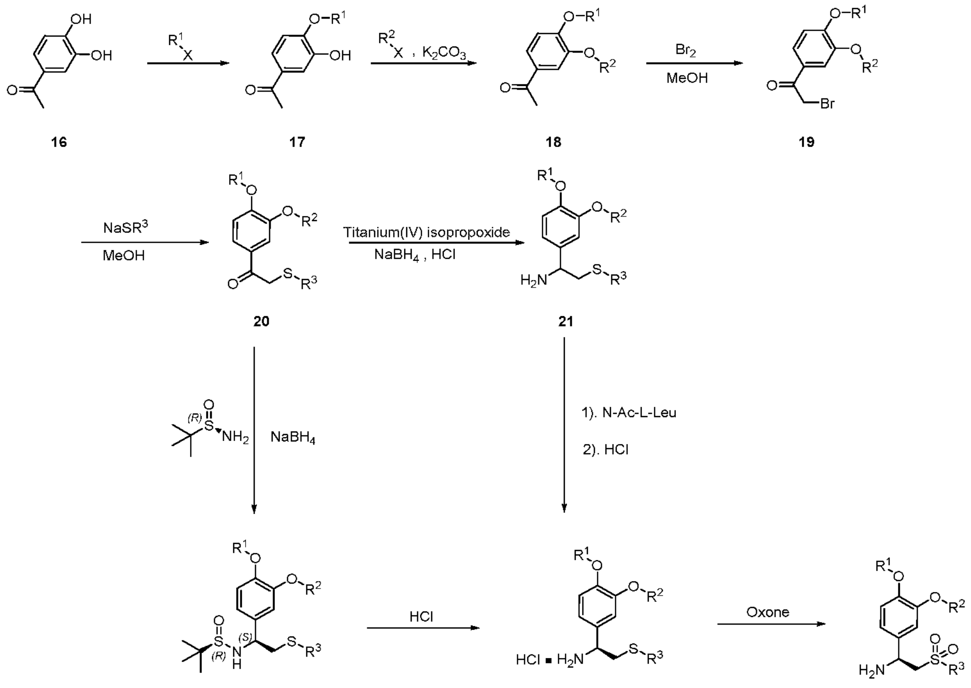


16 17 18 19 20 21 22 23 11

$R^1$, $R^2$ and $R^3$ are as defined in the aforementioned formula I, and X is selected from halogen.

Corresponding chiral compounds can be separated from their racemic compounds by techniques available in the art. Examples include, but are not limited to, chiral salt formation, the use of chirality and high performance liquid chromatography (HPLC), a swell as the formation and crystallization of chiral salts. See, e.g., Jacques, J. et al., Enantiomers, Racemates and Resolutions (Wiley-Interscience, New York, 1981); Wilen, S.H. et al., Tetrahedron, 33:2725 (1977); Eliel, E.L., Stereochemistry of Carbon Compounds (McGraw-Hill, New York, 1962) and Wilen, S.H., Tables of

Resolving Agents and Optical Resolutions, 268 (written by Eliel E.L., Notre Dame University Press, Notre Dame, IN, 1972).

In specific examples, chiral amino acid salts of (S)-2-(3-alkoxy-4-alkoxyphenyl)-1-(alkylsulfonyl)-ethylenediamine 11 include, but are not limited to, salts formed with L-isomers of amino acids or L-isomers of acylated amino acids.

According to the embodiments of the present disclosure, the compound provided by the present disclosure can be synthesized according to one selected from the following synthetic routes (referring to WO2018157779A1):

Synthetic route 1:

Halogenated o-methyl benzoic acid 24 undergoes a nitration reaction to generate 25, undergoes an oxidation reaction to obtain substituted phthalic acid 26, then undergoes an anhydride reaction to obtain halogenated 4-nitrobenzoic anhydride 27, and further reacts with 1-(3-alkoxy-4-alkoxyphenyl)-2-alkylsulfonyl ethylamine 11 in acetic acid to obtain halogenated (S)-2-[1-(3-alkoxy-4-alkoxyphenyl)-2-alkylsulfonyl ethyl]-4-nitroisoindoline-1, 3-diketone 28; a nitro group is further reduced to obtain an intermediate 29, which is subjected to acylation to obtain 30; and long-chain hydrocarbon-substituted (S)-2-[1-(3-alkoxy-4-alkoxyphenyl)-2-alkylsulfonyl ethyl]-4-amidoisoindoline-1, 3-diketone II is generated by a Suzuki reaction or Sonogashira reaction.

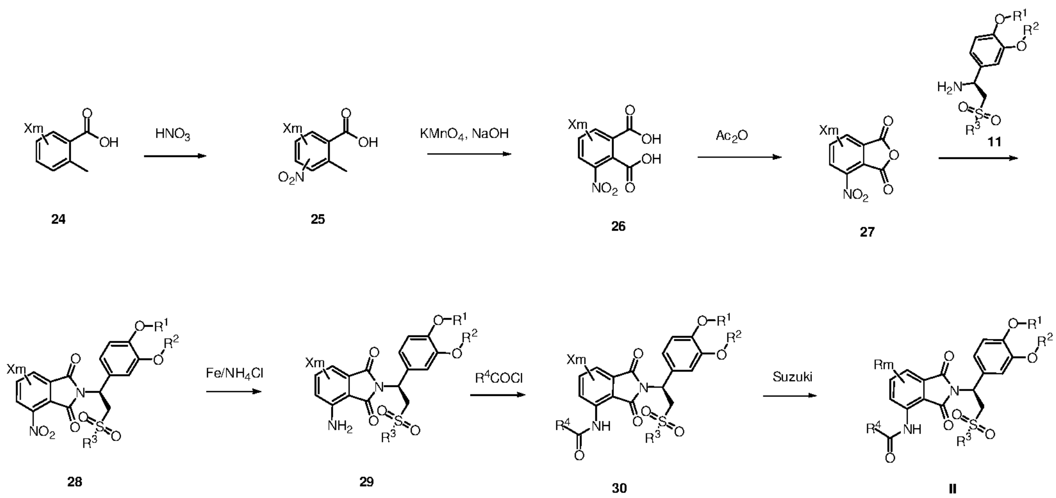

24 25 26 27 11 28 29 30 II

R, $R^1$, $R^2$ $R^3$, $R^4$, and m are as defined in the aforementioned formula I, and X is selected from halogen (Cl, Br, I).

Synthetic route 2:

Halogenated 3-nitrobenzoic anhydride 27 and a nitro group are reduced to obtain an intermediate 31 and 4-acylated benzoic anhydride 32, and further reacts with 1-(3-alkoxy-4-alkoxyphenyl)-2-alkylsulfonyl ethylamine 11 in acetic acid to obtain halogenated (S)-2-[1-(3-alkoxy-4-alkoxyphenyl)-2-alkylsulfonyl ethyl]-4-amidoisoindoline-1, 3-diketone 30; and long-chain hydrocarbon-substituted (S)-2-[1-(3-alkoxy-4-alkoxyphenyl)-2-alkylsulfonyl ethyl]-4-amidoisoindoline-1, 3-diketone II is generated by a Suzuki reaction or Sonogashira reaction.

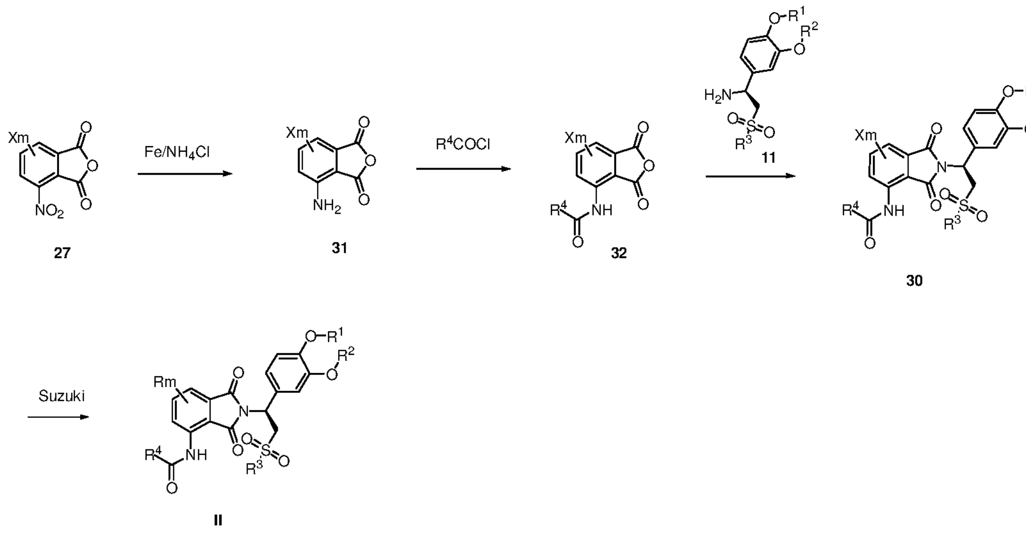


27 31 32 30 II

R, $R^1$, $R^2$ $R^3$, $R^4$, and m are as defined in the aforementioned formula I, and X is selected from halogen (Cl, Br, I).

Synthetic route 3:

Hydrocarbonated 4-nitrobenzoic anhydride 33 further reacts with 1-(3-alkoxy-4-alkoxyphenyl)-2-alkylsulfonyl ethylamine 11 in acetic acid to obtain hydrocarbonated (S)-2-[1-(3-alkoxy-4-alkoxyphenyl)-2-alkylsulfonyl ethyl]-4-nitroisoindoline-1, 3-diketone 34; and a nitro group is reduced to obtain an intermediate (S)-2-[1-(3-alkoxy-4-alkoxyphenyl)-2-alkylsulfonyl ethyl]-4-amidoisoindoline-1, 3-diketone 35, which is subjected to acylation to obtain (S)-2-[1-(3-alkoxy-4-alkoxyphenyl)-2-alkylsulfonyl ethyl]-4-amidoisoindoline-1, 3-diketone II.

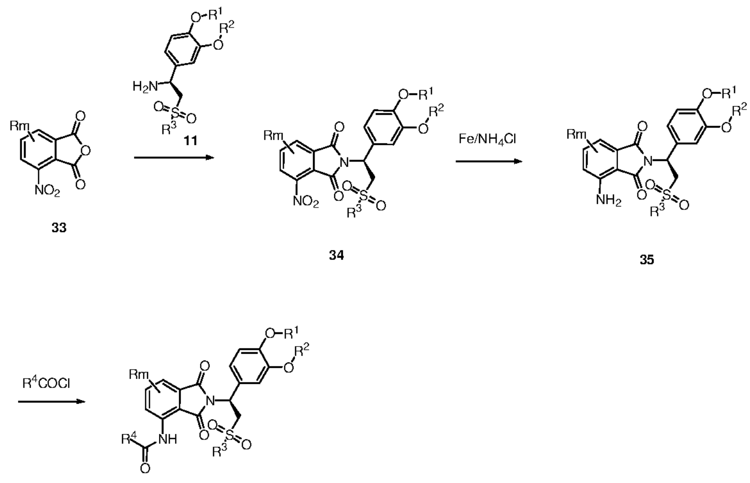


33 34 35 II

R, $R^1$, $R^2$ $R^3$, $R^4$, and m are as defined in the aforementioned formula I.

Synthetic route 4:

Hydrocarbonated 4-aminobenzoic anhydride 36 is acylated to obtain an intermediate 37, and further reacts with 1-(3-alkoxy-4-alkoxyphenyl)-2-alkylsulfonyl ethylamine 11 in acetic acid to obtain chain hydrocarbon-substituted (S)-2-[1-(3-alkoxy-4-alkoxyphenyl)-2-alkylsulfonyl ethyl]-4-amidoisoindoline-1, 3-diketone II.

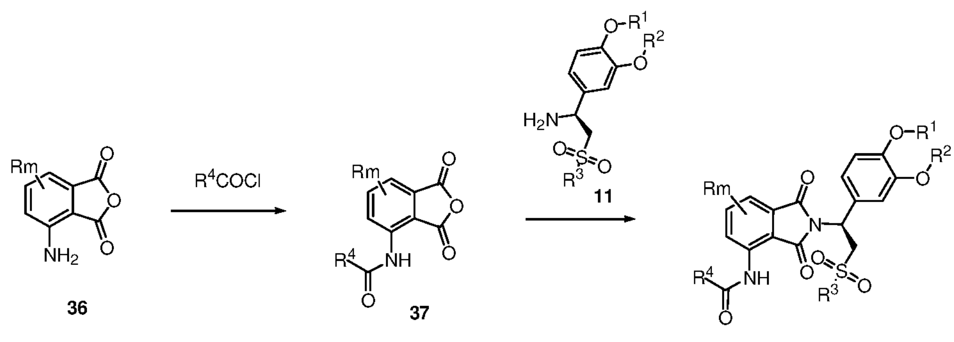


36 37 11 II

R, $R^1$, $R^2$ $R^3$, $R^4$, and m are as defined in the aforementioned formula II.

Synthetic route 5:

4-nitrobenzoic anhydride 27 is aminated to obtain 38, and is subjected to a Mitsunobu reaction with alcohol 39 to obtain an intermediate 28; and after a Suzuki reaction or Sonogashira reaction, an intermediate 34 is generated, which is further reduced to obtain an amino group 35, and then subjected to an acylation reaction to obtain chain hydrocarbon-substituted (S)-2-[1-(3-alkoxy-4-alkoxyphenyl)-2-alkylsulfonyl ethyl]-4-amidoisoindoline-1, 3-diketone II.

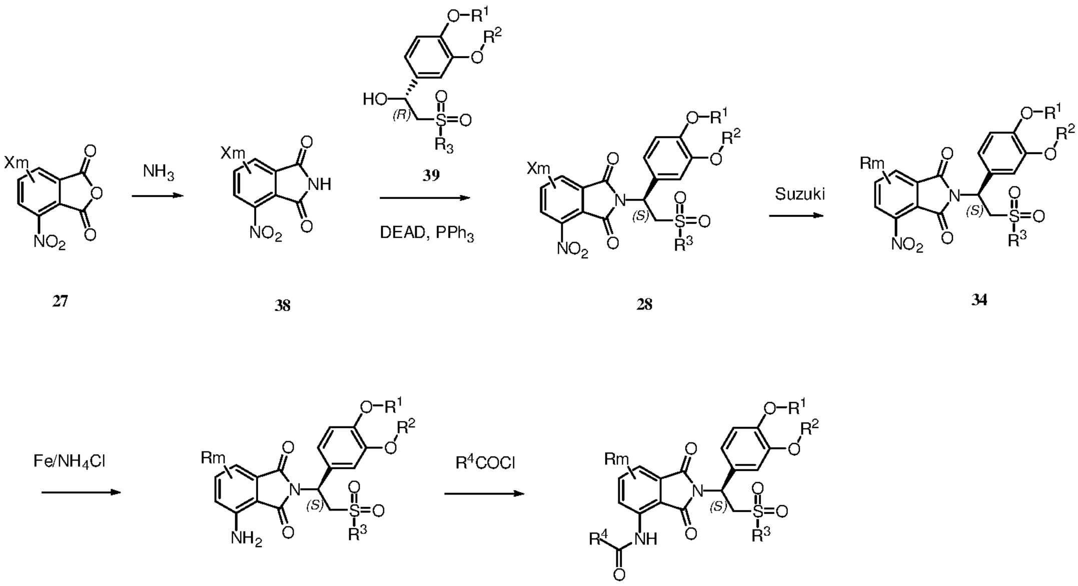


39 27 38 28 34 35 II

R, $R^1$, $R^2$ $R^3$, $R^4$, and m are as defined in the aforementioned formula II.

Synthetic route 6:

Hydrocarbonated (S)-2-[1-(3-alkoxy-4-alkoxyphenyl)-2-alkylsulfonyl ethyl]-4-aminoisoindoline-1, 3-diketone 35 is brominated to obtain 39, cyanated to obtain a cyano compound 40, reduced to obtain a 4-aminomethyl substituted group intermediate 41, and acylated to obtain chain hydrocarbon-substituted (S)-2-[1-(3-alkoxy-4-alkoxyphenyl)-2-alkylsulfonyl ethyl]-4-(amidomethyl) isoindoline-1, 3-diketone.

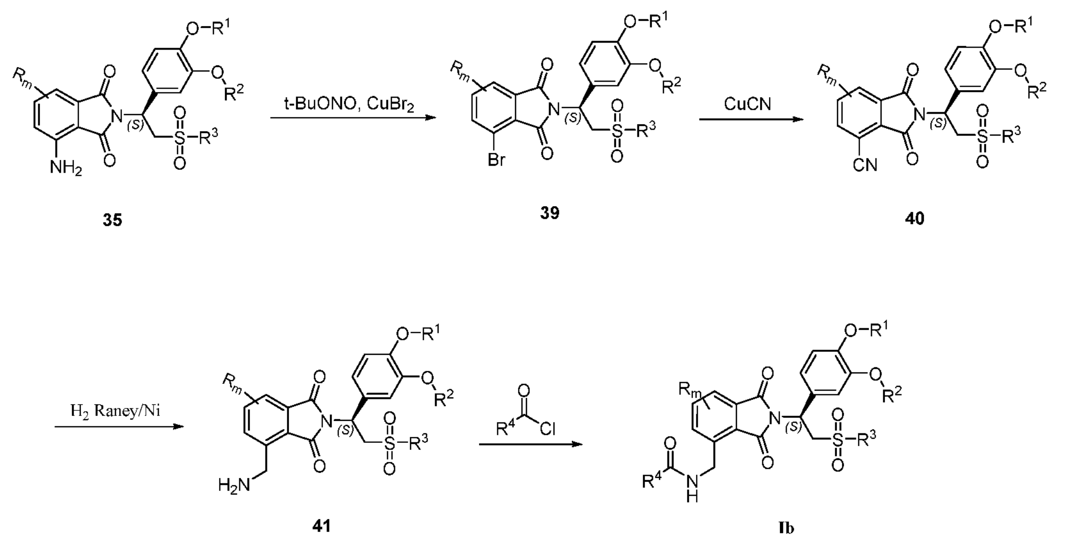


35 39 40

41 Ib

R, R1, R2 R3, R4, and m are as defined in the aforementioned formula I.

The present disclosure further provides a pharmaceutical composition, which contains the compound as shown in formula I and its racemates, stereoisomers, tautomers, isotopic labels, solvates, polymorphs, esters, prodrugs or pharmaceutically acceptable salts.

In some embodiments, the pharmaceutical composition according to the present disclosure contains a therapeutically effective amount of the compound as shown in formula I and its racemates, stereoisomers, tautomers, isotopic labels, solvates, polymorphs, metabolites, esters, prodrugs, or pharmaceutically acceptable salts and pharmaceutically acceptable carriers.

The carrier in the pharmaceutical composition is "acceptable", which is compatible with (and preferably, is capable of stabilizing) the active components of the composition and is not deleterious to the subject being treated. One or more solubilizers may be used as pharmaceutical excipients for delivery of active compounds.

The present disclosure further provides the use of the compound as shown in formula I and its racemates, stereoisomers, tautomers, isotopic labels, solvates, polymorphs, esters, prodrugs or pharmaceutically acceptable salts, or the pharmaceutical composition in the preparation of medicines for inhibiting PDE4 enzyme.

The present disclosure further provides the use of the compound as shown in formula I and its racemates, stereoisomers, tautomers, isotopic labels, solvates, polymorphs, esters, prodrugs or pharmaceutically acceptable salts, or the pharmaceutical composition in the preparation of medicines for treating diseases related to the regulation of intracellular cAMP level.

The present disclosure further provides the use of the compound as shown in formula I and its racemates, stereoisomers, tautomers, isotopic labels, solvates, polymorphs, esters, prodrugs or pharmaceutically acceptable salts, or the pharmaceutical composition in the preparation of medicines for inhibiting the production of TNF-$\alpha$ or NF-$\kappa$B.

According to the embodiments of the present disclosure, the conditions ameliorated by by inhibiting the PDE4 with the medicines for inhibiting the PDE4 include, but are not limited to, dermatitis, psoriasis, atopic dermatitis, seborrheic dermatitis, stasis dermatitis, palmoplantar abscess, asthma, inflammation (e.g., inflammation caused by reperfusion), chronic or acute obstructive pulmonary disease, chronic or acute pneumonia, pulmonary diseases caused by viruses such as Covid-19, enteritis, segmental ileitis, psoriasis, psoriatic arthritis, Behcet's disease or colitis.

In particular method of the present disclosure, the compound provided by the present disclosure, or the pharmaceutically acceptable polymorph, prodrug, salt, solvate, hydrate or clathrate thereof, is administered in combination with at least one another therapeutic agent.

The unit dosage forms of the present disclosure are suitable for oral, mucosal (e.g., nasal, sublingual, vaginal, buccal or rectal), parenteral (e.g., subcutaneous, intravenous, single bolus injection, intramuscular or arterial) or transdermal administration to patients, as well as topical dosage forms in the form of topical or inhalants. The dosage forms include, but are not limited to: tablets, pills, caplets, sustained release dosage forms, capsules such as soft elastic gelatin capsules, cachets, troches, dispersants, suppositories, ointments, cataplasm (poultices), plasters, powders, dressings, creams, medicinal paste, solutions, patches, aerosols (such as nasal sprays or inhalants), gels, dry powder inhalants, liquid dosage forms suitable for oral or transmucosal administration to patients, including suspensions (such as aqueous or non-aqueous suspensions, oil-in-water emulsions, or water-in-oil liquid emulsions), solutions, and elixirs, liquid dosage forms suitable for parenteral administration to patients, and sterile solid dosage forms (e.g., crystalline or amorphous solids) that can be reconstituted to provide liquid dosage forms suitable for parenteral administration to patients. The

specific dosage forms encompassed by the present disclosure vary in various aspects, which will be apparent to those skilled in the art. See, for example, Remington's Pharmaceutical Sciences, 18th edition, published by Mack, Easton PA (1990).

Terminology:

Unless otherwise stated, definitions of groups and terms set forth in the Description and Claims of the present application, including their definitions as examples, exemplary definitions, preferred definitions, definitions set forth in tables, definitions of specific compounds described in the examples etc., may be arbitrarily combined and bonded with each other. The group definitions and compound structures after such combinations and bindings shall fall within the scope contained in the Description of the present application.

The term "halogen" refers to F, C1, Br and I. In other words, F, C1, Br, and I may be described as "halogen" in this Description.

Optionally substituted with substituents described herein encompasses both unsubstituted as well as substituted with one or more substituents, e.g., "optionally substituted with one, two or more R" means that it may be not substituted with R (unsubstituted) or substituted with one, two or more R.

The term "hydrocarbyl groups" include saturated or unsaturated chain or cyclic hydrocarbyl groups with straight or branched chains, the types of which may be selected from alkyl groups, alkenyl groups, alkynyl groups, etc., and the number of carbon atoms of each of the hydrocarbyl groups (alkyl groups, alkenyl groups, alkynyl groups) is preferably 1-16, more preferably 1-12, 1-8, 5-8, 1-5, 1-3, etc. Specific groups may be included, but not limited to the followings: methyl, ethyl, n-propyl, isopropyl, n-butyl, isobutyl, tert-butyl, n-pentyl, isopentyl, neopentyl, n-hexyl, vinyl, 1-propenyl, 2-propenyl, 1-methylvinyl, 1-butenyl, 1-ethylvinyl, 1-methyl-2-propenyl, 2-butenyl, 3-butenyl, 2-methyl-1-propenyl, 2-methyl-2-propenyl, 1-pentenyl, 1-hexenyl, ethynyl, 1-propynyl, 2-propynyl, 1-butynyl, 1-methyl-2-propynyl, 3-butynyl, 1-pentynyl, 1-hexynyl, cyclopropyl, cyclobutyl, cyclopentyl, and cyclohexyl; and the hydrocarbyl groups in other terms (including alkyl groups, alkenyl groups, and alkynyl groups) also conforms to this definition.

The term "heteroalkyl group" by itself or in combination with another term represents a stable straight-chain or branched-chain alkyl atom group or its combination, composed of a certain number of carbon atoms and at least one heteroatom. The number of the carbon atoms may be 1-16, for example, 1, 2, 3, 4, 5, 6, 7, 8, 9, 10, 11, 12, 13, 14, 15, 16. The heteroalkyl group may optionally contain one, two, or more heteroatoms selected from N, O, S (or be interpreted as optional heteroatoms inserted into either a C-C bond or a C-H bond in an alkyl group). The heteroatoms O, N, and S may be located at any internal positions of the heteroalkyl group or at the positions where alkyl groups attach to the rest of a molecule. Examples include but are not limited to $-CH_2-CH_2-O-CH_3$, $-CH_2-CH_2-NH-CH_3$, $-CH_2-CH_2-N(CH_3)-CH_3$, $-CH_2-S-CH_2-CH_3$, - $CH_2-CH_2$, $-S(O)-CH_3$, $-CH_2-CH_2-S(O)_2-CH_3$, $-CH=CH-O-CH_3$, $-CH_2-CH=N-OCH_3$, and - $CH=CH-N(CH_3)-CH_3$. At most two heteroatoms may be consecutive, e.g., $-CH_2-NH-OCH_3$.

The term "cycloalkyl group", including "cycloalkyl groups of $C_{3-12}$", should be understood to refer to a saturated or unsaturated monovalent mono- or bicyclic ring having 3-12 carbon atoms, preferably cycloalkyl groups of $C_{3-8}$, and more preferably cycloalkyl groups of $C_{3-6}$. For example, the cycloalkyl groups of $C_{3-8}$ should be understood to refer to saturated or unsaturated monovalent monocyclic or bicyclic rings having 3, 4, 5, 6, 7 or 8 carbon atoms. The cycloalkyl groups of $C_{3-12}$ may be monocyclic hydrocarbyl groups, such as cyclopropyl, cyclobutyl, cyclopentyl, cyclohexyl, cycloheptyl, cyclooctyl, cyclononyl or cyclodecyl, or may be bicyclic hydrocarbyl groups such as tetrahydronaphthalene or decahydronaphthalene.

The term "3-12 membered heterocyclic groups" should be understood to refer to saturated monovalent monocyclic, bicyclic hydrocarbon-ring or bridged alkane, including 1-5 non-aromatic cyclic groups having heteroatoms independently selected from N, O, and S, with a total number of ring formation atoms being 3-12 (e.g., 3, 4, 5, 6, 7, 8, 9, 10, 11, 12), preferably "3-10 membered heterocyclic groups". The term "3-10 membered heterocyclic groups" refers to saturated monovalent monocyclic, bicyclic hydrocarbon-ring or bridged alkane, which includes 1-5, preferably 1-3 heteroatoms independently selected from N, O, and S, for example, 1, 2, and 3 heteroatoms independently selected from N, O, and S. The heterocyclic groups may be attached to the rest of a molecule through any of the carbon atoms or a nitrogen atom (if present). In particular, the heterocyclic groups may include but are not limited to: 4-membered rings, such as azetidinyl, and oxetanyl; 5-membered rings, such as tetrahydrofuranyl, dioxolenyl, pyrrolidinyl, imidazolidinyl, pyrazolidyl, and pyrrolinyl; 6-membered rings, such as tetrahydropyranyl, piperidinyl, morpholinyl, dithianyl, thiomorpholinyl, piperazinyl or trithianyl; or 7-membered rings, such as diazacycloheptyl. Optionally, the heterocyclyl groups may be benzo-fused. The heterocyclyl groups may be bicyclic, including, but not limited to, 5, 5-membered rings, such as a hexahydrocyclopento [c] pyrrol-2 (1H)-yl ring, or a 5,6-membered bicyclic ring, such as a hexahydropyrrolo [1, 2-a] pyrazin-2 (1H)-yl ring. The nitrogen atom-containing ring may be partially unsaturated, i.e., it may contain one or more double bonds, including, but not limited to, 2,5-dihydro-1H-pyrrolyl, 4H-[1, 3, 4] thiadiazinyl, 4, 5-dihydrooxazolyl or 4H-[1, 4] thiazinyl, or it may be benzo-fused, including, but not limited to, dihydroisoquinolinyl. According to the present disclosure, the heterocyclic groups are non-aromatic. When the 3-12 membered heterocyclic groups are attached to other

groups to form the compound provided by the present disclosure, the carbon atoms on the 3-12 membered heterocyclic groups may be attached to other groups, or the heterocyclic atoms on the 3-12 membered heterocyclic groups may be attached to other groups. For example, when the 3-12 membered heterocyclic group is selected from a piperazinyl group, the nitrogen atom of the piperazinyl group may be attached to other groups. Alternatively, when the 3-12 membered heterocyclic group is selected from a piperidinyl group, the nitrogen atom on the piperidinyl ring and the carbon atom in its para position may be attached to other groups.

The term "aryl groups of $C_{6-14}$" should be understood to refer to aromatic or partially aromatic monovalent monocyclic, bicyclic or tricyclic hydrocarbon rings having 6 to 14 carbon atoms, such as an aromatic or partially aromatic monovalent monocyclic, bicyclic or tricyclic hydrocarbon ring having 6, 7, 8, 9, 10, 11, 12, 13 or 14 carbon atoms, in particular a ring ("aryl group of $C_6$") having 6 carbon atoms, i.e., phenyl; or biphenyl, or a ring ("aryl group of $C_9$") having 9 carbon atoms, such as indanyl or indenyl, or a ring ("aryl group of $C_{10}$") having 10 carbon atoms, such as tetrahydronaphthyl, dihydronaphthyl or naphthyl, or a ring ("aryl group of $C_{13}$") having 13 carbon atoms, such as fluorenyl, or a ring ("aryl group of $C_{14}$") having 14 carbon atoms, such as anthryl. When the aryl groups of $C_{6-20}$ are substituted, it may be mono-or poly-substituted. Also, the substitution site thereof is not limited, and may be, for example, ortho-, para-, or meta-substitution.

The term "5-14 membered heteroaryl groups" or "5-14 membered heteroaryl groups" should be understood to include such monovalent monocyclic, bicyclic or tricyclic aromatic ring systems, which are aromatic or partially aromatic, have 5 to 14 ring atoms and contain 1 to 5 heteroatoms independently selected from N, O and S. For example, it may have 5, 6, 7, 8, 9, 10, 11, 12, 13 or 14 ring atoms, in particular 5, 6, 9 or 10 carbon atoms, and contain 1 to 5, preferably, 1 to 3 heteroatoms independently selected from N, O and S; and in addition, in each case, it may be benzo-fused. In particular, the heteroaryl groups are selected from the group consisting of thienyl, furyl, pyrrolyl, oxazolyl, thiazolyl, imidazolyl, pyrazolyl, isoxazolyl, isothiazolyl, oxadiazolyl, triazolyl, thiadiazolyl, and thi-4H-pyrazolyl, and benzo derivatives thereof, for example, benzofuryl, benzothienyl, benzoxazolyl, benzisoxazolyl, benzimidazolyl, benzotriazolyl, indazolyl, indolyl, isoindolyl, and the like; or pyridyl, pyridazinyl, pyrimidinyl, pyrazinyl, and triazinyl, and benzo derivatives thereof, such as quinolyl, quinazolinyl, and isoquinolyl; or acridyl, indolazinyl, purinyl, and benzo derivatives thereof; or cinolinyl, phthalazinyl, quinazolinyl, quinoxalinyl, naphthyridinyl, pteridyl, carbazolyl, acridinyl, phenazinyl, phenothiazinyl, phenoxazinyl and the like. When the 5-14 membered heteroaryl groups are attached to other groups to form the compound provided by the present disclosure, the carbon atoms on the 5-14 membered heteroaryl group rings may be attached to other groups, or the heteroatoms on the 5-14 membered heteroaryl group rings may be attached to other groups. When the 5-14 membered heteroaryl groups are substituted, it may be mono-or poly-substituted. Also, the substitution site thereof is not limited, for example, the hydrogen attached to the carbon atoms on the heteroaryl group rings may be substituted, or the hydrogen attached to the heteroatoms on the heteroaryl group rings may be substituted.

Unless otherwise specified, a heterocyclyl, heteroaryl or sub-heteroaryl group includes all possible isomeric forms thereof, such as positional isomers thereof. Thus, for some illustrative non-limiting examples, they may include forms of substitution or bonding to other groups at one, two, or more sites such as 1-, 2-, 3-, 4-, 5-, 6-, 7-, 8-, 9-, 10-, 11-, and 12- (if present), including pyridine-2-yl, pyridylidene-2-yl, pyridine-3-yl, pyridylidene-3-yl, pyridine-4-yl, and pyridylidene-4-yl; thiophene or thenylidene includes thiophene-2-yl, thenylidene-2-yl, thiophene-3-yl, and thenylidene-3-yl; and pyrazole-1-yl, pyrazole-3-yl, pyrazole-4-yl, pyrazole-5-yl.

The term "effective amount" or "therapeutically effective amount" refers to an amount of a compound described herein that is sufficient to effect the intended use, including but not limited to the disease treatment as defined below. The therapeutically effective amount may vary depending on the intended use (in vitro or in vivo), or the subject and disease being treated, such as the weight and age of the subject, the severity of the disease, and the mode of administration, which can be easily determined by a person generally skilled in the art. The specific dosage will vary depending upon the particular compound selected, the dosing regimen pursuant to which it is administered, whether it is administered in combination with other compounds, the timing of administration, the tissue to which it is administered, and the physical delivery system being carried.

The term "solvates" are those forms of the compounds provided by the present disclosure which, in solid or liquid states, form complexes by coordination with solvent molecules. Hydrates are specific forms of the solvates, in which the coordination is achieved in water. In the present disclosure, the preferred solvates are hydrates. Further, the pharmaceutically acceptable solvates (hydrates) of the compound as shown in formula I of the present disclosure refers to co-crystals and clathrates formed by the compound I with one or more molecules of water or other solvents based on stoichiometry. The solvents that can be used for the solvates include, but are not limited to, water, methanol, ethanol, ethylene glycol, and acetic acid.

The term "prodrugs" or "drug precursors" refer to compounds converted in vivo to the compounds as shown in the foregoing general formula or specific compound. Such conversion is affected by hydrolysis of the prodrugs in blood or enzymatic conversion to a parent structure in blood or tissue. The prodrugs described in the present disclosure may be esters. In the present disclosure, the esters that may be used as the prodrugs include phenyl esters, aliphatic esters, acyloxymethyl esters, carbonates, carbamates and amino-acid esters. For example, one of the compounds provided by

the present disclosure contains a hydroxy/carboxy group, i.e., it can be acylated to obtain the compound in a prodrug form. Other prodrug forms include phosphate esters, such as those obtained by phosphorylation of the hydroxyl group on the parent.

Beneficial effects

The isoindoline-1, 3-diketone compound provided by the present disclosure has a prominent and highly inhibited biological response to PDE4 enzyme, which further increases cAMP level or inhibits factors such as TNF-$\alpha$, thereby effectively treating psoriasis, psoriatic arthritis, scalp psoriasis, Behcet's disease, atopic dermatitis (AD), vitiligo, seborrheic dermatitis, stasis dermatitis, palmoplantar abscess, chronic obstructive pulmonary disease (COPD), acute pneumonia (ARDS), virus-induced lung diseases, and respiratory inflammatory diseases. The compound provided by the present disclosure has an outstanding inhibitory activity of the isoindoline-1, 3-diketone series compounds on the biological enzyme PDE4, and has the beneficial effect of higher efficacy.

## Detailed Description

The technical solution of the present disclosure will be described in further detail below with reference to specific examples. It should be understood that the following examples are only exemplary to illustrate and explain the present disclosure, and should not be construed as limiting the scope of the present disclosure. All technologies implemented based on the above content of the present disclosure are covered within the intended protection scope of the present disclosure.

Unless otherwise specified, the raw materials and reagents used in the following examples are commercially available or can be prepared by known methods.

Example 1. (S)-2-[1-(3-ethoxy-4-methoxyphenyl)-2-alkylsulfonyl ethyl]-4-(y-chlorobutyramido)-5-hexyl isoindoline-1, 3-diketone

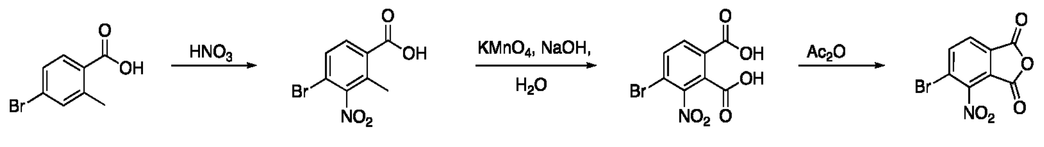


1-1 1-2 1-3 1-4

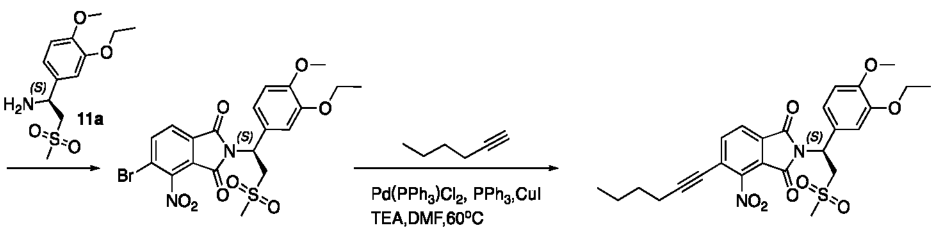


1-5 1-6

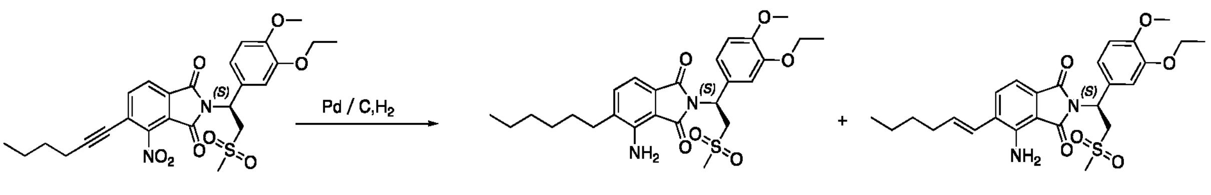


1-6

1-7 1-7A

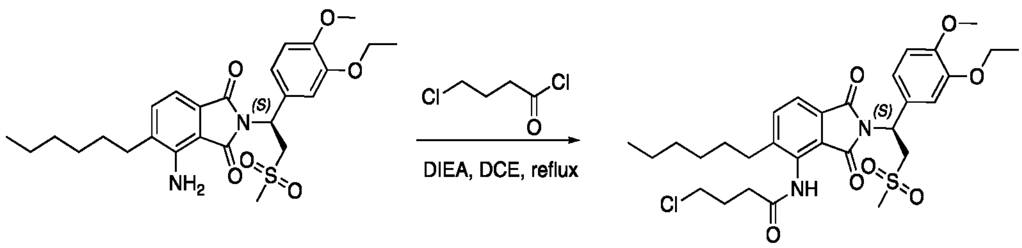


1-7

Example 1

Synthesis of compound 1-2: A compound 1-1 (5g, 23.25mmol, 1eq) was added in batches to fuming nitric acid (1.47g, 23.25mmol, 16mL, 1eq) at 0°C, after addition, the resulting mixture was further stirred at 0°C for 1h, the yellow suspension was formed. The mixture was stirred and poured into ice water (100mL), the suspension was filtered, the filter cake was washed with water (30mL), the washed filter cake was dissolved in ethyl acetate (100mL), and the product was dried with $Na_2SO_4$ and concentrated in vacuo. A compound 1-2 (5.3g, crude) was obtained as a yellow solid.

Synthesis of compound 1-3: The compound 1-2 (5.3g, 6.73mmol, 1eq) was dissolved in $H_2O$ (60mL), NaOH (2.42g, 60.53mmol, 9eq) was added into the solution, and the obtained mixture was heated to 80°C and added with $KMnO_4$ (25.51g, 161.42mmol, 24eq) in batches within 3h. After the addition was completed, stirring was continued for 30min, suction filtration was carried out, the solid was washed with hot water (30mL*3), the aqueous phase was cooled with ice water, and the pH was adjusted to 2 with 2M HCl. The product was extracted with ethyl acetate (100mL*2), washed with a saturated saline solution after combination of organic phases, dried with $Na_2SO_4$, filtered, and concentrated to obtain a compound 1-3 (1.9g, crude) as a yellow solid.

Synthesis of compound 1-4: The compound 1-3 (1.9g, 3.93mmol, 1eq) was dissolved in $Ac_2O$ (21.80g, 213.54mmol, 20mL, 54.33eq), stirred at 140°C for 16h, and concentrated to obtain a compound 1-4 (1.6g, crude) as a light brown solid.

Synthesis of compound 1-5: The compound 1-4 (1.6g, 3.53mmol, 1eq) and a compound 11a (1.54g, 5.65mmol, 1.6eq) were dissolved in AcOH (20mL) and stirred at 120°C for 18h. The reaction solution was concentrated to obtain a crude product, and the crude product was purified by silica gel chromatography (petroleum ether: ethyl acetate=10:1 to 1:1) to obtain a compound 1-5 (1.2g, crude) as a yellow solid.

Synthesis of compound 1-6: The compound 1-5 (1.2g, 1.50mmol, 1eq), $PdCl_2(PPh3)2$ (210.83mg, 300.37μmol,

0.2eq), CuI (57.21mg, 300.37μmol, 0.2eq), DIEA (582.31mg, 4.51mmol, 784.78μL, 3eq) and 1-hexyne (370.11mg, 4.51mmol, 506.99μL, 3eq) were dissolved in DMF (12mL), the reaction solution was stirred at 60°C for 18h under $N_2$ atmosphere. The reaction mixture was added with water (15mL) and ethyl acetate (20mL), the aqueous phase was extracted with ethyl acetate (30mL*3), the combined organic layers were washed with a saturated saline solution, dried with $Na_2SO_4$, filtered, and concentrated to obtain a crude product, and the residue was purified by silica gel chromatography ($SiO_2$, PE: EtOAc=10:1 to 1:1) to obtain a compound 1-6 (376mg, yield: 47.36%) as a yellow solid.

1H-NMR(400MHz, $CDCl_3$) of the compound 1-6: δ = 7. 87 (d, J = 8.0 Hz, 1H), 7.81 (d, J=8.0 Hz, 1H), 7.11 - 7.06 (m, 2H), 6.83 (d, J=8.4 Hz, 1H), 5.86 (dd, J=4.4, 10.4 Hz, 1H), 4.50 (dd, J=10.4, 14.0 Hz, 1H), 4.10 (q, J=7.2 Hz, 2H), 3.87 - 3.84 (m, 3H), 3.70 (dd, J=4.4, 14.4 Hz, 1H), 2.89 - 2.84 (m, 3H), 2.44 (t, J=7.2 Hz, 2H), 1.62 - 1.56 (m, 2H), 1.49 - 1.41 (m, 5H), 0.97 - 0.91 (m, 3H).

Synthesis of compound 1-7: The compound 1-6 (370.00mg, 700.00μmol, 1eq) was dissolved in methanol (10mL), Pd/C (100mg, 10% purity) was added under nitrogen atmosphere, and the mixture was replaced with hydrogen for 3 times under vacuum condition, and stirred at 60° C under hydrogen atmosphere (50 Psi) for 16h. The reaction solution was filtered with diatomite to remove solids, and the filter cake was washed with EtOAc, concentrated, and purified by using prep-HPLC (a formic acid system) to obtain a compound 1-7 (128mg, yield: 36.38%) and a compound 1-7A (25mg, yield: 7.13%).

$^1$H-NMR(400MHz, $CDCl_3$) of the compound 1-7: δ = 7.25 (br s, 1H), 7.15 - 7.07 (m, 3H), 6.82 (d, J=8.0 Hz, 1H), 5.83 (dd, J=5.2, 9.6 Hz, 1H), 5.28 (s, 2H), 4.52 (dd, J=9.2, 14.4 Hz, 1H), 4.10 (q, J=7.2 Hz, 2H), 3.84 (s, 3H), 3.79 (dd, J=5.2, 14.4 Hz, 1H), 2.80 (s, 3H), 2.51 (t, J=8.0 Hz, 2H), 1.64 - 1.58 (m, 2H), 1.46 (t, J=7.2 Hz, 3H), 1.35-1.29 (m, 6H), 0.90 - 0.87 (m, 3H).

$^1$H-NMR(400MHz, $CDCl_3$) of the compound 1-7A: δ = 7.43 (d, J=7.2 Hz, 1H), 7.16 - 7.10 (m, 3H), 6.84 (d, J=8.4 Hz, 1H), 6.36 - 6.30 (m, 1H), 6.26 - 6.18 (m, 1H), 5.85 (dd, J=5.2, 9.6 Hz, 1H), 5.36 (s, 2H), 4.52 (dd, J=9.6, 14.4 Hz, 1H), 4.12 (q, J=7.2 Hz, 2H), 3.85 (s, 3H), 3.80 (dd, J=4.8, 14.4 Hz, 1H), 2.81 (s, 3H), 2.30 - 2.23 (m, 2H), 1.51 - 1.447 (m, 5H), 1.43-1.35 (m, 2H),0.97 - 0.92 (m, 3H).

Synthesis of Example 1:

The compound 1-7 (40mg, 79.58μmol, 1eq) and chlorobutyryl chloride (11.22mg, 79.58μmol, 8.91μL, 1eq) were dissolved in DCE (2mL), DIEA (10.29mg, 79.58μmol, 13.86μL, 1eq) was added to the reaction solution, and the mixture was stirred at 50°C for 3h. The reaction solution was concentrated and purified by using prep-HPLC (a formic acid system) to obtain a white solid of Example 1 (14.04mg, yield: 29.06%).

$^1$H-NMR (400MHz, $CDCl_3$) δ =7.94 (br s, 1H), 7.65 - 7.57 (m, 2H), 7.07 (d, J=2.0 Hz, 1H), 7.09 (s, 1H), 6.83 (d, J=8.4 Hz, 1H), 5.85 (dd, J=4.4, 10.0 Hz, 1H), 4.51 (dd, J=10.4, 14.4 Hz, 1H), 4.10 (q, J=6.8 Hz, 2H), 3.85 (s, 3H), 3.74 (dd, J=4.4, 14.4 Hz, 1H), 3.69 (t, J=6.4 Hz, 2H), 2.84 (s, 3H), 2.74 - 2.60 (m, 4H), 2.24 (quin, J=6.8 Hz, 2H), 1.63 - 1.56 (m, 2H), 1.46 (t, J=7.2 Hz, 3H), 1.28 (br s, 6H), 0.92 - 0.82 (m, 3H).

LCMS: 607.0([M+H]+).

Example 2. (S)-2-[1-(3-ethoxy-4-methoxyphenyl)-2-alkylsulfonyl ethyl]-4-(y-chlorobutyramido)-5-hexene (-1) yl isoindoline-1, 3-diketone

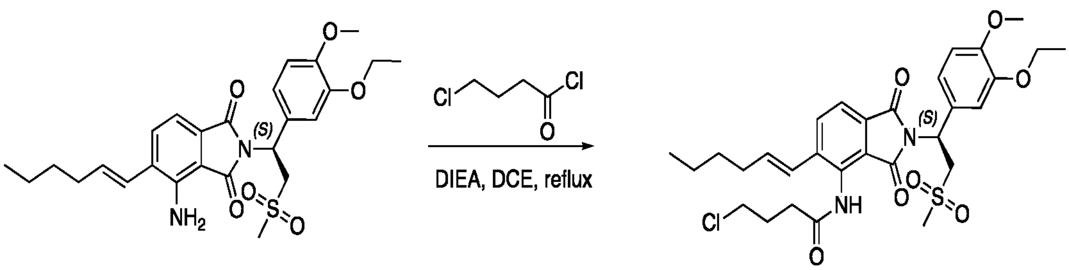


1-7A

Example 2

The compound 1-7A (20.00mg, 39.95μmol, 1eq) and chlorobutyryl chloride (6.20mg, 43.95μmol, 4.92μL, 1.1eq) were dissolved in DCE (2mL), DIEA (5.16mg, 39.95μmol, 6.96μL, 1eq) was added to the reaction solution, and the mixture was stirred at 50°C for 16h. The mixture was concentrated in reduced pressure and the crude product was purified by using prep-HPLC (a formic acid system) to obtain a white solid of Example 2 (5.13mg, yield: 21.22%).

$^1$H-NMR (400MHz, $CDCl_3$) $\delta$ = 8.03 (br s, 1H), 7.83 (d, J=7.6 Hz, 1H), 7.62 (d, J=7.6 Hz, 1H), 7.11 - 7.06 (m, 2H), 6.83 (d, J=8.8 Hz, 1H), 6.42 - 6.28 (m, 2H), 5.85 (dd, J=4.4, 10.4 Hz, 1H), 4.51 (br dd, J=10.4, 14.4 Hz, 1H), 4.10 (q, J=6.8 Hz, 2H), 3.85 (s, 3H), 3.77 - 3.62 (m, 3H), 2.83 (s, 3H), 2.69 (br s, 2H), 2.23 (quin, J=7.2 Hz, 4H), 1.50 - 1.43 (m, 5H), 1.40 - 1.33 (m, 2H), 0.96 - 0.87 (m, 3H).

LCMS: 605.1([M+H]+).

Example 3. (S)-2-[1-(3-ethoxy-4-methoxyphenyl)-2-alkylsulfonyl ethyl]-4-cyclopropanamido-5-octyl isoindoline-1, 3-diketone

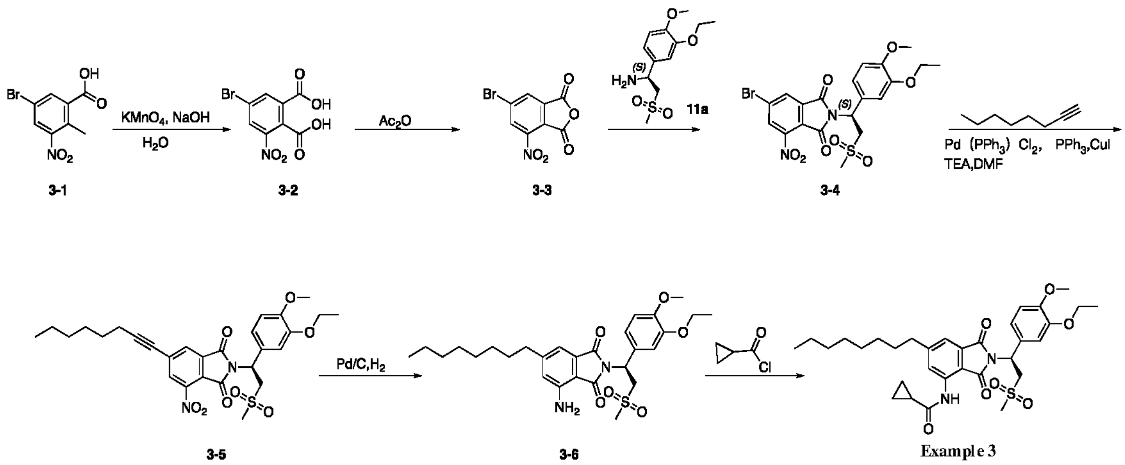


3-1 3-2 3-3 3-4 3-5 3-6 Example 3

Synthesis of compound 3-2: A compound 3-1 5-bromo-2-methyl-3-nitrobenzoic acid (20g, 76.91mmol, 1eq.) was dissolved in $H_2O$ (20mL), NaOH (9.23g, 230.73gmmol, 3eq) was added into the obtained solution, the mixture was heated up to 80°C, and $KMnO_4$ (97.24g, 615.29mmol, 8eq) was added in batches within 3h; and after the addition was finished, stirring was continued for 30min at 80°C, the reaction mixture was filtered and the filter cake was washed with hot water (300mL*3). The aqueous phase was cooled with ice water, and the pH was adjusted to 1 with 2M HCl. The product was extracted with EtOAc (400mL*3), washed with a saturated saline solution after combination of organic phases, dried with $Na_2SO_4$, filtered, and concentrated to obtain a compound 3-2 (5g, yield: 22.42%) as a yellow solid.

$^1$H-NMR (400MHz, DMSO-d6) $\delta$ = 13.98 (br s, 2H) , 8.52 (d, J = 2.0 Hz, 1H), 8.33 (d, J = 2.0 Hz, 1H).

Synthesis of compound 3-3: The compound 3-2 (5g, 17.24mmol, 1eq) was dissolved in $Ac_2O$ (20mL), and stirred at 140°C for 16h. The reaction mixture was concentrated to obtain a crude product, and the crude product was purified by silica gel chromatography (petroleum ether: ethyl acetate=100/0 to 1/1) to obtain a compound 3-3 (4.5g, crude) as a yellow solid.

Synthesis of compound 3-4: The compound 3-3 (4g, 14.71mmol, 1eq) and a compound 11a (4.02g, 14.71mmol, 1eq.) were dissolved in AcOH (80mL), and stirred at 120°C for 16h. The reaction mixture was concentrated to obtain a crude product, and the crude product was purified by silica gel chromatography (petroleum ether: ethyl acetate=100/0 to 1/1) to obtain a compound 3-4 (2.9g, yield: 37.40%) as a yellow solid.

$^1$H-NMR (400MHz, $CDCl_3$) $\delta$ = 8.24 (d, J = 1.6 Hz, 1H) , 8.21 (d, J = 1.6 Hz, 1H) , 7.13 - 7.06 (m, 2H), 6.84 (d, J = 7.6 Hz, 1H), 5.91 (dd, J = 11.2, 4.4 Hz, 1H), 4.57 (dd, J = 14.4, 11.2 Hz, 1H) , 4.14 - 4.07 (m, 2H), 3.85 (s, 3H) , 3.66 (dd, J = 14.4, 4.0 Hz, 1H), 2.91 (s, 3H) , 1.47 (t, J = 7.2 Hz, 3H).

Synthesis of compound 3-5: The compound 3-4 (400.00mg, 758.52$\mu$mol, 1eq), $PdCl_2(PPh3)2$ (106.48mg, 151.70$\mu$mol, 0.2eq), CuI (28.89mg, 151.70$\mu$mol, 0.2eq), DIEA (294.09mg, 2.28mmol, 396.35$\mu$L, 3eq) and 1-octyne (417.93mg, 3.79mmol, 5eq) were dissolved in DMF (4mL), the reaction solution was stirred at 60°C under $N_2$ atmosphere for 16h, and water was (10mL) added to the reaction system. The product was extracted with ethyl acetate (10mL*3), washed with a saturated saline solution after combination of organic phases, dried with $Na_2SO_4$, filtered, and concentrated to obtain a crude product; and the crude product was purified by silica gel chromatography (petroleum ether: ethyl acetate=100:1 to 1: 1) to obtain a compound 3-5 (130mg, yield: 30.79%) as a yellow solid.

$^1$H-NMR (400MHz $CDCl_3$ ) $\delta$ = 7.13 - 7.07 (m, 3H), 6.85 - 6.80 (m, 2H), 5.82 (dd, J = 9.6, 5.2 Hz, 1H), 5.15 (s, 2H), 4.49 (dd, J = 14.4, 9.6 Hz, 1H), 4.10 (d, J = 7.2 Hz, 2H) , 3.84 (s, 3H) , 3.78 (dd, J = 14.4, 5.2 Hz, 1H) , 2.80 (s, 3H), 2.39 (t, J = 7.2 Hz, 2H) , 1.63 - 1.56 (m, 2H), 1.46 (t, J = 7.2 Hz, 3H) , 1.32 - 1.30 (m, 3H) , 0.88 - 0.87 (m, 4H).

Synthesis of compound 3-6: The compound 3-5 (130mg, 233.55$\mu$mol, 1eq) was dissolved in EtOAc (15mL), Pd/C (140mg, 10%) was added under nitrogen atmosphere, and the mixture was replaced with hydrogen for 3 times

under vacuum condition, and stirred at 60° C under hydrogen atmosphere (50 Psi) for 16h. The reaction solution was filtered with diatomite to remove solids, the filter cake was washed with EtOAc, and the filtrate was concentrated to obtain a compound 3-6 (100mg, yield: 80.68%) as a yellow solid.

$^1$H-NMR (400MHz $CDCl_3$) δ =7.14 - 7.08 (m, 2H) , 7.00 - 6.96 (m, 1H) , 6.82 (d, J = 8.4 Hz, 1H) , 6.62 (s, 1H), 5.82 (dd, J = 5.2, 9.6 Hz, 1H), 5.30 (s, 1H), 5.12 (s, 2H), 4.51 (dd, J = 14.8, 9.6 Hz, 1H) , 4.11 (q, J = 7.2 Hz, 2H) , 3.84 (s, 3H) , 3.79 (dd, J = 14.8, 5.2 Hz, 1H) , 2.80 (s, 3H) , 2.57 (t, J = 7.6 Hz, 2H) , 2.05 - 1.98 (m, 1H) , 1.57 (br s, 2H) , 1.46 (t, J = 7.2 Hz, 3H) , 1.26 (br d, J = 6.8 Hz, 8H) , 0.89 - 0.86 (m, 3H).

Synthesis of Example 3:

The compound 3-6 (10mg, 18.84μmol, 1eq) and cyclopropionyl chloride (9.85mg, 94.22μmol, 8.56μL, 5eq) were dissolved in DCE (1mL), and DIEA (19.48mg, 150.75μmol, 26.26μL, 8eq) was added into the reaction solution, and stirred at 90°C for 2h. The reaction mixture was concentrated, and the crude product was purified by using prep-HPLC (a formic acid system) to obtain a white solid of Example 3 (8.2mg, yield: 72.68%).

$^1$H-NMR (400MHz, $CDCl_3$) δ = 9.60 (s, 1H) , 8.58 (s, 1H) , 7.30 (s, 1H) , 7.12 - 7.08 (m, 2H) , 6.86 - 6.82 (m, 1H) , 5.86 (dd, J = 10.4, 4.4 Hz, 1H) , 4.55 (dd, J = 14.4, 10.4 Hz, 1H), 4.11 (q, J = 6.8 Hz, 2H), 3.85 (s, 3H), 3.74 (dd, J = 14.4, 4.4 Hz, 1H) , 2.86 (s, 3H) , 2.69 - 2.64 (m, 2H), 1.67 - 1.58 (m, 3H) , 1.47 (t, J = 7.2 Hz, 3H) , 1.26 (br d, J = 12.4 Hz, 10H) , 1.12 (quin, J = 3.6 Hz, 2H), 0.97 - 0.91 (m, 2H), 0.89 - 0.84 (m, 3H).

LCMS: 599.1 [M+H]+.

Example 4. (S)-2-[1-(3-ethoxy-4-methoxyphenyl)-2-alkylsulfonyl ethyl]-4-acetamidomethyl-7-pentyl isoindoline-1, 3-diketone

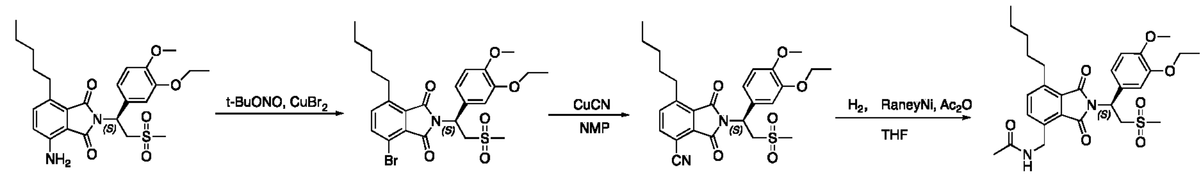


5-5 4-2 4-3 Example 4

Example 4 was synthesized via synthetic route 6.

LCMS: 545.1 ([M+H]+).

Example 5. (S)-2-[1-(3-ethoxy-4-methoxyphenyl)-2-alkylsulfonyl ethyl]-4-(y-chlorobutyramido)-7-pentyl isoindoline-1, 3-diketone

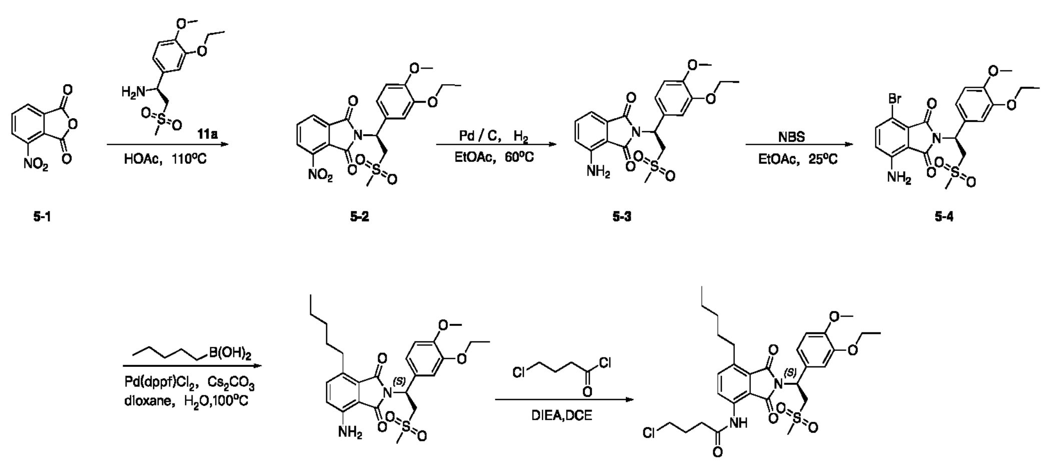


5-1 5-2 5-3 5-4

5-5 Example 5

Synthesis of compound 5-2: A compound 5-1 (21.19g, 109.75mmol, 1eq) and the compound 11a (30g, 109.75mmol, 1eq) were dissolved in HOAc (500mL), and stirred at 120° C for 16h. The reaction mixture was concentrated to obtain a crude product, and the crude product was purified by silica gel chromatography (petroleum ether: ethyl acetate=100/0 to 1/1) to obtain a compound 5-2 (43.3g, yield: 89.98%) as a yellow solid.

$^1$H-NMR (400MHz, $CDCl_3$) $\delta$ = 8.13 - 8.07 (m, 2H), 7.92 - 7.86 (m, 1H), 7.15 - 7.09 (m, 2H), 6.84 (d, J=8.0 Hz, 1H), 5.93 (dd, J=4.0, 10.8 Hz, 1H), 4.58 (dd, J=10.8, 14.4 Hz, 1H), 4.15 - 4.07 (m, 2H), 3.85 (s, 3H), 3.70 (dd, J=4.4, 14.4 Hz, 1H), 2.90 (s, 3H), 1.47 (t, J=7.2 Hz, 3H).

Synthesis of compound 5-3: The compound 5-2 (50.00g, 111.15mmol, 1eq) was dissolved in EtOAc (400mL), Pd/C (9g, 10%) was added under nitrogen atmosphere, and the mixture was replaced with hydrogen for 3 times under vacuum condition, and stirred at 60° C under hydrogen atmosphere (50 Psi) for 12h. The reaction solution was filtered with diatomite to remove solids, the filter cake was washed with EtOAc, and the filtrate was concentrated to obtain a compound 5-3 (4g, yield: 85.73%) as a yellow solid.

$^1$H-NMR (400MHz, $CDCl_3$) $\delta$ = 7.37 (dd, J=7.2, 8.4 Hz, 1H), 7.13 - 7.10 (m, 2H), 7.10 - 7.08 (m, 1H), 6.83 - 6.78 (m, 2H), 5.83 (dd, J=4.8, 9.8 Hz, 1H), 5.20 (s, 2H), 4.54 - 4.47 (m, 1H), 4.12 - 4.06 (m, 2H), 3.83 (s, 3H), 3.78 (dd, J=5.2, 14.8 Hz, 1H), 2.79 (s, 3H), 1.47 - 1.42 (m, 3H).

Synthesis of compound 5-4: The compound 5-3 (47.4g, 113.27mmol, 1eq) was dissolved in ethyl acetate (500mL), added with NBS (20.16g, 113.27mmol, 1eq), and stirred at 25° C for 16h. The reaction solution was concentrated to obtain a crude product, and the crude product was purified by silica gel chromatography (petroleum ether: ethyl acetate=100/0 to 1/1) to obtain a compound 5-4 (26.52g, yield: 42.07%) as a yellow solid.

$^1$H-NMR (400MHz, $CDCl_3$) $\delta$ = 7.44 (d, J=8.4 Hz, 1H), 7.16 - 7.08 (m, 2H), 6.84 (d, J=8.0 Hz, 1H), 6.71 (d, J=8.4 Hz, 1H), 5.86 (dd, J=4.4, 10.4 Hz, 1H), 5.55 - 5.15 (m, 2H), 4.55 (dd, J=10.4, 14.4 Hz, 1H), 4.15 - 4.02 (m, 2H), 3.86 (s, 3H), 3.77 (dd, J=4.8, 14.4 Hz, 1H), 2.85 (s, 3H), 1.47 (t, J=7.2 Hz, 3H).

Synthesis of compound 5-5: The compound 5-4 (13.98g, 28.11mmol, 1eq), $Cs_2CO_3$ (27.47g, 84.33mmol, 3eq), and amyl boric acid (6.52g, 56.22mmol, 2eq) were dissolved in dioxane (150mL) and water (30mL), Pd(dppf)$Cl_2$ (4.11g, 5.62mmol, 0.2eq) was added under $N_2$ atmosphere, the mixture was stirred at 60°C under $N_2$ atmosphere for 18h, the reaction solution was concentrated, water (50mL) and ethyl acetate (50mL) were added into the reaction solution, extraction was performed with ethyl acetate (50mL*3), the combined organic layers were washed with a saturated saline solution, dried with $Na_2SO_4$, filtered, and concentrated to obtain a crude product, and the crude product was purified by silica gel chromatography ($SiO_2$, petroleum ether: ethyl acetate=100/0 to 1/1) to obtain a compound 5-5 (4.78g, yield: 34.80%) as a yellow solid.

$^1$H-NMR (400MHz, $CDCl_3$) $\delta$ = 7.18 (d, J=8.4 Hz, 1H), 7.16 - 7.10 (m, 2H), 6.83 (d, J=8.4 Hz, 1H), 6.75 (d, J=8.4 Hz, 1H), 5.83 (dd, J=5.2, 9.6 Hz, 1H), 5.14 (s, 2H), 4.50 (dd, J=9.6, 14.4 Hz, 1H), 4.16 - 4.09 (m, 2H), 3.85 (s, 3H), 3.83 - 3.78 (m, 1H), 3.64 (t, J=6.8 Hz, 1H), 2.96 - 2.86 (m, 2H), 2.83 - 2.76 (m, 3H), 1.62 - 1.57 (m, 2H), 1.46 (t, J=7.2 Hz, 3H), 1.38 - 1.27 (m, 4H), 0.94 - 0.85 (m, 3H).

Synthesis of Example 5:

The compound 5-5 (4.04g, 8.27mmol, 1eq) and chlorobutyryl acid chloride (2.33g, 16.54mmol, 1.85mL, 2eq) were dissolved in DCE (80mL), DIEA (4.27g, 33.07mmol, 5.76mL, 4eq) was added to the reaction solution, and the mixture was stirred at 90°C for 3h. The reaction solution was concentrated, a saturated $NaHCO_3$ (20mL) aqueous solution and DCM (20mL) were added, extraction was performed with dichloromethane (3*20mL), the combined organic layers were washed with a saturated saline solution, dried with $Na_2SO_4$, filtered, and concentrated to obtain a crude product, and the crude product was purified by silica gel chromatography ($SiO_2$, petroleum ether: ethyl acetate=1/0 to 1/1) to obtain a yellow solid as Example 5 (4.12g, ee value: 96.7%, yield: 84.01%).

$^1$H-NMR (400MHz, $CDCl_3$) $\delta$ = 9.58 (s, 1H), 8.63 (d, J=8.8 Hz, 1H), 7.43 (d, J=8.8 Hz, 1H), 7.13 - 7.08 (m, 2H), 6.88 - 6.82 (m, 1H), 5.86 (dd, J=4.8, 10.0 Hz, 1H), 4.53 (dd, J=10.4, 14.4 Hz, 1H), 4.12 (q, J=7.2 Hz, 2H), 3.85 (s, 3H), 3.75 (dd, J=4.8, 14.4 Hz, 1H), 3.66 (t, J=6.4 Hz, 2H), 3.03 - 2.94 (m, 2H), 2.85 (s, 3H), 2.66 (t, J=7.2 Hz, 2H), 2.22 (quin, J=6.8 Hz, 2H), 1.65 - 1.57 (m, 2H), 1.47 (t, J=7.2 Hz, 3H), 1.36 - 1.30 (m, 4H), 0.91 - 0.86 (m, 3H).

LCMS: 593.1([M+H]+).

Example 6. (S)-2-[1-(3-ethoxy-4-methoxyphenyl)-2-alkylsulfonyl ethyl]-4-cyclopropanamido-7-pentyl isoindoline-1, 3-diketone

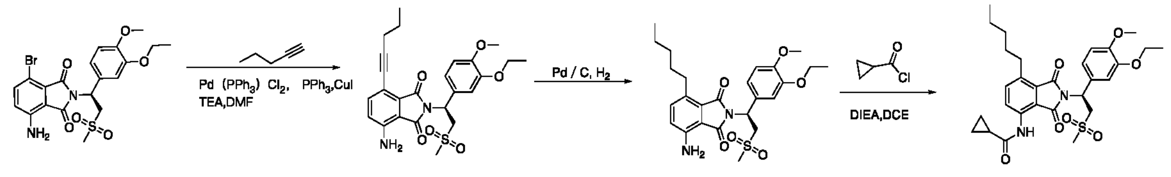


5-4 6-2 6-3 Example 6

Synthesis of compound 6-2:

The compound 5-4 (200mg, 402.12μmol, 1eq), PdCl2(PPh3)2 (56.45mg, 80.42μmol, 0.2eq), CuI (15.32mg, 80.42μmol, 0.2eq), DIEA (155.91mg, 1.21mmol, 210.12μL, 3eq), and 1-pentylene (273.91mg, 4.02mmol, 394.69μL, 10eq) were dissolved in DMF(2mL), the reaction solution was stirred at 60°C under $N_2$ atmosphere for 16h, water (5mL) was added into the reaction system, extraction was performed with ethyl acetate (50mL*3), the combined organic layers were washed with a saturated saline solution, dried with $Na_2SO_4$, filtered, and concentrated to obtain a crude product, and the crude product was purified by silica gel chromatography ($SiO_2$, petroleum ether: ethyl acetate=1/0 to 1/1) to obtain a compound 6-2 (S)-2-[1-(3-ethoxy-4-methoxyphenyl)-2-methylsulfonyl ethyl]-4-amino-6-[pentyl-1-alkynyl] isoindoline-1, 3-diketone (98mg, yield: 50.29%) as a yellow solid.

$^1$H-NMR (400MHz $CDCl_3$) δ = 7.36 (d, J = 8.4 Hz, 1H), 7.15 - 7.10 (m, 2H), 6.82 (d, J = 8.8 Hz, 1H) , 6.74 (d, J = 8.4 Hz, 1H) , 5.84 (dd, J = 9.6, 5.2 Hz, 1H) , 5.32 (d, J = 13.2 Hz, 2H) , 4.48 (dd, J = 14.4, 9.2 Hz, 1H) , 4.14 - 4.08 (m, 2H) , 3.84 (s, 3H) , 3.84 - 3.79 (m, 1H) , 2.81 - 2.77 (m, 3H) , 2.46 (t, J = 7.2 Hz, 2H) , 1.67 (sxt, J = 7.2 Hz, 2H) , 1.46 (t, J = 7.2 Hz, 3H) , 1.08 (t, J = 7.2 Hz, 3H).

Synthesis of compound 6-3:

The compound 6-2 (98mg, 202.24μmol, 1eq) was dissolved in EtOAc (10mL), Pd/C (100mg, 10%) was added under nitrogen atmosphere, and the mixture was replaced with hydrogen for 3 times under vacuum condition, and stirred at 60° C under hydrogen atmosphere (50 Psi) for 16h. The reaction solution was filtered with diatomite to remove solids, the filter cake was washed with EtOAc, and the filtrate was concentrated to obtain a compound 6-3 (60mg, yield: 60.72%) as a yellow solid.

$^1$H-NMR (400MHz $CDCl_3$) δ = 7.20 - 7.10 (m, 3H), 6.83 (d, J = 8.4 Hz, 1H) , 6.75 (d, J = 8.4 Hz, 1H), 5.84 (dd, J = 9.2, 5.2Hz, 1H), 5.14 (s, 2H) , 4.50 (dd, J = 14.8, 9.6 Hz, 1H), 4.11 (q, J = 6.8 Hz, 2H), 3.85 - 3.84 (m, 3H) , 3.84 - 3.79 (m, 1H) , 2.93 - 2.88 (m, 2H), 2.78 (s, 3H) , 1.62 - 1.55 (m, 2H), 1.46 (t, J = 7.2 Hz, 3H) , 1.35 - 1.28 (m, 4H) , 0.91 - 0.85 (m, 3H).

Synthesis of Example 6:

The compound 6-3 (12.5mg, 25.58μmol, 1eq) and cyclopropionyl chloride (13.37mg, 127.92μmol, 11.63μL, 5eq) were dissolved in DCE (1mL), and DIEA (26.45mg, 204.67μmol, 35.65μL, 8eq) was added and stirred at 90°C for 2h. The reaction solution was concentrated and purified by using prep-HPLC (a formic acid system) to obtain a white solid as Example 6 (5mg, yield: 35.11%).

$^1$H-NMR (400MHz, $CDCl_3$) δ = 9.76 (s, 1H) , 8.63 (d, J = 8.8 Hz, 1H) , 7.41 (d, J = 8.8 Hz, 1H) , 7.14 - 7.08 (m, 2H), 6.88 - 6.81 (m, 1H), 5.87 (dd, J = 10.0, 4.8 Hz, 1H) , 4.53 (dd, J = 14.4, 10.0 Hz, 1H) , 4.12 (q, J = 7.2 Hz, 2H) , 3.85 (s, 3H) , 3.76 (dd, J = 14.4, 4.4 Hz, 1H) , 3.01 - 2.94 (m, 2H) , 2.84 (s, 3H) , 1.67 - 1.58 (m, 3H) , 1.47 (t, J = 7.2 Hz, 3H) , 1.37 - 1.27 (m, 4H) , 1.15 - 1.07 (m, 2H) , 0.96 - 0.84 (m, 5H).

LCMS: 557.1 ([M+H]+).

Example 7. (S)-2-[1-(3-ethoxy-4-methoxyphenyl)-2-alkylsulfonyl ethyl]-4-acetamido-7-pentenyl (-1) isoindoline-1, 3-diketone

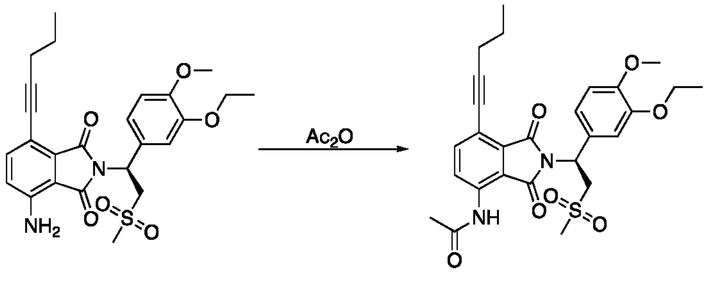


**6-2** Example 7

Synthesis of Example 7:

The compound 6-2 (180mg, 371.47/μmol, 1.0eq) was dissolved in $Ac_2O$ (1mL), the reaction solution was stirred for 3h, and then the reaction solution was concentrated and purified by using prep-HPLC (a formic acid system) to obtain a white solid as Example 7 (119mg, yield: 60.8%).

$^1$H-NMR (400MHz DMSO-$\delta_6$) δ = 9.77 (s, 1H), 8.46 (d, J = 8.7 Hz, 1H), 7.74 (d, J = 8.7 Hz, 1H), 7.08 (d, J = 1.9 Hz, 1H), 7.03 - 6.93 (m, 2H), 5.79 (dd, J = 10.4, 4.3 Hz, 1H), 4.35 (dd, J = 14.3, 10.5 Hz, 1H), 4.17 (dd, J = 14.3, 4.4 Hz, 1H), 4.04 (d, J = 7.0 Hz, 2H), 3.75 (s, 3H), 3.04 (s, 3H), 2.49(d, J = 6.9Hz, 2H), 2.22 (s, 3H), 1.62 (p, J = 7.2 Hz, 2H), 1.34 (t, J = 7.0 Hz, 3H), 1.06 (t, J = 7.4 Hz, 3H).

LCMS :527.2 ([M+H]+).

Example 8. (S)-2-[1-(3-ethoxy-4-methoxyphenyl)-2-alkylsulfonyl ethyl]-4-decanamido-7-pentenyl (-1) isoindoline-1, 3-diketone

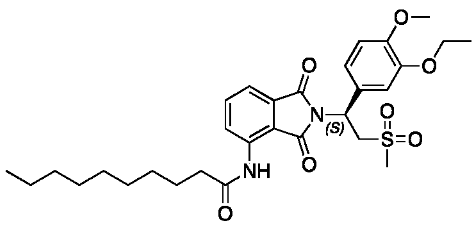

Example 8 was synthesized via synthetic route 3.

$^1$H-NMR (400MHz, $CDCl_3$) δ = 9.47 (s, 1H), 8.79 (d, J = 8.4 Hz, 1H), 7.74-7.59 (m, 1H), 7.48 (d, J = 7.2 Hz, 1H), 7.11 (dd, J = 5.9, 2.1 Hz, 2H), 6.84 (d, J = 8.9 Hz, 1H), 5.87 (dd, J = 10.4 Hz, 4.4Hz, 1H), 4.62-4.47 (m, 1H), 4.17-4.05 (m, 2H), 3.85 (s, 3H), 3.73 (s, 1H), 2.86 (s, 3H), 2.46 (d, J = 7.5 Hz, 2H), 1.84-1.69 (m, 2H), 1.50-1.44 (m, 3H), 1.43-1.17 (m, 12H), 0.94-0.75 (m, 3H).

LCMS: 573.6 ([M+H]+).

Example 9. (S)-2-[1-(3-ethoxy-4-methoxyphenyl)-2-alkylsulfonyl ethyl]-4-acetamido-7-pentyl isoindoline-1, 3-diketone

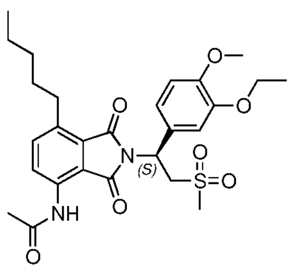

Example 9 was synthesized via synthetic route 3.

$^1$H-NMR (400MHz DMSO-$\delta_6$) $\delta$= 9.70 (s, 1H), 8.35 (d, J = 8.6 Hz, 1H), 7.62 (d, J = 8.6 Hz, 1H), 7.09 (d, J = 1.8 Hz, 1H), 7.03-6.92 (m, 2H), 5.78 (dd, J = 10.4, 4.3 Hz, 1H), 4.37 (dd, J = 14.3 Hz,10.5Hz, 1H), 4.15 (dd, J = 14.3 Hz, 4.4Hz, 1H), 4.03 (q, J = 7.0 Hz, 2H), 3.75 (s, 3H), 3.02 (s, 3H), 2.99 - 2.92 (m, 2H), 2.19 (s, 3H), 1.57(p, J = 7.3 Hz, 2H), 1.38-1.25 (m, 7H), 0.87 (t, J = 6.9 Hz, 3H).

LCMS: 531.2 ([M+H]+).

Example 10. (S)-2-[1-(3-ethoxy-4-methoxyphenyl)-2-alkylsulfonyl ethyl]-4-butylamido-7-pentyl isoindoline-1, 3-diketone

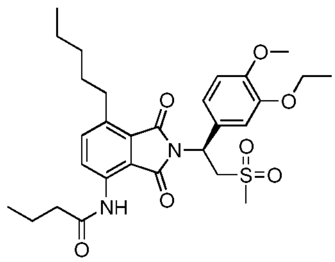

Example 10 was synthesized via synthetic route 3.

$^1$H-NMR (400MHz, $CDCl_3$) $\delta$ = 9.54 (s, 1H) , 8.66 (d, J = 8.4 Hz, 1H) , 7.42 (d, J = 8.4 Hz, 1H) , 7.15 - 7.06 (m, 2H), 6.84 (d, J = 8.8 Hz, 1H), 5.85 (dd, J = 4.4, 10.0 Hz, 1H), 4.52 (dd, J = 10.4, 14.4 Hz, 1H) , 4.11 (q, J = 6.8 Hz, 2H) , 3.85 (s, 3H) , 3.76 (dd, J = 4.4, 14.4 Hz, 1H) , 3.03 - 2.94 (m, 2H), 2.84 (s, 3H) , 2.42 (t, J = 7.6 Hz, 2H) , 1.78 (qd, J = 7.2, 14.8 Hz, 2H) , 1.62 - 1.57 (m, 2H) , 1.47 (t, J = 7.2 Hz, 3H) , 1.37 - 1.28 (m, 4H) , 1.02 (t, J = 7.2 Hz, 3H) , 0.94 - 0.82 (m, 3H).

LCMS: 559.1([M+H]+).

Example 11. (S)-2-[1-(3-ethoxy-4-methoxyphenyl)-2-alkylsulfonyl ethyl]-4-isovaleramido-7-butyl isoindoline-1, 3-diketone

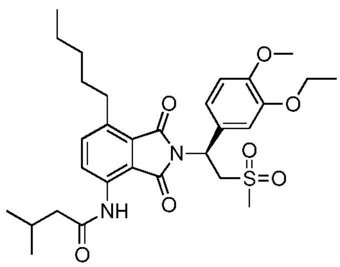

$^1$H-NMR (400MHz, $CDCl_3$) $\delta$ = 9.52 (s, 1H) , 8.67 (d, J = 8.4 Hz, 1H) , 7.42 (d, J = 8.8 Hz, 1H) , 7.14 - 7.08 (m, 2H), 6.85 (d, J = 8.4 Hz, 1H), 5.85 (dd, J = 4.8, 10.0 Hz, 1H), 4.52 (dd, J = 10.0, 14.4 Hz, 1H) , 4.11 (q, J = 7.2 Hz, 2H) , 3.85 (s, 3H) , 3.77 (dd, J = 4.8, 14.4 Hz, 1H) , 3.03 - 2.93 (m, 2H), 2.83 (s, 3H) , 2.34 - 2.28 (m, 2H), 2.28 - 2.19 (m, 1H), 1.64 - 1.58 (m, 2H), 1.47 (t, J = 7.2 Hz, 3H) , 1.37 - 1.29 (m, 4H) , 1.03 (d, J = 6.4 Hz, 6H) , 0.92 - 0.85 (m, 3H).

LCMS: 573.1 ([M+H]+).

Example 12. (S)-2-[1-(3-ethoxy-4-methoxyphenyl)-2-alkylsulfonyl ethyl]-4-caproamido-7-butyl isoindoline-1, 3-diketone

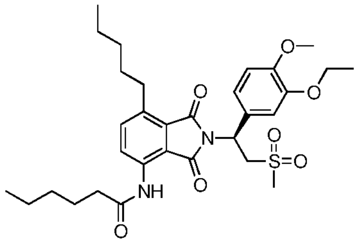

$^1$H-NMR (400MHz, $CDCl_3$) $\delta$ = 9.54 (br s, 1H) , 8.66 (br d, J = 8.4 Hz, 1H), 7.42 (br d, J = 8.8 Hz, 1H) , 7.19 - 7.03 (m, 2H) , 6.84 (br d, J = 8.4 Hz, 1H) , 5.85 (br dd, J = 4.0, 9.2 Hz, 1H), 4.58 - 4.44 (m, 1H) , 4.18 - 4.04 (m, 2H), 3.85 (s, 3H) , 3.76 (br dd, J = 4.0, 14.4 Hz, 1H), 2.98 (br t, J = 7.2 Hz, 2H) , 2.84 (s, 3H) , 2.44 (br t, J = 7.2 Hz, 2H) , 1.75 (br s, 2H) , 1.60 (br s, 2H), 1.47 (br t, J = 6.8 Hz, 3H) , 1.35 (br d, J = 16.4 Hz, 8H) , 0.95 - 0.85 (m, 6H).

LCMS: 587.1 ([M+H]+).

Example 13. (S)-2-[1-(3-ethoxy-4-methoxyphenyl)-2-alkylsulfonyl ethyl]-4-propanamido-7-pentyl isoindoline-1, 3-diketone

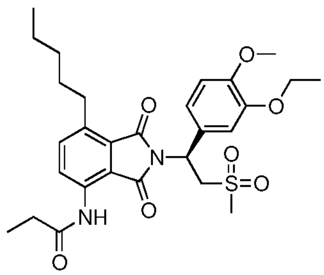

$^1$H-NMR (400MHz, $CDCl_3$) $\delta$= 9.56 (s, 1H), 8.66 (d, J = 8.8 Hz, 1H), 7.42 (d, J = 8.8 Hz, 1H), 7.15 - 7.06 (m, 2H), 6.84 (d, J = 8.8 Hz, 1H), 5.85 (dd, J = 4.8, 10.0 Hz, 1H), 4.52 (dd, J = 10.0, 14.4 Hz, 1H) , 4.15 - 4.08 (m, 2H) , 3.85 (s, 3H) , 3.76 (dd, J = 4.8, 14.4 Hz, 1H) , 3.02 - 2.94 (m, 2H) , 2.84 (s, 3H) , 2.48 (q, J = 7.6 Hz, 2H) , 1.63 - 1.58 (m, 2H) , 1.47 (t, J = 7.2 Hz, 3H) , 1.33 - 1.25 (m, 7H) , 0.89 - 0.86 (m, 3H).

LCMS: 545.1 ([M+H]+).

Example 14. (S)-2-[1-(3-ethoxy-4-methoxyphenyl)-2-alkylsulfonyl ethyl]-4-acetamido-7-tridecyl isoindoline-1, 3-diketone

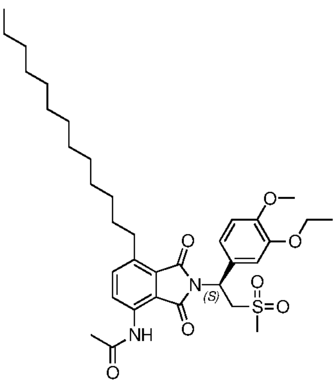

$^1$H-NMR (400MHz, $CDCl_3$) $\delta$= 9.53 (s, 1H), 8.64 (d, J = 8.8 Hz, 1H), 7.42 (d, J = 8.8 Hz, 1H), 7.15 - 7.06 (m, 2H), 6.85 (d, J = 8.8 Hz, 1H), 5.86 (dd, J = 4.4, 10.0 Hz, 1H), 4.53 (dd, J = 10.4, 14.4 Hz, 1H) , 4.12 (q, J = 7.2 Hz, 2H) , 3.85 (s, 3H) , 3.75 (dd, J = 4.8, 14.4 Hz, 1H) , 2.98 (dd, J = 6.4, 8.8 Hz, 2H) , 2.85 (s, 3H) , 2.25 (s, 3H) , 1.63 - 1.57 (m, 2H) , 1.47 (t, J = 7.2 Hz, 3H) , 1.33 - 1.18 (m, 20H) , 0.91 - 0.85 (m, 3H).

LCMS: 643.3 ([M+H]+).

Example 15. (S)-2-[1-(3-ethoxy-4-methoxyphenyl)-2-alkylsulfonyl ethyl]-4-cyclopropanamido-7-tridecyl isoindoline-1, 3-diketone

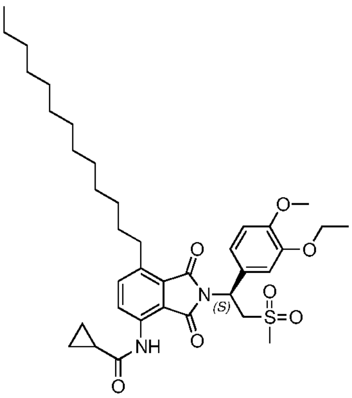

$^1$H-NMR (400MHz, $CDCl_3$) $\delta$= 9.76 (s, 1H) , 8.63 (d, J = 8.8 Hz, 1H), 7.40 (d, J = 8.8 Hz, 1H), 7.15 - 7.08 (m, 2H), 6.85 (d, J = 8.8 Hz, 1H), 5.87 (dd, J = 4.4, 10.0 Hz, 1H), 4.53 (dd, J = 10.4, 14.4 Hz, 1H) , 4.12 (q, J = 6.8 Hz, 2H) , 3.85 (s, 3H) , 3.76 (dd, J = 4.8, 14.4 Hz, 1H) , 2.97 (br t, J = 7.6 Hz, 2H) , 2.84 (s, 3H) , 1.68 - 1.57 (m, 3H) , 1.47 (t, J = 7.2 Hz, 3H) , 1.36 - 1.23 (m, 20H) , 1.13 - 1.09 (m, 2H), 0.92 (br dd, J = 3.2, 7.6 Hz, 2H) , 0.88 (br t, J = 6.8 Hz, 3H).

LCMS: 669.2 ([M+H]+).

Example 16. (S)-2-[1-(3-ethoxy-4-methoxyphenyl)-2-alkylsulfonyl ethyl]-4-butylamido-7-nonyl isoindoline-1, 3-diketone

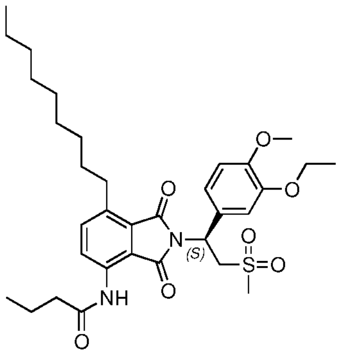

$^1$H-NMR (400MHz, $CDCl_3$) $\delta$= 9.55 (s, 1H) , 8.67 (d, J = 8.8 Hz, 1H), 7.42 (d, J = 8.8 Hz, 1H), 7.14 - 7.08 (m, 2H), 6.85 (d, J = 8.8 Hz, 1H), 5.85 (dd, J = 4.8, 10.0 Hz, 1H), 4.52 (dd, J = 10.0, 14.4 Hz, 1H) , 4.12 (q, J = 7.2 Hz, 2H) , 3.85 (s, 3H) , 3.76 (dd, J = 4.8, 14.4 Hz, 1H) , 2.98 (dd, J = 6.8, 8.8 Hz, 2H) , 2.84 (s, 3H), 2.43 (t, J = 7.6 Hz, 2H) , 1.79 (sxt, J = 7.6 Hz, 2H) , 1.64 - 1.57 (m, 2H) , 1.47 (t, J = 7.2 Hz, 3H) , 1.32 - 1.18 (m, 12H) , 1.03 (t, J = 7.6 Hz, 3H) , 0.89 - 0.86 (m, 3H).

LCMS: 615.2 ([M+H]+).

Example 17. (S)-2-[1-(3-ethoxy-4-methoxyphenyl)-2-alkylsulfonyl ethyl]-4-cyclopropanamido-7-nonyl isoindoline-1, 3-diketone

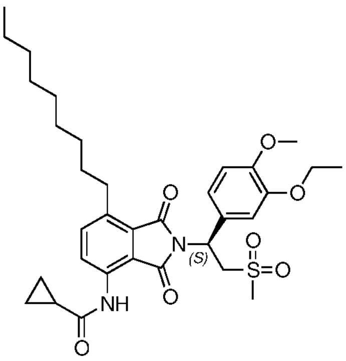

$^1$H-NMR (400MHz, $CDCl_3$) $\delta$ = 9.76 (s, 1H) , 8.62 (d, J = 8.8 Hz, 1H) , 7.40 (d, J = 8.8 Hz, 1H) , 7.15 - 7.08 (m, 2H), 6.85 (d, J = 8.8 Hz, 1H) , 5.87 (dd, J = 4.8, 10.0 Hz, 1H) , 4.53 (dd, J = 10.0, 14.4 Hz, 1H) , 4.12 (q, J = 7.2 Hz, 2H) , 3.85 (s, 3H) , 3.77 (dd, J = 4.8, 14.4 Hz, 1H) , 3.03 - 2.92 (m, 2H) , 2.84 (s, 3H) , 1.67 - 1.57 (m, 3H) , 1.47 (t, J = 7.2 Hz, 3H) , 1.33 - 1.23 (m, 12H) , 1.14 - 1.08 (m, 2H), 0.95 - 0.90 (m, 2H) , 0.89 - 0.86 (m, 3H).

LCMS: 613.1 ([M+H]+).

Example 18. (S)-2-[1-(3-ethoxy-4-methoxyphenyl)-2-alkylsulfonyl ethyl]-4-propanamido-7-nonyl isoindoline-1, 3-diketone

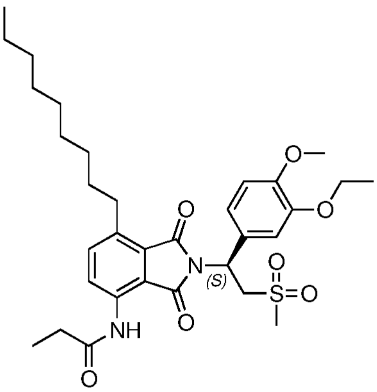

$^1$H-NMR (400MHz, $CDCl_3$) $\delta$= 9.56 (s, 1H) , 8.66 (d, J = 8.8 Hz, 1H), 7.42 (d, J = 8.8 Hz, 1H), 7.14 - 7.07 (m, 2H), 6.84 (d, J = 8.8 Hz, 1H), 5.85 (dd, J = 4.8, 10.0 Hz, 1H), 4.52 (dd, J = 10.0, 14.4 Hz, 1H) , 4.11 (q, J = 7.2 Hz, 2H) , 3.85 (s, 3H) , 3.76 (dd, J = 4.8, 14.4 Hz, 1H) , 2.98 (dd, J = 6.8, 8.4 Hz, 2H) , 2.84 (s, 3H), 2.48 (q, J = 7.6 Hz, 2H) , 1.61 - 1.56 (m, 2H) , 1.47 (t, J = 7.2 Hz, 3H) , 1.34 - 1.21 (m, 15H) , 0.87 (t, J = 6.8 Hz, 3H).

LCMS: 601.2 ([M+H]+).

Example 19. (S)-2-[1-(3-ethoxy-4-methoxyphenyl)-2-alkylsulfonyl ethyl]-4-propanamido-7-tridecyl isoindoline-1, 3-diketone

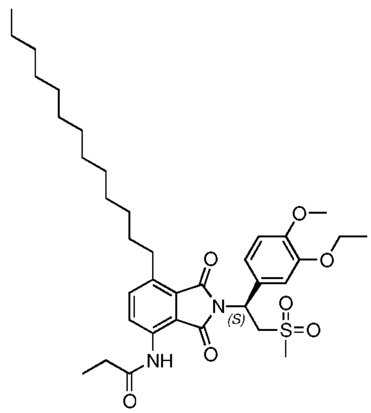

$^1$H-NMR (400MHz, $CDCl_3$) $\delta$= 9.56 (s, 1H) , 8.66 (d, J = 8.8 Hz, 1H), 7.42 (d, J = 8.8 Hz, 1H), 7.16 - 7.06 (m, 2H), 6.84 (d, J = 8.8 Hz, 1H), 5.85 (dd, J = 4.4, 10.0 Hz, 1H), 4.52 (dd, J = 10.0, 14.4 Hz, 1H) , 4.11 (q, J = 7.2 Hz, 2H) , 3.85 (s, 3H) , 3.76 (dd, J = 4.8, 14.4 Hz, 1H) , 2.98 (dd, J = 6.8, 8.8 Hz, 2H) , 2.84 (s, 3H) , 2.48 (q, J = 7.6 Hz, 2H) , 1.62 (br s, 2H) , 1.47 (t, J = 7.2 Hz, 2H) , 1.49 - 1.43 (m, 3H) , 1.37 - 1.23 (m, 23H) , 0.90 - 0.84 (m, 3H).

LCMS: 657.3 ([M+H]+).

Example 20. (S)-2-[1-(3-ethoxy-4-methoxyphenyl)-2-alkylsulfonyl ethyl]-4-(y-chlorobutyramido)-7-hexyl isoindoline-1, 3-diketone

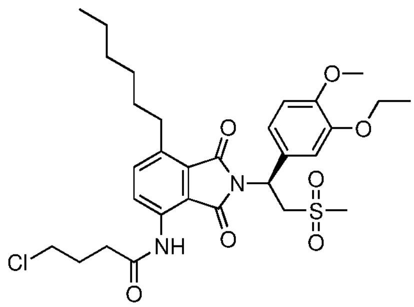

$^1$H-NMR (400MHz, $CDCl_3$) $\delta$= 9.58 (s, 1H), 8.63 (d, J = 8.4 Hz, 1H), 7.43 (d, J = 8.8 Hz, 1H) , 7.11 (br d, J = 4.4 Hz, 2H) , 6.85 (br d, J = 8.8 Hz, 1H), 5.86 (br dd, J = 4.4, 10.0 Hz, 1H) , 4.53 (br dd, J = 10.4, 14.4 Hz, 1H) , 4.12 (q, J = 6.8 Hz, 2H) , 3.85 (s, 3H) , 3.75 (br dd, J = 4.4, 14.4 Hz, 1H) , 3.66 (t, J = 6.4 Hz, 2H) , 2.99 (br t, J = 7.6 Hz, 2H) , 2.85 (s, 3H) , 2.66 (br t, J = 7.2 Hz, 2H) , 2.22 (quin, J = 6.6 Hz, 2H) , 1.64 - 1.57 (m, 2H) , 1.47 (t, J = 7.2 Hz, 3H) , 1.34 - 1.25 (m, 6H) , 0.90 - 0.84 (m, 3H).

LCMS: 607.1 ([M+H]+).

Example 21. (S)-2-[1-(3-ethoxy-4-methoxyphenyl)-2-alkylsulfonyl ethyl]-4-(y-chlorobutyramido)-7-heptyl isoindoline-1, 3-diketone

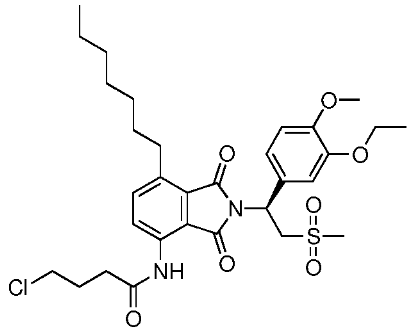

$^1$H-NMR (400MHz, $CDCl_3$) δ= 9.58 (s, 1H) , 8.63 (br d, J = 8.4 Hz, 1H) , 7.42 (br d, J = 8.8 Hz, 1H) , 7.11 (br s, 2H) , 6.85 (br d, J = 8.8 Hz, 1H) , 5.86 (br dd, J = 4.4, 10.0 Hz, 1H) , 4.53 (br dd, J = 10.4, 14.0 Hz, 1H) , 4.12 (q, J = 6.8 Hz, 2H) , 3.85 (s, 3H) , 3.75 (br dd, J = 4.4, 14.4 Hz, 1H) , 3.66 (br t, J = 6.0 Hz, 2H) , 3.07 - 2.91 (m, 2H) , 2.85 (s, 3H) , 2.65 (br t, J = 7.2 Hz, 2H) , 2.22 (quin, J = 6.5 Hz, 2H) , 1.58 (br d, J = 6.4 Hz, 2H) , 1.47 (br t, J = 6.8 Hz, 3H) , 1.34 - 1.23 (m, 8H) , 0.87 (br t, J = 6.4 Hz, 3H).

LCMS: 621.1 ([M+H]+).

Example 22. (S)-2-[1-(3-ethoxy-4-methoxyphenyl)-2-alkylsulfonyl ethyl]-4-(y-chlorobutyramido)-7-octyl isoindoline-1, 3-diketone

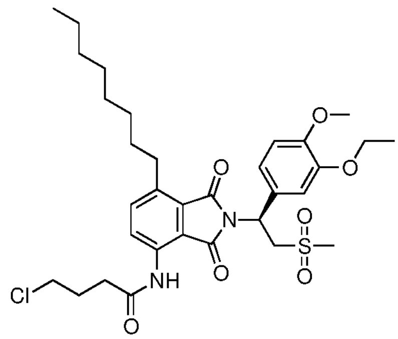

$^1$H-NMR (400MHz, $CDCl_3$) δ = 9.58 (s, 1H) , 8.63 (d, J = 8.8 Hz, 1H) , 7.42 (d, J = 8.8 Hz, 1H) , 7.11 (dd, J = 2.4, 4.4 Hz, 2H) , 6.88 - 6.82 (m, 1H) , 5.86 (dd, J = 4.4, 10.0 Hz, 1H) , 4.53 (dd, J = 10.4, 14.4 Hz, 1H) , 4.12 (q, J = 7.2 Hz, 2H) , 3.85 (s, 3H) , 3.75 (dd, J = 4.8, 14.4 Hz, 1H) , 3.66 (t, J = 6.4 Hz, 2H) , 3.03 - 2.91 (m, 2H) , 2.85 (s, 3H) , 2.66 (t, J = 7.2 Hz, 2H) , 2.22 (quin, J = 6.8 Hz, 2H) , 1.64 - 1.56 (m, 2H) , 1.47 (t, J = 7.2 Hz, 3H) , 1.35 - 1.23 (m, 10H), 0.87 (t, J = 6.8 Hz, 3H).

LCMS: 635.1 ([M+H]+).

Example 23. (S)-2-[1-(3-ethoxy-4-methoxyphenyl)-2-alkylsulfonyl ethyl]-4-propanamido-6-hexyl isoindoline-1, 3-diketone

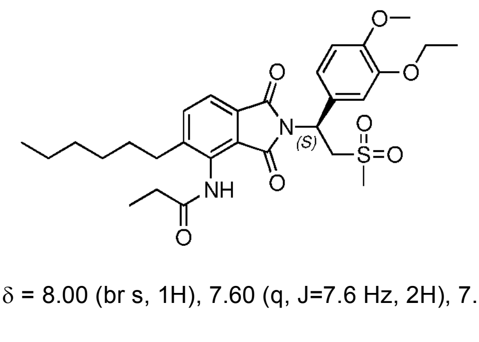

$^1$H-NMR (400MHz, $CDCl_3$) δ = 8.00 (br s, 1H), 7.60 (q, J=7.6 Hz, 2H), 7.11 - 7.05 (m, 2H), 6.83 (d, J=7.6 Hz,

1H), 5.85 (dd, J=4.8, 10.0 Hz, 1H), 4.51 (dd, J=9.6, 14.4 Hz, 1H), 4.10 (q, J=7.2 Hz, 2H), 3.85 (s, 3H), 3.75 (dd, J=4.4, 14.4 Hz, 1H), 2.83 (s, 3H), 2.71 - 2.63 (m, 2H), 2.51 (q, J=7.2 Hz, 2H), 1.60 - 1.56 (m, 2H), 1.46 (t, J=7.2 Hz, 3H), 1.32 - 1.25 (m, 9H), 0.90 - 0.84 (m, 3H).

LCMS: 559 ([M+H]+).

Example 24. (S)-2-[1-(3-ethoxy-4-methoxyphenyl)-2-alkylsulfonyl ethyl]-4-(y-chlorobutyramido)-7-pentyl isoindoline-1, 3-diketone

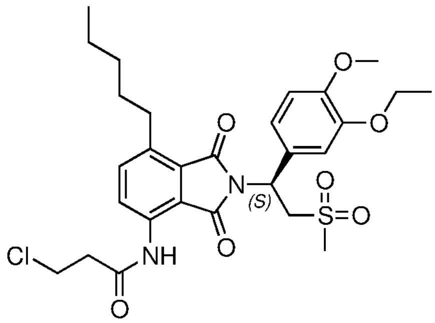

$^1$H-NMR (400MHz, $CDCl_3$) $\delta$ = 9.63 (s, 1H) , 8.65 (d, J = 8.8 Hz, 1H) , 7.44 (d, J = 8.8 Hz, 1H) , 7.15 - 7.07 (m, 2H), 6.85 (d, J = 8.8 Hz, 1H) , 5.86 (dd, J = 4.4, 10.4 Hz, 1H) , 4.53 (dd, J = 10.4, 14.4 Hz, 1H) , 4.12 (q, J = 7.2 Hz, 2H) , 3.88 (t, J = 6.4 Hz, 2H) , 3.85 (s, 3H) , 3.75 (dd, J = 4.4, 14.4 Hz, 1H), 3.03 - 2.95 (m, 2H), 2.91 (t, J = 6.4 Hz, 2H) , 2.85 (s, 3H) , 1.64 - 1.56 (m, 2H), 1.47 (t, J = 7.2 Hz, 3H) , 1.37 - 1.29 (m, 4H) , 0.93 - 0.85 (m, 3H).

LCMS: 579 ([M+H]+).

Example 25. (S)-2-[1-(3-ethoxy-4-methoxyphenyl)-2-alkylsulfonyl ethyl]-4-(chloroacetamido)-7-pentyl isoindoline-1, 3-diketone

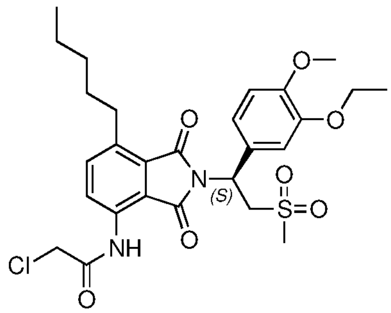

$^1$H-NMR (400MHz, $CDCl_3$) $\delta$ = 10.57 (s, 1H) , 8.64 (d, J = 8.4 Hz, 1H) , 7.46 (d, J = 8.8 Hz, 1H) , 7.17 - 7.09 (m, 2H), 6.85 (d, J = 8.0 Hz, 1H) , 5.87 (dd, J = 4.8, 10.0 Hz, 1H) , 4.53 (dd, J = 10.4, 14.4 Hz, 1H) , 4.21 (s, 2H) , 4.16 - 4.09 (m, 2H) , 3.85 (s, 3H) , 3.77 (dd, J = 4.8, 14.4 Hz, 1H), 3.04 - 2.98 (m, 2H), 2.84 (s, 3H) , 1.63 - 1.59 (m, 2H), 1.47 (t, J = 6.8 Hz, 3H) , 1.36 - 1.31 (m, 4H), 0.91 - 0.87 (m, 3H).

LCMS: 587 ([M+Na]+).

Example 26. (S)-2-[1-(3-ethoxy-4-methoxyphenyl)-2-alkylsulfonyl ethyl]-4-chloroacetamido-7-butyl isoindoline-1, 3-diketone

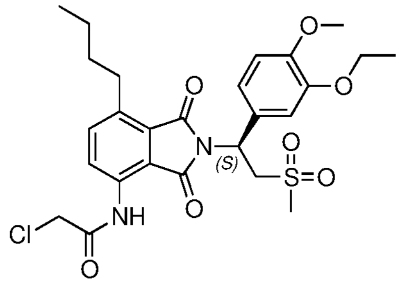

$^1$H-NMR (400MHz, $CDCl_3$) δ = 10.57 (s, 1H), 8.64 (d, J=8.4 Hz, 1H), 7.46 (d, J=8.8 Hz, 1H), 7.17 - 7.10 (m, 2H), 6.85 (d, J=8.4 Hz, 1H), 5.87 (dd, J=4.8, 10.0 Hz, 1H), 4.53 (dd, J=9.6, 14.4 Hz, 1H), 4.21 (s, 2H), 4.17 - 4.08 (m, 2H), 3.85 (s, 3H), 3.77 (dd, J=4.8, 14.4 Hz, 1H), 3.05 - 2.98 (m, 2H), 2.85 (s, 3H), 1.65 - 1.57 (m, 2H), 1.50 - 1.45 (m, 2H), 1.42 - 1.35 (m, 2H), 0.94 (t, J=7.2 Hz, 3H).

LCMS: 551 ([M+H]+).

Example 27. (S)-2-[1-(3-ethoxy-4-methoxyphenyl)-2-alkylsulfonyl ethyl]-4-(γ-chlorobutyramido)-7-butyl isoindoline-1, 3-diketone

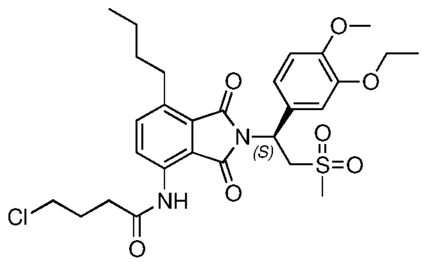

$^1$H-NMR (400MHz, $CDCl_3$) δ = 9.59 (s, 1H), 8.63 (d, J=8.8 Hz, 1H), 7.43 (d, J=8.8 Hz, 1H), 7.15 - 7.07 (m, 2H), 6.85 (d, J=8.8 Hz, 1H), 5.86 (dd, J=4.4, 10.0 Hz, 1H), 4.53 (dd, J=10.0, 14.4 Hz, 1H), 4.12 (q, J=7.2 Hz, 2H), 3.86 (s, 3H), 3.75 (dd, J=4.8, 14.4 Hz, 1H), 3.67 (t, J=6.4 Hz, 2H), 3.04 - 2.96 (m, 2H), 2.85 (s, 3H), 2.66 (t, J=7.2 Hz, 2H), 2.22 (quin, J=6.8 Hz, 2H), 1.63 - 1.56 (m, 2H), 1.47 (t, J=7.2 Hz, 3H), 1.43 - 1.34 (m, 2H), 0.93 (t, J=7.2 Hz, 3H).

LCMS: 579 ([M+H]+).

Example 28. (S)-2-[1-(3-ethoxy-4-methoxyphenyl)-2-alkylsulfonyl ethyl]-4-fluoroacetamido-7-pentyl isoindoline-1, 3-diketone

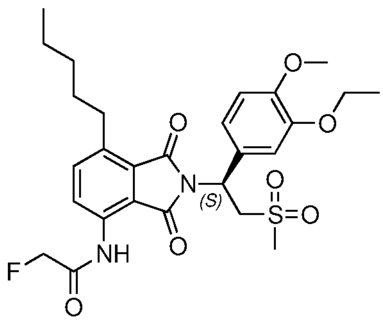

$^1$H-NMR (400MHz, $CDCl_3$) = 10.34 (br d, J=4.4 Hz, 1H), 8.65 (d, J=8.4 Hz, 1H), 7.46 (d, J=8.8 Hz, 1H), 7.16 - 7.10 (m, 2H), 6.84 (d, J=8.8 Hz, 1H), 5.86 (dd, J=4.4, 10.0 Hz, 1H), 5.04 - 4.86 (m, 2H), 4.54 (dd, J=10.0, 14.4 Hz, 1H), 4.12 (q, J=7.2 Hz, 2H), 3.85 (s, 3H), 3.75 (dd, J=4.4, 14.4 Hz, 1H), 3.01 (dd, J=6.8, 8.8 Hz, 2H), 2.85 (s, 3H), 1.67 - 1.59

(m, 2H), 1.47 (t, J=7.2 Hz, 3H), 1.39 - 1.32 (m, 4H), 0.93 - 0.87 (m, 3H).

LCMS: 571 ([M+Na]+).

Example 29. (S)-2-[1-(3-ethoxy-4-methoxyphenyl)-2-alkylsulfonyl ethyl]-4-acrylamido-7-pentyl isoindoline-1, 3-diketone

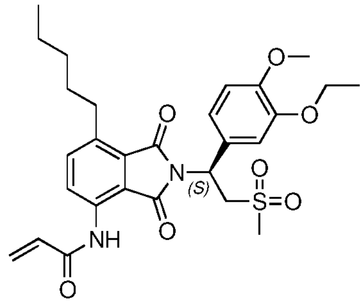

$^1$H-NMR (400MHz, $CDCl_3$) δ = 9.73 (s, 1H) , 8.73 (d, J = 8.8 Hz, 1H) , 7.45 (d, J = 8.8 Hz, 1H) , 7.11 (qd, J = 2.0, 4.4 Hz, 2H) , 6.85 (d, J = 8.8 Hz, 1H) , 6.50 - 6.43 (m, 1H) , 6.36 - 6.27 (m, 1H) , 5.90 - 5.83 (m, 2H) , 4.53 (dd, J = 10.4, 14.4 Hz, 1H) , 4.12 (q, J = 6.8 Hz, 2H) , 3.85 (s, 3H) , 3.76 (dd, J = 4.4, 14.4 Hz, 1H) , 3.03 - 2.96 (m, 2H), 2.85 (s, 3H) , 1.66 - 1.57 (m, 2H), 1.47 (t, J = 7.2 Hz, 3H) , 1.38 - 1.31 (m, 4H) , 0.92 - 0.85 (m, 3H).

LCMS: 543 ([M+H]+).

Example 30:

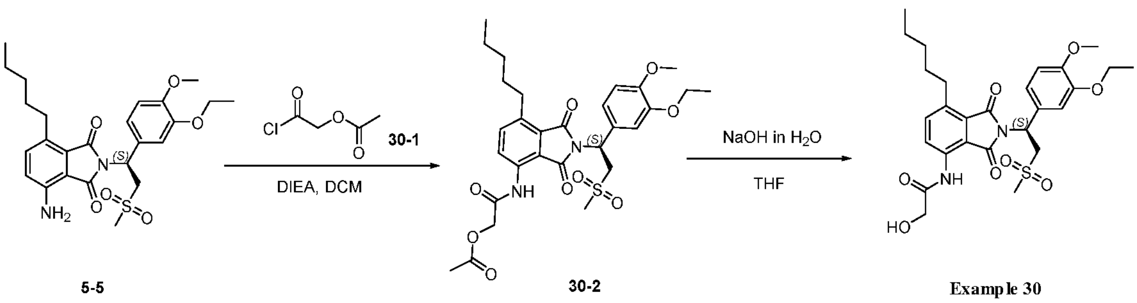


5-5 30-2 Example 30

Synthesis of compound 30-2:

The compound 5-5 (200mg, 0.409mmol, 1.0eq) was dissolved in DCM (7mL), DIEA (158mg, 1.228mmol, 3.0eq) and a compound 30-1 (111.77mg, 0.818mmol, 2.0eq) were added to the reaction solution, and the mixture was stirred at room temperature for 1h. The reaction solution was quenched with water and then added with water (10mL), extraction was performed with ethyl acetate (20*2mL), the combined organic layers were washed with a saturated saline solution, dried with $Na_2SO_4$, filtered, and concentrated to obtain a crude product, and the crude product was purified by column chromatography ($SiO_2$, petroleum ether: ethyl acetate=1/0 to 1/1) to obtain a compound 30-2 (150mg, yield: 62.5%).

$^1$H-NMR (400 MHz, DMSO) δ 10.10 (s, 1H), 8.42 (d, J = 8.6 Hz, 1H), 7.64 (d, J = 8.6 Hz, 1H), 7.12 (d, J = 1.8 Hz, 1H), 6.97 (dt, J = 16.7, 5.1 Hz, 2H), 5.77 (dd, J = 10.1, 4.6 Hz, 1H), 4.76 (s, 2H), 4.34 (dd, J = 14.3, 10.2 Hz, 1H), 4.16 (dd, J = 14.4, 4.7 Hz, 1H), 4.03 (d, J = 7.1 Hz, 2H), 3.74 (s, 3H), 3.00 (s, 3H), 2.98 - 2.89 (m, 2H), 2.24 (s, 3H), 1.66 - 1.47 (m, 2H), 1.33 (d, J = 6.9 Hz, 3H), 1.30 - 1.22 (m, 4H), 0.85 (t, J = 6.8 Hz, 3H).

Synthesis of Example 30:

The compound 30-2 (150mg, 0.255mmol, 1.0eq) was dissolved in THF (5mL) and $H_2O$ (2.5mL), NaOH (2.5g) was added, and the mixture was stirred at room temperature for 1h. The reaction solution was added with water (20mL), and extracted with ethyl acetate (20mL*2); the combined organic layers were washed with a saturated saline solution, dried with $Na_2SO.$, filtered, and concentrated to obtain a crude product; and the crude product was purified by using

prep-TLC ($SiO_2$, dichloromethane: methanol=10:1) to obtain Example 30 (43.86mg, yield: 31.49%).

$^1H$ NMR (400 MHz, DMSO) δ 10.66 (s, 1H), 8.65 (d, J = 8.6 Hz, 1H), 7.64 (d, J = 8.7 Hz, 1H), 7.08 (d, J = 1.9 Hz, 1H), 6.98 (dt, J = 19.0, 5.2 Hz, 2H), 6.32 (t, J = 5.6 Hz, 1H), 5.77 (dd, J = 10.3, 4.4 Hz, 1H), 4.35 (dd, J = 14.3, 10.5 Hz, 1H), 4.15 (dd, J = 14.3, 4.5 Hz, 1H), 4.09 - 3.98 (m, 4H), 3.74 (s, 3H), 3.02 (d, J = 5.9 Hz, 3H), 2.99 - 2.88 (m, 2H), 1.66 - 1.41 (m, 2H), 1.35 - 1.23 (m, 7H), 0.85 (t, J = 6.9 Hz, 3H).

LCMS: (M+H) +: 547.

Example 31:

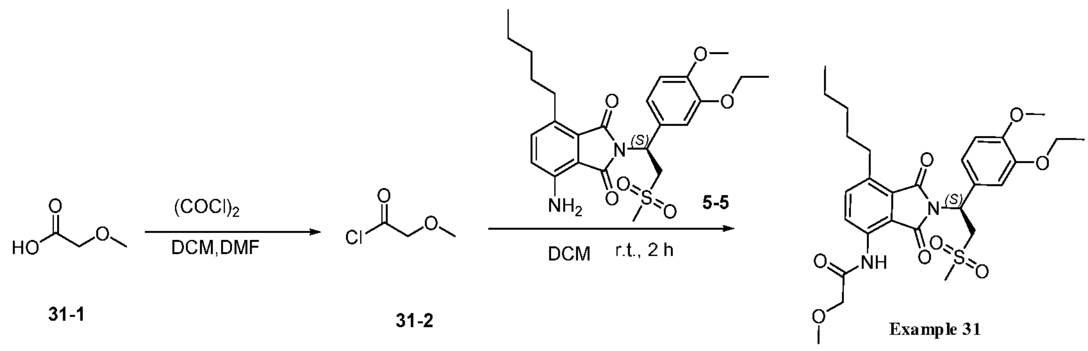


31-1

31-2

Synthesis of compound 31-2:

A compound 31-1 (1.0g, 11.1mmol, 1.0eq) was dissolved in DCM (15mL), $(COCl)_2$ (1.8g, 14.4mmol, 1.3eq) was added at 0°C, followed by a catalytic amount of DMF (0.1mL), and the mixture was stirred at room temperature for 18h. The reaction solution was directly used in the next step.

Synthesis of Example 31:

The compound 5-5 (100mg, 0.20mmol, 1.0eq) was dissolved in DCM (5mL), DIEA (400mg, 3.05mmol, 5.0eq) and the compound 31-2 (1.0mL) were added, and the mixture was stirred at room temperature for 2h. The reaction solution was added with water (10mL), and extracted with ethyl acetate (20mL*2); the combined organic layers were washed with a saturated saline solution, dried with $Na_2SO_4$, filtered, and concentrated to obtain a crude product; and the crude product was purified by using prep-HPLC (a formic acid system) to obtain Example 31 (15.28mg, yield: 10%).

$^1H$-NMR (400 MHz, $CDCl_3$) δ 10.47 (s, 1 H), 8.70 (d, J = 8.4 Hz, 1H),7.45 (d, J= 8.4 Hz, 1 H), 7.14-7.12 (m, 2H), 6.85 (d, J= 8.4 Hz, 1H),5.88 (q, J= 4.8 Hz, 1 H),4.52-4.47 (m, 1 H), 4.13-4.12(m, 2 H),4.06 (d, J = 1.0 Hz, 2H), 3.85 (s, 3 H), 3.81 (dd, J = 5.2 Hz, 14.4 Hz, 1H),3.58 (s, 3H), 2.99 (t, J =8.0 Hz, 2H),2.82 (s, 3 H),1.48 (t, J = 10.2 Hz, 4H),1.35-1.32 (m, 5H),1.26(s, 1H), 0.92-0.87 (m, 3H).

LCMS: (M+H) +:561.

Example 32

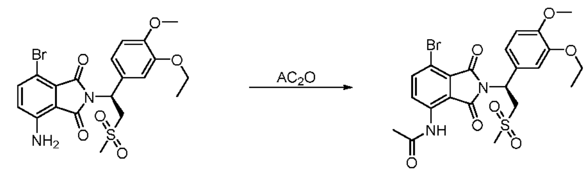


5-4 32-1

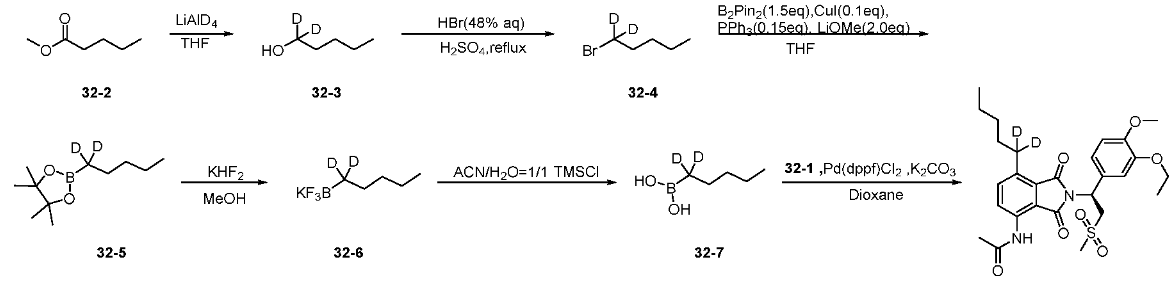


32-2 32-3 32-4

32-5 32-6 32-7

Example 32

Synthesis of compound 32-1:

The compound 5-4 (2g, 4.12mmol, 1.0eq) was dissolved in acetic anhydride (9mL) and stirred at 110°C for 2h. The reaction solution was concentrated to obtain a residue, the residue was slurried with methyl tert-butyl ether/ethyl acetate (20mL/10mL) and then filtered, and the filter cake was washed with methyl tert-butyl ether to obtain a compound 32-1 (1.8g, 3.34mmol, yield: 80.99%).

Synthesis of compound 32-3:

A compound 32-2 (8.05g, 69.30mmol, 1.0eq) was dissolved in THF (100mL), $LiAlD_4$ (3.2g, 41.98mmol, 1.1eq) was added at 0°C, and the mixture was gradually heated up to room temperature and stirred for 5h. The reaction solution was quenched with ethyl acetate (4mL) and then concentrated. The solid was suspended in ethyl acetate (100mL) at 0°C, a small amount of cold water (80mL) was added into the suspension, the pH of the solution was adjusted to 1 with 2M HCl, and extraction was carried out with ethyl acetate (60mL*2). the combined organic layers were washed with a saturated saline solution, dried with $Na_2SO_4$, filtered, and concentrated to obtain a compound 32-3 (5.13g, 56.89mmol, yield: 82.11%).

Synthesis of compound 32-4:

HBr (14.62g, 40% purity, 72.26mmol, 1.27eq) was dissolved in $H_2SO_4$ (3.64mL), the compound 32-3 (5.13g, 56.89mmol, 1eq) was added, and the mixture was stirred at 120°C for 2h. The reaction solution was added with water (500mL), and extracted with ethyl acetate (100mL*2); the combined organic layers were washed with a saturated saline solution, dried with $Na_2SO_4$, filtered, and concentrated to obtain a compound 32-4 (1.79g, 11.69mmol, yield: 20.56%).

Synthesis of compound 32-5:

B2Pin2 (3.199g, 12.64mmol, 1.5eq), CuI (160mg, 0.84mmol, 0.1eq), $LiO^tBu$ (1.349g, 16.86mmol, 2eq) were dissolved in tetrahydrofuran (10mL), the compound 32-4 (1.29g, 8.42mmol, 1eq) was added, and the mixture was stirred at room temperature under nitrogen atmosphere for 16h. The reaction solution was filtered and concentrated to obtain a crude product, and the crude product was purified by column chromatography (silica, hexane: ethyl acetate=100:1 to 50:1) to obtain a compound 32-5 (1.28g, 6.39mmol, yield: 75.96%).

Synthesis of compound 32-6:

The compound 32-5 (400mg, 1.99mmol, 1.0eq) was dissolved in methanol (5mL), $KHF_2$ (4.5mL, 19.99mmol, 4.5M, 10eq) was added, and the mixture was stirred at room temperature for 16h. The reaction solution was concentrated to obtain a crude product, which was dissolved and filtered with hot acetone (10mL); the combined filtrate was concentrated to 4mL, and then added with ether (10mL); and the precipitated white solid was filtered, and the filter cake was dried to obtain a compound 32-6 (260mg, 1.44mmol, yield: 72.36%).

Synthesis of compound 32-7:

The compound 32-6 (260mg, 1.44mmol, 1.0eq) was dissolved in acetonitrile/water (2mL/1mL), trimethoxy chlorosilane (468mg, 4.31mmol, 3eq) was added, and the mixture was stirred at room temperature for 16h. The reaction solution was diluted with a saturated sodium bicarbonate solution (10mL), and extracted with ethyl acetate (10mL*2); and the combined organic layers were washed with a saturated saline solution, dried with $Na_2SO_4$, filtered, and concentrated to obtain a compound 32-7 (30mg, 0.25mmol, yield: 17.66%).

Synthesis of Example 32

The compound 32-7 (30mg, 0.25mmol, 2eq) was dissolved in dioxane (0.5mL), the compound 32-1 (68.5mg, 0.127mmol, 1eq), $K_2CO_3$ (52.6mg, 0.381mmol, 3eq) and Pd(dppf)$Cl_2$ (4.6mg, 6.35μmol, 0.05eq) were added, and the mixture was stirred at 100°C for 4h under nitrogen atmosphere. The reaction solution was added with water (10mL), and extracted with ethyl acetate (3mL*2); the combined organic layers were washed with a saturated saline solution, dried with $Na_2SO_4$, filtered, and concentrated to obtain a crude product; and the crude product was purified by using prep-HPLC (a formic acid system) to obtain Example 32 (24.87mg, 0.047mmol, yield: 36.81%).

$^1$H NMR (400 MHz, $CDCl_3$) δ 9.53 (s, 1H), 8.64 (d, J = 8 Hz, 1H), 7.42 (d, J = 8 Hz, 1H), 7.10 (s, 2H), 6.85 (d, J = 8 Hz, 1H), 5.86 (m, 1H), 4.53 (m, 1H), 4.11 (q, 2H), 3.85 (s, 3H), 3.74 (m, 1H), 2.85 (s, 3H), 2.25 (s, 3H), 1.47 (t, 3H), 1.36 - 1.31 (m, 4H), 1.26 (d, J = 4 Hz, 2H), 0.88 (t, 3H).
LCMS: (M+H) +:533.3

Example 33

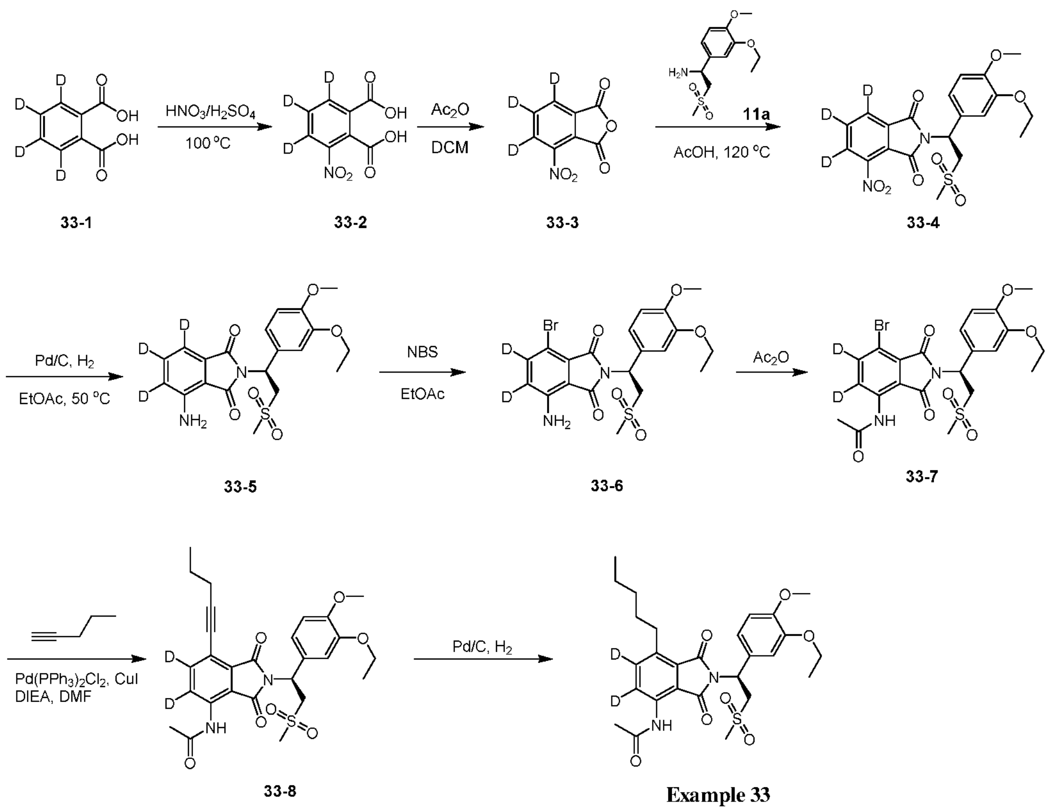

33-1 33-2 33-3 33-4 33-5 33-6 33-7 33-8 Example 33

Synthesis of compound 33-2: A compound 33-1 (1g, 5.88mmol, 1eq) was dissolved in $H_2SO_4$ (3.75mL), and $HNO_3$ (1.48g, 23.51mmol, 1.06mL, 4eq) was added. The reaction solution was stirred at 100°C for 3h. The reaction solution was slowly poured into ice water (50mL), and extracted with ethyl acetate (50mL*3); the combined organic layers were washed with a saturated saline solution, dried with $Na_2SO_4$, filtered, and concentrated to obtain a crude product; and the crude product was purified by column chromatography to obtain a compound 33-2 (970mg, 4.08mmol, yield: 69.37%).

LCMS (ESI+): m/z 258.9 (M+1+46)+

Synthesis of compound 33-3: The compound 33-2 (970mg, 4.53mmol, 1.0eq) was dissolved in acetic anhydride (15mL), and stirred at 120°C for 2h. The reaction solution was concentrated to obtain a crude product, which was a compound 33-3 (888mg, 4.53mmol, yield: 99.96%) directly used in the next step.

Synthesis of compound 33-4: The compound 11a (1.36g, 4.98mmol, 1.1eq) and the compound 33-3 (888mg, 4.53mmol, 1eq) were dissolved in AcOH (15mL), and stirred at 120°C for 16h. The reaction solution was concentrated to obtain a crude product, and the crude product was purified by column chromatography (petroleum ether: ethyl acetate 10/1 to 4:1) to obtain a compound 33-4 (680mg, 1.51mmol, yield: 33.27%).
LCMS (ESI+): m/z 451.5 (M+1)+ :

Synthesis of compound 33-5: The compound 33-4 (580mg, 1.28mmol, 1eq) was dissolved in ethyl acetate (5mL), Pd/C (100mg, 7.71mmol, 10% purity) was added under hydrogen atmosphere, and the mixture was stirred at 50°C under hydrogen (15 psi) atmosphere for 3h. The reaction solution was filtered and concentrated to obtain a compound 33-5 (520mg, 1.23mmol, yield: 96.03%).
LCMS (ESI+): m/z 451.5 (M+1)+

Synthesis of compound 33-6: The compound 33-5 (520mg, 1.23mmol, 1eq) was dissolved in ethyl acetate (10mL), NBS (219.59mg, 1.23mmol, 1eq) was added, and the mixture was stirred at 25°C for 4h. Water (30mL) and ethyl acetate (3*30mL) were added for extraction, the combined organic layers were washed with a saturated saline solution, dried with $Na_2SO_4$, filtered, and concentrated to obtain a crude product, and the crude product was purified by column chromatography (SiOz, petroleum ether: ethyl acetate=100/0 to 1/1) to obtain a compound 33-6 (534mg, 1.07mmol, yield: 86.67%).
LCMS (ESI+): m/z 499.6 (M+1)+

Synthesis of compound 33-7: The compound 33-6 (534mg, 1.07mmol, 1eq) was dissolved in acetic anhydride (190.33mg, 1.86mmol, 174.61μL, 1.74eq), the reaction solution was stirred at 120°C for 3h, and then the reaction solution was concentrated to obtain a crude compound 33-7 (450mg, 831.17μmol, yield: 77.73%).

Synthesis of compound 33-8: The compound 33-7 (100mg, 184.70μmol, 1eq), CuI (7.04mg, 36.94umol, 0.2eq), Pd(PPh3)2$Cl_2$ (25.93mg, 36.94μmol, 0.2eq), DIEA (71.61mg, 554.11μmol, 96.52μL, 3eq) and 1-pentyne (125.81mg, 1.85mmol, 181.29μL, 10eq) were dissolved in DMF (4mL); the reaction solution was stirred at 60°C under $N_2$ atmosphere for 16h; water (5mL) and ethyl acetate (5mL*3) were added into the reaction system for extraction; the combined organic layers were washed with a saturated saline solution, dried with $Na_2SO_4$, filtered, and concentrated to obtain a crude product; and the crude product was purified by column chromatography (SiOz, petroleum ether: ethyl acetate 1/0 to 1:1) to obtain a compound 33-8 (75mg, 141.88μmol, yield: 76.82%).
LCMS (ESI+): m/z 529.4 (M+1)+

Synthesis of Example 33: The compound 33-8 (75mg, 141.88μmol, 1eq) was dissolved in EtOAc (5mL), Pd/C (160mg, 141.88μmol, 10% purity) was added under nitrogen atmosphere, and the mixture was replaced with hydrogen for 3 times under vacuum condition, and stirred at 50° C under hydrogen atmosphere (50 Psi) for 16h. The reaction solution was filtered with diatomite to remove solids, the filter cake was washed with EtOAc, the filtrate was concentrated to obtain a crude product, and the crude product was purified by using prep-HPLC (a formic acid system) to obtain Example 33 (19mg, 35.67μmol, yield: 25.14%).

$^1$H NMR (400 MHz, DMSO-d6) δ 0.85 (s, 3 H), 1.22 - 1.36 (m, 7 H), 1.44 - 1.66 (m, 2 H), 2.17 (s, 3 H), 2.95 (t, J=7.15 Hz, 2 H), 3.01 (s, 3 H) 3.73 (s, 3 H), 4.01 (d, J=6.85 Hz, 2 H), 4.08 - 4.19 (m, 1 H), 4.28 - 4.41 (m, 1 H), 5.76 (d, J=5.99 Hz, 1 H), 6.89 - 7.01 (m, 2 H), 7.07 (s, 1 H) 9.68 (s, 1 H).
LCMS (ESI+): m/z 533.1 (M+1)+

Example 55

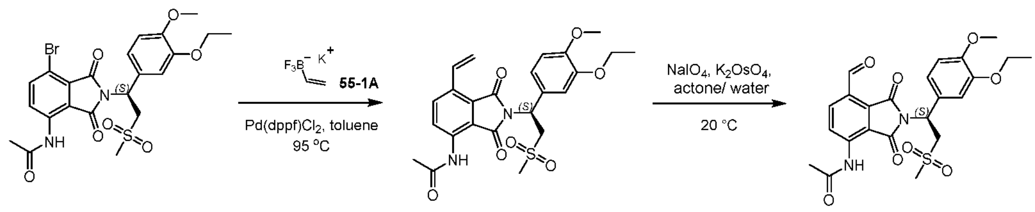


32-1 55-2 55-3

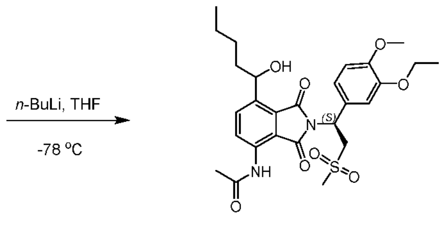


Example 55

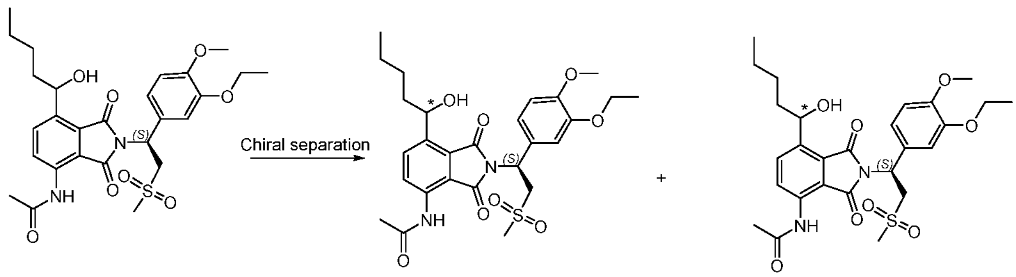


Example 55 Example 55A Example 55B

Synthesis of compound 55-2: The compound 32-1 (5g, 9.27mmol) was dissolved in dioxane (60mL) and water (15mL); Pd(dppf)$Cl_2$ (900mg, 1.23mmol), a compound 55-1A, and potassium phosphate (6.89g, 32.4mmol) were added into the reaction solution under nitrogen atmosphere, and the mixture was replaced with nitrogen and kept under nitrogen atmosphere; and the product was stirred at 95°C for 16h, diluted with water (100mL), extracted with ethyl acetate (200mL*2), and then washed with a saturated saline solution (200mL). The organic phases were combined, and the combined organic layers were dried with anhydrous $Na_2SO_4$, filtered, concentrated under reduced pressure, and purified by column chromatography, so that a yellow solid compound 55-2 (4.2g, 7.77mmol, yield: 83.8%) was obtained. LCMS (ESI+): m/z 478.0 (M+1)+ :

Synthesis of compound 55-3: The compound 55-2 (4.2g, 8.63mmol, 1eq) was dissolved in acetone (400mL), dichloromethane (150mL) and water (150mL), $K_2O_SO_4$ (1.26g, 3.42mmol, 0.4eq) was added to the reaction solution, the mixture was stirred at 0°C for 5min, and $NaIO_4$ (7.39g, 34.6mmol, 1.92mL, 4eq) was added to the reaction solution, and stirred at 20°C for 6h. The mixture was diluted with water (100mL), extracted with ethyl acetate (1000mL), and washed with a saturated saline solution (300mL). The organic phases were combined, and the combined organic layers were dried with anhydrous $Na_2SO_4$, filtered, concentrated under reduced pressure, and purified by column chromatography, so that a yellow solid compound 55-3 (3.5g, 6.45mmol, yield: 74.7%) was obtained.

LCMS (ESI+): m/z 489.3 (M+1)+ : 1.34 min.

Synthesis of Example 55: The compound 55-3 (3.5g, 7.16mmol, 1eq) was dissolved in THF (10mL), the reaction solution was cooled to -78°C, and n-BuLi (1.6M, 13.5mL, 3eq) was added dropwise into the reaction solution; the reaction solution was stirred at -78°C for 1h, the reaction solution was quenched with a saturated $NH_4Cl$ solution (20mL), and then diluted with ethyl acetate (200mL); and the organic phases were combined, and the obtained combined organic layers were washed with a saturated saline solution (50mL), dried with anhydrous $Na_2SO_4$, filtered, and purified by using pre-HPLC (FA), so that a pair of diastereoisomeric compounds in Example 55 (289.80.74mg, 0.53mmol, yield: 7.4%) were obtained. Example 55 was subjected to chiral separation to obtain Example 55A (107.1mg, 99% purity) and Example 55B (1 17.5mg, 99% purity).

$^1H$ NMR (400 MHz, $CDCl_3$) of Example 55: δ ppm 0.79 - 0.84 (m, 3 H), 1.19 - 1.32 (m, 4 H), 1.41 (s, 3 H), 1.69 -

1.80 (m, 1 H), 1.70 - 1.76 (m, 1 H), 2.19 (s, 3 H), 2.80 (s, 3 H), 3.67 (dt, J = 14.4, 5.1 Hz, 1 H), 3.79 (s, 3 H), 4.04 (q, J = 7.0 Hz, 2 H), 4.41 - 4.50 (m, 1 H), 4.96 (br d, J = 5.4 Hz, 1 H), 5.77 - 5.84 (m, 1 H), 6.76 - 6.81 (m, 1 H), 6.99 - 7.05 (m, 2 H), 7.19 (s, 7 H), 7.54 (d, J = 8.8 Hz, 1 H), 8.64 (d, J = 8.3 Hz, 1 H), 9.48 (s, 1 H).
LCMS (ESI+): m/z 569.3 (M+Na)+

Synthesis of Example 56:

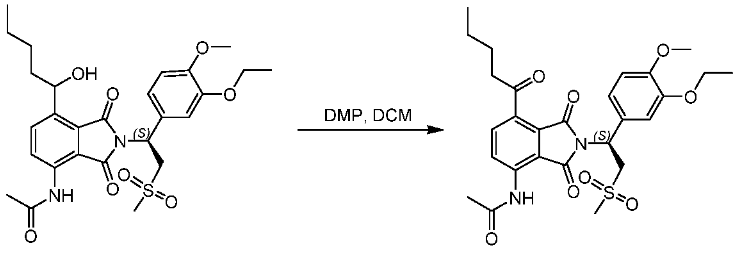


Example 55 Example 56

Synthesis of Example 56: The compound 55 (60mg, 0.11mmol, 1eq) was dissolved in DCM (1mL), DMP (120mg, 0.28mmol, 2.6eq) was added, and the mixture was stirred at room temperature for 1h; the reaction solution was diluted with DCM (30mL), a saturated $NaHCO_3$ solution (3mL) and a saturated $NH_4Cl$ (3mL); and the organic phases were combined, and the obtained combined organic layers were washed with a saturated saline solution (30mL), dried with anhydrous $Na_2SO_4$, filtered, and purified by using pre-HPLC (FA), so that a compound in Example 56 (43.1mg, 0.08mmol, yield: 72.1%) was obtained.

$^1H$ NMR (400 MHz, $CDCl_3$) $\delta$ ppm 0.86 (t, J = 7.3 Hz, 3 H), 1.27 - 1.36 (m, 2 H), 1.41 (s, 3 H), 1.57 - 1.66 (m, 2 H), 2.15 - 2.30 (m, 3 H), 2.78 - 2.88 (m, 3 H), 2.98 (t, J = 7.4 Hz, 2 H), 3.63 (dd, J = 14.3, 4.3 Hz, 1 H), 3.79 (s, 3 H), 4.04 (q, J = 7.0 Hz, 2 H), 4.48 (dd, J = 14.3, 10.8 Hz, 1 H), 5.81 (dd, J = 10.7, 4.2 Hz, 1 H), 6.78 (d, J = 8.2 Hz, 1 H), 6.96 - 7.07 (m, 2 H), 7.61 (d, J = 8.7 Hz, 1 H), 8.73 (d, J = 8.7 Hz, 1 H), 9.66 (s, 1 H).
LCMS (ESI+): m/z 545.3 (M+H)+

Example 34-54

| Compound No. | Compound structure | $(M+H)^+$ | Reference example for synthesis method |
|---|---|---|---|
| Example 34 | 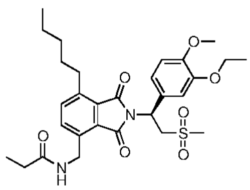 | 559.1 | 4 |
| Example 35 | 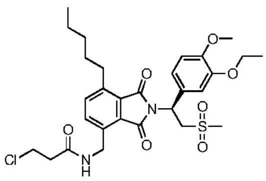 | 593.1 | 4 |

(continued)

| Compound No. | Compound structure | (M+H)+ | Reference example for synthesis method |
|---|---|---|---|
| Example 36 | 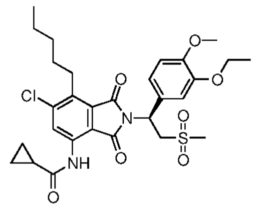 | 591 | 5 |
| Example 37 | 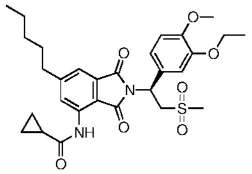 | 557.1 | 2 |
| Example 38 | 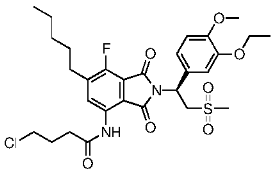 | 611.1 | 2 |
| Example 39 | 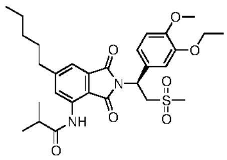 | 559.1 | 2 |
| Example 40 | 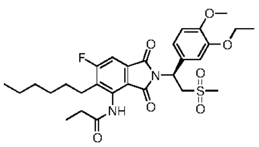 | 577.1 | 1 |
| Example 41 | 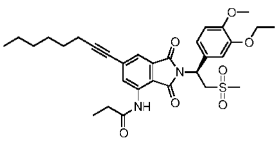 | 583.1 | 3 |
| Example 42 | 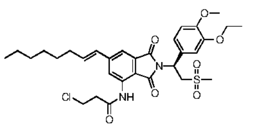 | 619.1 | 3 |
| Example 43 | 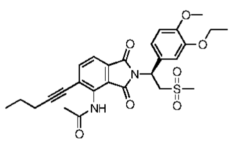 | 527.1 | 1 |
| Example 44 | 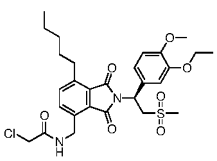 | 579.1 | 4 |

(continued)

| Compound No. | Compound structure | (M+H)+ | Reference example for synthesis method |
|---|---|---|---|
| Example 45 | 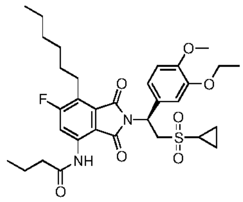 | 617.1 | 5 |
| Example 46 | 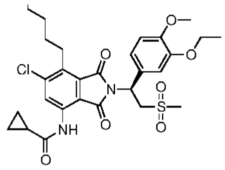 | 591.1 | 5 |
| Example 47 | 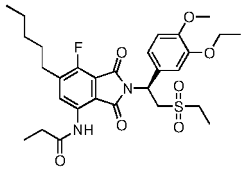 | 577.2 | 3 |
| Example 48 | 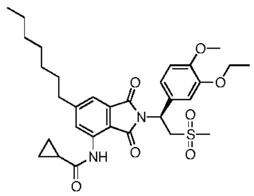 | 584.1 | 3 |
| Example 49 | 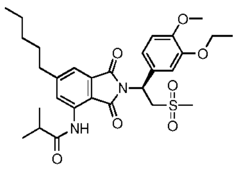 | 559.2 | 3 |
| Example 50 | 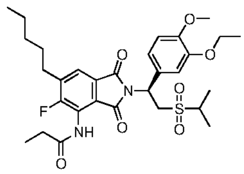 | 591.1 | 3 |
| Example 51 | 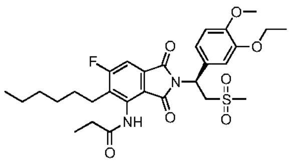 | 577.1 | 1 |
| Example 52 | 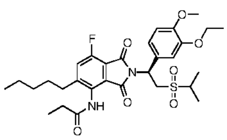 | 591.1 | 1 |

(continued)

| Compound No. | Compound structure | (M+H)+ | Reference example for synthesis method |
|---|---|---|---|
| Example 53 | 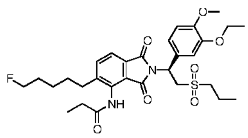 | 591.1 | 1 |
| Example 54 | 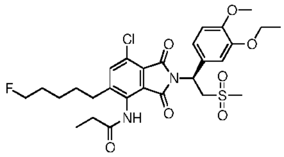 | 597.1 | 1 |

Refer to Examples 9, 55, or 56 for synthesizing the following compounds in Examples 57-82

| Compound No. | Compound structure | (M+H)+ |
|---|---|---|
| Example 57 | 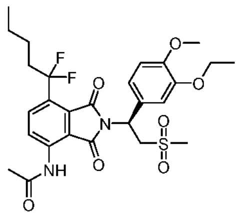 | 567.1 |
| Example 58 | 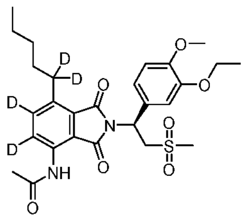 | 535.2 |
| Example 59 | 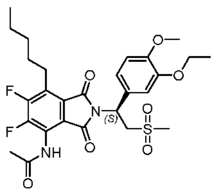 | 567.1 |
| Example 60 | 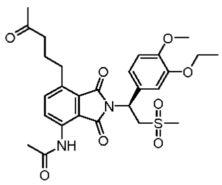 | 545.3 |
| Example 61 | 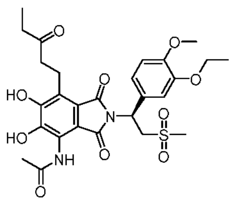 | 599.1 (M+Na)+ |

(continued)

| Compound No. | Compound structure | (M+H)+ |
|---|---|---|
| Example 62 | 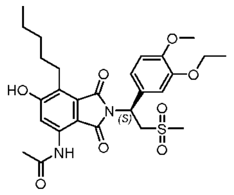 | 569.3 (M+Na)+ |
| Example 63 | 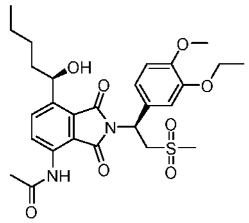 | 569.3 (M+Na)+ |
| Example 64 | 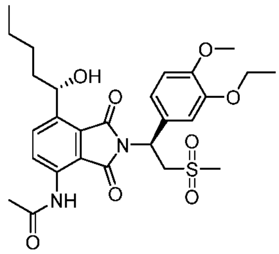 | 569.3 (M+Na) |
| Example 65 | 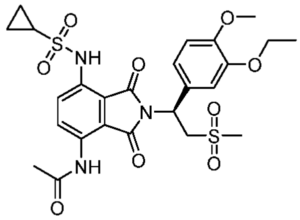 | 580.1 |
| Example 66 | 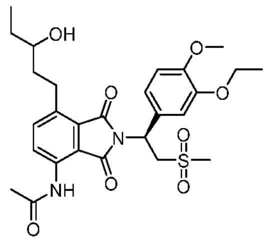 | 569.3 (M+Na)+ |
| Example 67 | 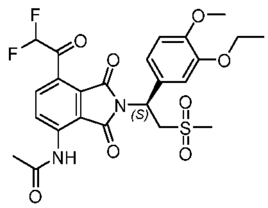 | 539.1 |
| Example 68 | 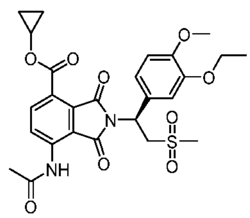 | 545.1 |

(continued)

| Compound No. | Compound structure | (M+H)+ |
|---|---|---|
| Example 69 | 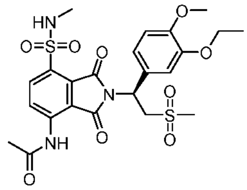 | 554.1 |
| Example 70 | 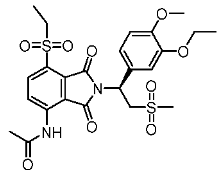 | 553.1 |
| Example 71 | 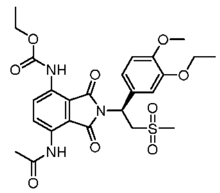 | 548.1 |
| Example 72 | 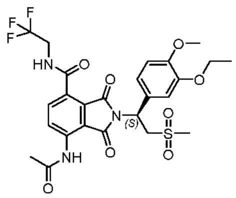 | 586.1 |
| Example 73 | 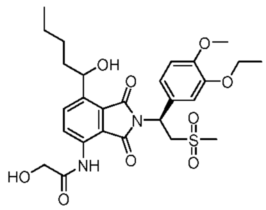 | 585.2 |
| Example 74 | 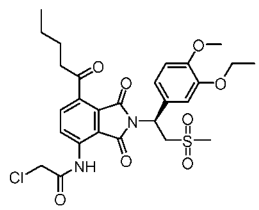 | 579.1 |
| Example 75 | 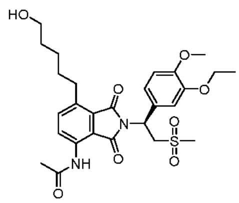 | 569.3 (M+Na)+ |

(continued)

| Compound No. | Compound structure | $(M+H)^+$ |
|---|---|---|
| Example 76 | 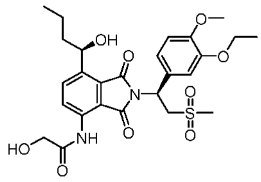 | 571.2 (M+Na)+ |
| Example 77 | 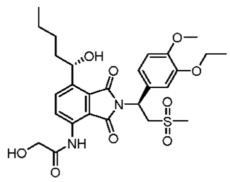 | 585.2 (M+Na)+ |
| Example 78 | 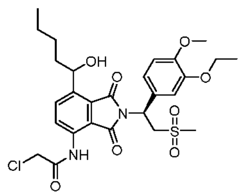 | 603.1 (M+Na)+ |
| Example 79 | 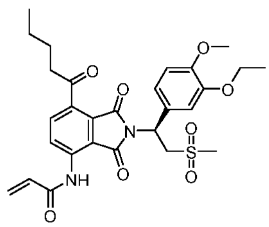 | 557.1 |
| Example 80 | 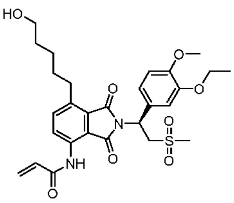 | 581.2 (M+Na)+ |
| Example 81 | 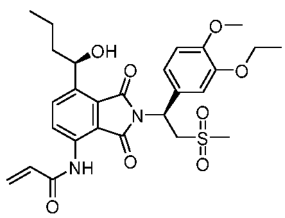 | 567.1 (M+Na)+ |
| Example 82 | 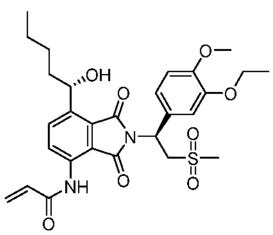 | 581.2 (M+Na)+ |

Examples of biological activity of compounds

1. cLogP value calculation:

Compound cLogP is one of the methods for evaluating the lipophilicity of compounds. A high cLogP value indicates that a compound has stronger lipophilicity, and the compound tends to pass through the lipid layer of the human body more passively (through the principle of compound concentration diffusion). The CLogP values of compounds are shown in the table below:

| Compound No. | CLog P |
|---|---|
| Example 0 | 1.4606 |
| Example 5 | 4.9966 |
| Example 9 | 4.0756 |
| Example 13 | 4.6046 |
| Example 32 | 4.0756 |
| Example 33 | 4.0756 |

Note: Example 0 involved in the present disclosure represents the control drug Apremilast.

2. Inhibition assay of PDE4D3 enzyme:

Materials and Instruments:

| |
|---|
| PDE4D3 TR-FRET detection kit (BPS, Cat.60701): |
| PDE4D3 recombinase |
| FAM-Cyclic-3', 5'-AMP |
| PDE buffer |
| Tb donor |
| Binder |
| Binding buffer A |
| Binding buffer B |
| Black plate (VWR62408-936) |
| SpectraMax M4 multi-mode reader |

Test conditions:

| | Component | Concentration |
|---|---|---|
| Final concentration in enzyme reaction step (25μL) | FAM substrate | 100nM |
| | PDE4D3 | 30 pg/well |
| | Enzyme reaction time | 60min |
| Final concentration in assay reaction (50μL) | Binder | Dilute at 1:50 |
| | Tb donor | Dilute at 1:1000 |

Reagent preparation:

FAM substrate: A stock solution of 20μM FAM substrate was diluted to 200nM with PDE assay buffer. 12.5μL of the diluted substrate was added to each reaction well.

Compounds: A compound to be tested was first dissolved in DMSO into a lOmM stock solution. 5μL of the compound stock solution was added to 45μL of DMSO to prepare a 1mM diluent. 5μL of the 1mM diluent was added to 45μL of PDE assay buffer to prepare a gradient dilution starting point. Then, according to the method of adding 5μL of the previous concentration solution to 15μL of PDE assay buffer, gradient dilution was carried out for 9 times to prepare compound working solutions with 10 concentrations. The working solutions were added to compound wells at 2.5μL/well.

PDE4D3: 0.054μL of a PDE4D3 recombinase stock solution was added to 1500μL of PDE buffer, and the mixture was added to all compound wells and positive control wells at 10μL/well. 10μL of PDE assay buffer was added to substrate control wells.

Binding solution: 3750μL of binding buffer A and 3750μL of binding buffer B were mixed well. Then, 150μL of a binder was added into the mixture and mixed well. 7.5μL of a Tb donor was added and mixed well. The obtained mixture was added to all wells at 50μL/well.

| | | | Sample | Substrate | Tb |
|---|---|---|---|---|---|
| Enzym e reactio n step | Substrate | 12.5μL | 12.5μL | 12.5μL | 25μL of assay buffer |
| | Compound | 2.5μL | 2.5μL of assay buffer | 2.5μL of assay buffer | |
| | PDE4D3 | 10μL | 10μL of assay buffer | 10μL | |
| Assay reactio n step | Binding solution | 50μL | 50μL | 50μL | 50μL |
| | Encapsulation at room temperature for one hour, reading 330nm Excitation, 490nm, 520nm Emission | | | | |

Data processing:

$$FRET=(S_{520}-(Tb_{520}\times S_{490}/Tb_{490}))\times 1000/S_{490}$$

$S_{520}$ = Read value of sample 520nm
$S_{490}$ = Read value of sample 490nm
$Tb_{520}$= Read value of Tb only 520nm
$Tb_{490}$ = Read value of Tb only 490nm

$$\%Inhibition\ rate=(FRET_P-FRET_S)/(FRET_P-FRET_{Sub})\times 100\%$$

$FRET_S$= Sample FRET
$FRET_P$ = Positive control FRET
$FRET_{Sub}$ = Substrate control FRET.

3. Inhibition assay of PDE4A1 enzyme:

Materials and Instruments:

| |
|---|
| PDE4A1 detection kit (BPS, Cat.60340) |
| PDE4A1 recombinase |

(continued)

| |
|---|
| FAM-Cyclic-3', 5'-AMP |
| PDE buffer |
| Binder |
| Binding buffer |
| Binder diluent (cAMP) |
| Black plate |
| Envision 2104 multi-tag reader (PerkinElmer) |

Test conditions:

| | Component | Concentration |
|---|---|---|
| Enzyme reaction step | FAM substrate | 100nM |
| | PDE4A1 | 80 pg/well |
| | Enzyme reaction time | 60min |
| Assay reaction step | Binder | Dilute at 1:100 |

Reagent preparation:

FAM substrate: 25μL of a FAM substrate stock solution was added into 2500μL of PDE assay buffer. 25μL of the diluted substrate was added to each reaction well.

Compounds: A compound to be tested was first dissolved in DMSO into a lOmM stock solution. 5μL of the compound stock solution was added to 45μL of DMSO to prepare a 1mM diluent. 5μL of the 1mM diluent was added to 45μL of PDE assay buffer to prepare a gradient dilution starting point. Then, according to the method of adding 5μL of the previous concentration solution to 15μL of PDE assay buffer, gradient dilution was carried out for 9 times to prepare compound working solutions with 10 concentrations. The working solutions were added to compound wells at 5μL/well.

PDE4A1: First, a PDE4A1 stock solution was diluted by 100 times to a concentration of 4.9ng/μL, 1.8μL of the diluent was added to 2200μL of PDE assay buffer, and the mixture was added to all compound wells and positive control wells at 20μL/well. 20μL of PDE assay buffer was added to substrate control wells.

Binding solution: 95μL of binding buffer was added into 9.5mL of binder diluent, and then mixed well. The obtained mixture was added to all wells at 100μL/well.

| | | Sample | Substrate | Positive |
|---|---|---|---|---|
| Enzyme reaction step | Substrate | 25μL | 25μL | 25μL |
| | Compound | 5μL of assay buffer | 5μL of assay buffer | 5μL of assay buffer |
| | PDE4A1 | 20μL of assay buffer | 20μL of assay buffer | 20μL |
| | Encapsulation at room temperature for one hour | | | |
| Assay reaction step | Binding solution | 100μL | 100μL | 100μL |
| | Encapsulation at room temperature for 30min, reading FP Excitation, 490nm, 520nm Emission | | | |

Data processing:

$$\text{Inhibition rate}=(FP_P-FP_S)/(FP_P-FP_{Sub})\times 100\%$$

$FP_S$ = Sample FP
$FP_P$ = Positive control FP
$FP_{Sub}$= Substrate control FP.

4. Inhibition assay of PDE4B2 enzyme:

Materials and Instruments:

| |
|---|
| PDE4B2 detection kit (BPS, Cat.60343) |
| PDE4B2 recombinase |
| FAM-Cyclic-3', 5'-AMP |
| PDE buffer |
| Binder |
| Binding buffer |
| Binder diluent (cAMP) |
| Black plate |
| Envision 2104 multi-tag reader (PerkinElmer) |

Test conditions:

| | Component | Concentration |
|---|---|---|
| Enzyme reaction step | FAM substrate | 100nM |
| | PDE4B2 | 150pg/well |
| | Enzyme reaction time | 60min |
| Assay reaction step | Binder | Dilute at 1:100 |

Reagent preparation:

FAM substrate: 25μL of a FAM substrate stock solution was added into 2500μL of PDE assay buffer. 25μL of the diluted substrate was added to each reaction well.
Compounds: A compound to be tested was first dissolved in DMSO into a lOmM stock solution. 5μL of the compound stock solution was added to 45μL of DMSO to prepare a 1mM diluent. 5μL of the 1mM diluent was added to 45μL of PDE assay buffer to prepare a gradient dilution starting point. Then, according to the method of adding 5μL of the previous concentration solution to 15μL of PDE assay buffer, gradient dilution was carried out for 9 times to prepare compound working solutions with 10 concentrations. The working solutions were added to compound wells at 5μL/well.
PDE4B2: First, a PDE4B2 stock solution was diluted by 100 times to a concentration of 5.2ng/μL, 3.2μL of the diluent was added to 2200μL of PDE assay buffer, and the mixture was added to all compound wells and positive control wells at 20μL/well. 20μL of PDE assay buffer was added to substrate control wells.
Binding solution: 95μL of binder was added into 9.5mL of binder diluent, and then mixed well. The obtained mixture was added to all wells at 100μL/well.

| | | Sample | Substrate | Positive |
|---|---|---|---|---|
| Enzym e reaction step | Substrate | 25μL | 25μL | 25μL |
| | Compound | 5μL of assay buffer | 5μL of assay buffer | 5μL of assay buffer |
| | PDE4B2 | 20μL of assay buffer | 20μL of assay buffer | 20μL |
| | Encapsulation at room temperature for one hour | | | |
| Assay reaction step | Binding solution | 100μL | 100μL | 100μL |
| | Encapsulation at room temperature for one hour, reading FP Excitation, 490nm, 520nm Emission | | | |

Data processing:

$$\%\text{Inhibition rate}=(FP_P-FP_S)/(FP_P-FP_{Sub})\times 100\%$$

$FP_S$= Sample FP
$FP_P$ = Positive control FP
$FP_{Sub}$ = Substrate control FP.

5. Inhibition assay of PDE4C1 enzyme:

Materials and Instruments:

| |
|---|
| PDE4C1 detection kit (BPS, Cat.60384) |
| PDE4C1 recombinase |
| FAM-Cyclic-3', 5'-AMP |
| PDE buffer |
| Binder |
| Binding buffer |
| Binder diluent (cAMP) |
| Black plate |
| Envision 2104 multi-tag reader (PerkinElmer) |

Test conditions:

| | Component | Concentration |
|---|---|---|
| Enzyme reaction step | FAM substrate | 100nM |
| | PDE4C1 | 400 pg/well |
| | Enzyme reaction time | 60min |
| Assay reaction step | Binder | Dilute at 1:100 |

Reagent preparation:

FAM substrate: 12.5μL of a FAM substrate stock solution was added into 1250μL of PDE assay buffer. 12.5μL of the diluted substrate was added to each reaction well.

Compounds: A compound to be tested was first dissolved in DMSO into a lOmM stock solution. 5μL of the compound stock solution was added to 45μL of DMSO to prepare a 1mM diluent. 5μL of the 1mM diluent was added to 45μL of PDE assay buffer to prepare a gradient dilution starting point. Then, according to the method of adding 5μL of the previous concentration solution to 15μL of PDE assay buffer, gradient dilution was carried out for 9 times to prepare compound working solutions with 10 concentrations. The working solutions were added to compound wells at 2.5μL/well.

PDE4C1: First, a PDE4C1 stock solution was diluted by 100 times to a concentration of 3.2ng/μL, 13.75μL of the diluent was added to 1100μL of PDE assay buffer, and the mixture was added to all compound wells and positive control wells at 10μL/well. 10μL of PDE assay buffer was added to substrate control wells.

Binding solution: 50μL of binder was added into SmL of binder diluent, and then mixed well. The obtained mixture was added to all wells at 50μL/well.

| | | Sample | Substrate | Positive |
|---|---|---|---|---|
| Enzyme reaction step | Substrate | 12.5μL | 12.5μL | 12.5μL |
| | Compound | 2.5 μL of assay buffer | 2.5 μL of assay buffer | 2.5μL of assay buffer |
| | PDE4C1 | 10μL of assay buffer | 10μL of assay buffer | 10μL |
| | Encapsulation at room temperature for one hour | | | |
| Assay reaction step | Binding solution | 50μL | 50μL | 50μL |
| | Encapsulation at room temperature for 20min, reading FP Excitation, 490nm, 520nm Emission | | | |

Data processing:

$$\%\text{Inhibition rate}=(FP_P-FP_S)/(FP_P-FP_{Sub})\times 100\%$$

$FP_s$= Sample FP
$FP_P$ = Positive control FP
$FP_{Sub}$ = Substrate control FP.

6. The experimental results show that the compound in the examples according to the present disclosure has an inhibitory effect on PDE4, with representative compound examples as follows:
$IC_{50}$ table of PDE4D3:

| Compound No. | PDE4D3 (nM) |
|---|---|
| Example 0 | B |
| Example 1 | C |
| Example 2 | B |
| Example 3 | D |
| Example 4 | B |
| Example 5 | B |
| Example 6 | C |

(continued)

| Compound No. | PDE4D3 (nM) |
|---|---|
| Example 7 | B |
| Example 8 | C |
| Example 9 | A+ |
| Example 10 | C |
| Example 11 | D |
| Example 12 | D |
| Example 13 | B |
| Example 14 | D |
| Example 15 | D |
| Example 16 | D |
| Example 17 | D |
| Example 18 | C |
| Example 19 | D |
| Example 20 | C |
| Example 21 | D |
| Example 22 | D |
| Example 23 | C |
| Example 24 | B |
| Example 25 | B |
| Example 26 | B |
| Example 27 | B |
| Example 28 | A |
| Example 29 | A |
| Example 30 | C |
| Example 31 | C |
| Example 32 | A+ |
| Example 33 | A+ |
| Example 55 | A+ |
| Example 56 | B |

The above biological activities are A+<5nM; A=5-10nM; B=10-50nM; C=50-200nM; D>200nM. The embodiments of the present disclosure have been described, but the present disclosure is not limited to the aforementioned embodiments. Any modification, equivalent replacement, improvement, and the like made within the spirit and principles of the present disclosure shall be included within the scope of protection of the present disclosure.

Note: Example 0 involved in the present disclosure represents the control drug Apremilast. $IC_{50}$ table of PDE4A1:

| Compound No. | PDE4A1 (nM) |
|---|---|
| Example 0 | B |
| Example 5 | A |
| Example 9 | A |

(continued)

| Compound No. | PDE4A1 (nM) |
|---|---|
| Example 13 | B |
| Example 32 | A |
| Example 33 | A |
| Example 55 | A |
| Example 56 | A |

The above biological activities are A<100nM; B=100-200nM. The embodiments of the present disclosure have been described, but the present disclosure is not limited to the aforementioned embodiments. Any modification, equivalent replacement, improvement, and the like made within the spirit and principles of the present disclosure shall be included within the scope of protection of the present disclosure.

Note: Example 0 involved in the present disclosure represents the control drug Apremilast. $IC_{50}$ table of PDE4B2:

| Compound No. | PDE4B2 (nM) |
|---|---|
| Example 0 | B |
| Example 5 | A |
| Example 9 | A |
| Example 13 | B |
| Example 32 | A |
| Example 33 | A |
| Example 55 | A |
| Example 56 | A |

The above biological activities are A<100nM; B=100-200nM. The embodiments of the present disclosure have been described, but the present disclosure is not limited to the aforementioned embodiments. Any modification, equivalent replacement, improvement, and the like made within the spirit and principles of the present disclosure shall be included within the scope of protection of the present disclosure.

Note: Example 0 involved in the present disclosure represents the control drug Apremilast. $IC_{50}$ table of PDE4C1:

| Compound No. | PDE4C1 (nM) |
|---|---|
| Example 0 | B |
| Example 5 | B |
| Example 9 | A |
| Example 13 | B |
| Example 32 | A |
| Example 33 | A |
| Example 55 | A |
| Example 56 | B |

The above biological activities are A<200nM; B=200-500nM. The embodiments of the present disclosure have been described, but the present disclosure is not limited to the aforementioned embodiments. Any modification, equivalent replacement, improvement, and the like made within the spirit and principles of the present disclosure shall be included within the scope of protection of the present disclosure.

Note: Example 0 involved in the present disclosure represents the control drug Apremilast.

7. Selective inhibitory activity assay for PDE1, 2, 3, 5, 7, 10 and 11 enzymes

The selective specificity of compounds for PDE4 was assessed by assaying single concentrations of the compounds. For example, PDE1a enzyme, PDE1c enzyme, PDE2a enzyme, PDE3a enzyme, PDE3b enzyme, PDE5a1 enzyme, PDE7a enzyme, PDE7b enzyme, PDE10a1 enzyme and PDE11a4 enzyme were tested. The selective inhibitory effects of different compounds on the enzymatic activities of PDE1C, PDE2A, PDE3B, PDE5A1, PDE7A, and PDE10A1 at concentrations of 10μM and 1μM, respectively, are shown in the table below: Inhibition rate (%) of PDE1C

| Compound | 1μM | 10μM |
|---|---|---|
| Example 0 | 7.77 | 37.15 |
| Example 5 | 8.26 | 30.84 |
| Example 6 | 0 | 17.97 |
| Example 7 | 6.80 | 26.47 |
| Example 9 | 0.49 | 30.60 |
| Example 13 | 5.34 | 51.72 |
| Example 24 | 3.89 | 34.48 |
| Example 25 | 3.89 | 33.75 |
| Example 27 | 12.14 | 67.53 |
| Example 28 | 1.46 | 24.77 |
| Example 29 | 4.13 | 25.98 |

Inhibition rate (%) of PDE2A

| Compound | 1μM | 10μM |
|---|---|---|
| Example 0 | 2.95 | 2.35 |
| Example 5 | 0.83 | 9.01 |
| Example 6 | 0 | 3.41 |
| Example 7 | 0.08 | 2.65 |
| Example 9 | 5.83 | 12.18 |
| Example 13 | 0.08 | 13.85 |
| Example 24 | 0 | 7.79 |
| Example 25 | 20.36 | 13.39 |
| Example 27 | 0 | 8.10 |
| Example 28 | 0 | 5.83 |
| Example 29 | 7.19 | 15.82 |

Inhibition rate (%) of PDE3B

| Compound | 1μM | 10μM |
|---|---|---|
| Example 0 | 2.44 | 1.56 |
| Example 5 | 0.06 | -0.69 |

(continued)

| Compound | 1μM | 10μM |
|---|---|---|
| Example 6 | 0.94 | -0.44 |
| Example 7 | 1.81 | 0.06 |
| Example 9 | 5.44 | 0.81 |
| Example 13 | 3.31 | 0.94 |
| Example 24 | 0 | -0.81 |
| Example 25 | 0 | 0.19 |
| Example 27 | 0 | -1.31 |
| Example 28 | 3.69 | 6.44 |
| Example 29 | 0 | -1.94 |

Inhibition rate (%) of PDE5A1

| Compound | 1μM | 10μM |
|---|---|---|
| Example 0 | 3.97 | 2.65 |
| Example 5 | 0.66 | 8.39 |
| Example 6 | 34.22 | 34.00 |
| Example 7 | 0 | -2.87 |
| Example 9 | 1.99 | 5.74 |
| Example 13 | 14.13 | 20.31 |
| Example 24 | 0 | 11.48 |
| Example 25 | 0 | 8.17 |
| Example 27 | 0 | 8.14 |
| Example 28 | 0 | 4.16 |
| Example 29 | 5.72 | 12.99 |

Inhibition rate (%) of PDE7A

| Compound | 1μM | 10μM |
|---|---|---|
| Example 0 | 9.41 | 11.07 |
| Example 5 | 0 | 1.94 |
| Example 6 | 0 | 13.83 |
| Example 7 | 3.87 | 15.49 |
| Example 9 | 20.75 | 44.54 |
| Example 13 | 0 | 9.96 |
| Example 24 | 0 | 4.15 |
| Example 25 | 4.98 | 24.62 |

(continued)

| Compound | 1μM | 10μM |
|---|---|---|
| Example 27 | 2.21 | 14.66 |
| Example 28 | 0.55 | 10.51 |
| Example 29 | 17.43 | 19.92 |

Inhibition rate (%) of PDE10A1

| Compound | 1μM | 10μM |
|---|---|---|
| Example 0 | 0 | -2.75 |
| Example 5 | 2.21 | 5.46 |
| Example 6 | 4.84 | -16.18 |
| Example 7 | 0 | 1.30 |
| Example 9 | 0 | 10.09 |
| Example 13 | 16.31 | -26.73 |
| Example 24 | 6.04 | 11.44 |
| Example 25 | 3.02 | 11.10 |
| Example 27 | 11.86 | 1.01 |
| Example 28 | 29.06 | 0.04 |
| Example 29 | 0 | 16.13 |

Note: Example 0 involved in the present disclosure represents the control drug Apremilast.

8. Inhibitory assay of inflammatory factors TNF-α, IL-2, INF-γ

Determination of TNF-α, IL-2 and INF-γ induced by LPS/SEB in human peripheral blood mononuclear cells:

1. The purchased PBMC frozen cells were thawed at 37°C, transferred to RMPI1640 medium, and incubated overnight at 37°C in an incubator containing 5% of $CO_2$.
2. The next day, the cells were planked at 2×105 cells/well, with 100μL per well.
3. Triple-series dilution was performed on compounds to be tested. The final drug concentrations were 3000, 1000, 333.33, 111.11, 37.04, 12.35, 4.12, 1.37, 0.46nM.
4. LPS stimulus was added to each well at a final concentration of 10ng/ml.
5. Incubation was carried out overnight at 7°C in an incubator containing 5% of $CO_2$.
6. On the third day, the cell culture supernatant was collected, and cytokine assay was performed using an MSD method.

The experimental results show that the compounds in the examples according to the present disclosure have inhibitory effects on inflammatory factors such as TNF-α, IL-2, and IFN-γ. Examples of representative compounds are as follows:

| Cytokine | Stimulus | $IC_{50}$ (nM) for Example 0 | IC50 (nM) for Example 9 | IC50 (nM) for Roflumilast |
|---|---|---|---|---|
| TNF-α | LPS | 63.8 | 0.82 | 2.83 |

(continued)

| Cytokine | Stimulus | $IC_{50}$ (nM) for Example 0 | IC50 (nM) for Example 9 | IC50 (nM) for Roflumilast |
|---|---|---|---|---|
| IL-2 | SEB | 109.9 | 0.44 | 14.7 |
| INF-r | LPS | 15.4 | 0.43 | 1.4 |
| INF-r | SEB | 77.8 | 1.82 | 10.9 |

The compounds provided by the present disclosure significantly increase the activity of inhibiting the expression of inflammation-related factors.

Note: Example 0 involved in the present disclosure represents the control drug Apremilast.

The embodiments of the present disclosure have been described above. However, the present disclosure is not limited to the aforementioned embodiments. Any modification, equivalent replacement, improvement, and the like made within the spirit and principles of the present disclosure shall be included within the scope of protection of the present disclosure. In particular, optical enantiomers, diastereomers, and stereoisomers of the compounds provided by the present disclosure, as well as mixtures thereof are all within the scope of protection of the present disclosure.

## Claims

1. A compound as shown in formula I and its racemates, stereoisomers, tautomers, isotopic labels, solvates, polymorphs, esters, prodrugs or pharmaceutically acceptable salts:

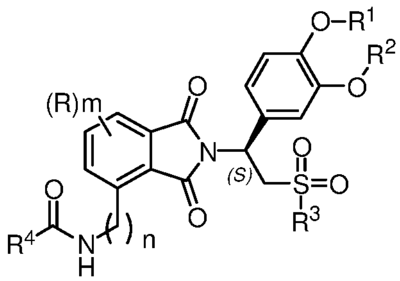

**I**

wherein each R may independently be H, deuterium, halogen, amino, hydroxyl, cyano, nitro, and the following groups unsubstituted or optionally substituted with one, two or more Ra: hydrocarbyl groups of C1-C16, heteroalkyl groups of C1-C16, cycloalkyl groups of C3-C12, $R'SO_2NH$-, alkyl- of $R'SO_2NH$-C1-C16, alkyl- of $R'SO_2$-C1-C16, $R'SO_2$-, 3-12 membered heterocyclic groups, aryl groups of C6-C14, and 5-14 membered arylheteroaryl groups; or cyclics may be formed between two Rs at different positions independently;

Ra is independently selected from deuterium, halogen, amino, hydroxyl, cyano, nitro, oxo (=O), and the following groups unsubstituted or optionally substituted with one, two or more Rb: hydrocarbyl groups of C1-C16, heteroalkyl groups of C1-C16, cycloalkyl groups of C3-C12, 3-12 membered heterocyclic groups, aryl groups of C6-C14, and 5-14 membered arylheteroaryl groups;

R' is independently selected from the following groups unsubstituted or optionally substituted with one, two or more Rb: hydrocarbyl groups of C1-C16, heteroalkyl groups of C 1-C16, cycloalkyl groups of C3-C12, 3-12 membered heterocyclic groups, aryl groups of C6-C14, and 5-14 membered arylheteroaryl groups;

Rb is selected from deuterium, halogen, amino, hydroxyl, cyano, nitro, oxo (=O), as well as hydrocarbyl groups of C1-C16, heteroalkyl groups of C1-C16, cycloalkyl groups of C3-C12, 3-12 membered heterocyclic groups, aryl groups of C6-C14, and 5-14 membered arylheteroaryl groups;

m is 1, 2, or 3; n is 0 or 1;

$R^1$ refers to the following groups unsubstituted or optionally substituted with one, two or more R1a: hydrocarbyl groups of C1-C16, heteroalkyl groups of C1-C16, and cycloalkyl groups of C3-C12;

$R^2$ refers to the following groups unsubstituted or optionally substituted with one, two or more R2a: hydrocarbyl groups of C1-C16, heteroalkyl groups of C1-C16, and cycloalkyl groups of C3-C12;

alternatively, R1 and R2 may form cyclics;

$R^3$ refers to the following groups unsubstituted or optionally substituted with one, two or more R3a: hydrocarbyl groups of C1-C16, heteroalkyl groups of C1-C16, and cycloalkyl groups of C3-C12;
$R^4$ refers to the following groups unsubstituted or optionally substituted with one, two or more R4a: hydrocarbyl groups of C1-C16, heteroalkyl groups of C1-C16, and cycloalkyl groups of C3-C12;
R1a is independently selected from deuterium, halogen, amino, hydroxyl, cyano, nitro, oxo (=O), and the following groups unsubstituted or optionally substituted with one, two or more R1b: hydrocarbyl groups of C1-C16, heteroalkyl groups of C1-C16, and cycloalkyl groups of C3-C12;
R2a is independently selected from deuterium, halogen, amino, hydroxyl, cyano, nitro, oxo (=O), and the following groups unsubstituted or optionally substituted with one, two or more R2b: hydrocarbyl groups of C1-C16, heteroalkyl groups of C1-C16, and cycloalkyl groups of C3-C12;
R3a is independently selected from deuterium, halogen, amino, hydroxyl, cyano, nitro, oxo (=O), and the following groups unsubstituted or optionally substituted with one, two or more R3b: hydrocarbyl groups of C1-C16, heteroalkyl groups of C1-C16, and cycloalkyl groups of C3-C12;
R4a is independently selected from deuterium, halogen, amino, hydroxyl, cyano, nitro, oxo (=O), and the following groups unsubstituted or optionally substituted with one, two or more R4b: hydrocarbyl groups of C1-C16, heteroalkyl groups of C1-C16, and cycloalkyl groups of C3-C12;
R1b is selected from deuterium, halogen, amino, hydroxyl, cyano, nitro, oxo (=O), as well as hydrocarbyl groups of C1-C16, and heteroalkyl groups of C1-C16;
R2b is selected from deuterium, halogen, amino, hydroxyl, cyano, nitro, oxo (=O), as well as hydrocarbyl groups of C1-C16, and heteroalkyl groups of C1-C16;
R3b is selected from deuterium, halogen, amino, hydroxyl, cyano, nitro, oxo (=O), as well as hydrocarbyl groups of C1-C16, and heteroalkyl groups of C1-C16; and
R4b is selected from deuterium, halogen, amino, hydroxyl, cyano, nitro, oxo (=O), as well as hydrocarbyl groups of C1-C16, and heteroalkyl groups of C1-C16.

**2.** The compound as shown in formula I and its racemates, stereoisomers, tautomers, isotopic labels, solvates, polymorphs, esters, prodrugs or pharmaceutically acceptable salts according to claim 1, wherein the compound as shown in formula I is further selected from the following formula II:

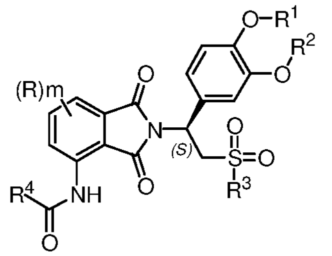

II

in formula II, the R, $R^1$, $R^2$, $R^3$, $R^4$, and m are as defined in claim 1.

**3.** The compound as shown in formula I and its racemates, stereoisomers, tautomers, isotopic labels, solvates, polymorphs, esters, prodrugs or pharmaceutically acceptable salts according to claim 1 or 2, wherein the compound as shown in formula I is further selected from the following formula III:

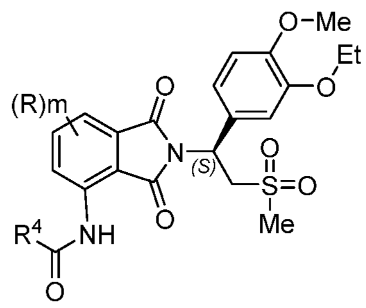

III

in formula III, the R, $R^4$, and m are as defined in claim 1 or 2.

4. The compound as shown in formula I and its racemates, stereoisomers, tautomers, isotopic labels, solvates, polymorphs, esters, prodrugs or pharmaceutically acceptable salts according to claim 1 or 2, wherein the compound as shown in formula I is further selected from the following formula IV:

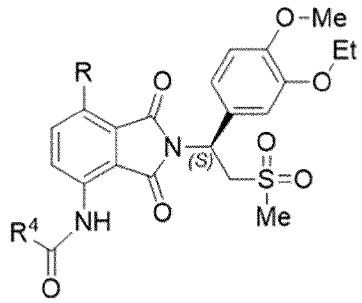

Formula IV

in formula IV, the R, $R^4$, and m are as defined in claim 1 or 2.

5. A pharmaceutical composition, which contains the compound as shown in formula I and its racemates, stereoisomers, tautomers, isotopic labels, solvates, polymorphs, esters, prodrugs or pharmaceutically acceptable salts according to any one of claims 1 to 4; and
preferably, the pharmaceutical composition contains a therapeutically effective amount of the compound as shown in formula I and its racemates, stereoisomers, tautomers, isotopic labels, nitrogen oxides, solvates, polymorphs, metabolites, esters, prodrugs or pharmaceutically acceptable salts and pharmaceutically acceptable carriers.

6. The use of the compound as shown in formula I and its racemates, stereoisomers, tautomers, isotopic labels, solvates, polymorphs, esters, prodrugs or pharmaceutically acceptable salts according to any one of claims 1 to 4, or the pharmaceutical composition in the preparation of medicines for inhibiting PDE4 enzyme; and
preferably, the conditions ameliorated by by inhibiting the PDE4 with the medicines for inhibiting the PDE4 include, but are not limited to, asthma, inflammation (e.g., inflammation caused by reperfusion), chronic or acute obstructive pulmonary disease, chronic or acute pneumonia, enteritis, segmental ileitis, psoriasis, seborrheic dermatitis, stasis dermatitis, palmoplantar abscess, psoriatic arthritis, Behcet's disease or colitis.

7. The use of the compound as shown in formula I and its racemates, stereoisomers, tautomers, isotopic labels, solvates, polymorphs, esters, prodrugs or pharmaceutically acceptable salts according to any one of claims 1 to 4, or the pharmaceutical composition in the preparation of medicines for treating diseases related to the regulation of intracellular cAMP level.

8. The use of the compound as shown in formula I and its racemates, stereoisomers, tautomers, isotopic labels, solvates, polymorphs, esters, prodrugs or pharmaceutically acceptable salts according to any one of claims 1 to 4, or the pharmaceutical composition in the preparation of medicines for inhibiting the production of TNF-$\alpha$ or NF-$\kappa$B.

9. The use of the compound as shown in formula I and its racemates, stereoisomers, tautomers, isotopic labels, solvates, polymorphs, esters, prodrugs or pharmaceutically acceptable salts according to any one of claims 1 to 4, or the pharmaceutical composition in the preparation of medicines for the treatment of diseases and conditions selected from the group consisting: depression, asthma, inflammation (e.g., contact dermatitis, atopic dermatitis, seborrheic dermatitis, stasis dermatitis, palmoplantar abscess, psoriasis, rheumatoid arthritis, psoriatic arthritis, osteoarthritis, inflammatory dermatosis, and inflammation caused by reperfusion), chronic or acute obstructive pulmonary disease, chronic or acute pneumonia, viral pneumonia, enteritis, segmental ileitis, Behcet's disease or colitis.

10. A method for controlling intracellular cAMP level, comprising the following steps:
contacting an effective amount of the compound according to any one of claims 1 to 4, or a pharmaceutically acceptable prodrug, polymorph, salt, solvate, hydrate or clathrate thereof with cells.

## INTERNATIONAL SEARCH REPORT

International application No.
**PCT/CN2022/088776**

**A. CLASSIFICATION OF SUBJECT MATTER**

C07D 209/48(2006.01)i； C07D 209/49(2006.01)i； A61P 11/06(2006.01)i； A61P 29/00(2006.01)i； A61P 11/00(2006.01)i； A61P 17/00(2006.01)i； A61P 17/08(2006.01)i； A61P 17/06(2006.01)i； A61P 19/02(2006.01)i； A61P 1/00(2006.01)i； A61K 31/4035(2006.01)i

According to International Patent Classification (IPC) or to both national classification and IPC

**B. FIELDS SEARCHED**

Minimum documentation searched (classification system followed by classification symbols)

C07D A61P A61K

Documentation searched other than minimum documentation to the extent that such documents are included in the fields searched

Electronic data base consulted during the international search (name of data base and, where practicable, search terms used)

CNABS; CNTXT; CJFD; DWPI; SIPOABS; WOTXT; USTXT; EPTXT; CNKI; PATENTICS; 万方; WANFANG; 超星读秀; CHAOXING DUXIU; ISI-Web of Science; STN-registry; STN-caplus: 苏州璞正医药, 朱加望, 姚瑶, 异吲哚啉, 二酮, 环腺苷酸, 环核苷酸磷酸二酯酶, 磷酸二酯酶, 肿瘤坏死因子, 炎症, 抑郁症, 哮喘, Isoindoline, + dione, Camp, PDE, TNF, Inflammation, Asthma, Depression, 结构式I, structural formula I

**C. DOCUMENTS CONSIDERED TO BE RELEVANT**

| Category* | Citation of document, with indication, where appropriate, of the relevant passages | Relevant to claim No. |
|---|---|---|
| PX | CN 113527179 A (INTRAGRAND PHARMA (SUZHOU) CO., LTD.) 22 October 2021 (2021-10-22)<br>claims 1-9, and description, embodiments 1-54, compounds | 1-9 |
| X | WO 2018157779 A1 (KANGPU BIOPHARMACEUTICALS LTD.) 07 September 2018 (2018-09-07)<br>claims 1, 15, 18, and 20, and description, p. 13, lines 13-21 | 1-9 |
| X | AU 2006200033 A1 (CELGENE CORP.) 02 February 2006 (2006-02-02)<br>claims 2, 43, and 53-59 | 1-9 |
| X | CN 111514135 A (HUANG YONGHUA) 11 August 2020 (2020-08-11)<br>claims 1, 3, 9, and 10 | 1-6, 9 |

☑ Further documents are listed in the continuation of Box C. ☑ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **16 June 2022** | **05 July 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2022/088776**

**C. DOCUMENTS CONSIDERED TO BE RELEVANT**

| Category* | Citation of document, with indication, where appropriate, of the relevant passages | Relevant to claim No. |
|---|---|---|
| X | LU, Yuting et al. "Identification and Characterization of Process-Related Substances and Degradation Products in Apremilast: Process Optimization and Degradation Pathway Elucidation" *Journal of Pharmaceutical and Biomedical Analysis,* Vol. 141, 02 April 2017 (2017-04-02), ISSN: 1873-264X, p. 76, the left column, lines 1-7 | 1-4 |
| X | FRIIS, S. D. et al. "Cobalt-Catalysed C–H Methylation for Late-Stage Drug Diversification" *Nature Chemistry,* Vol. 12, No. 6, 29 May 2020 (2020-05-29), ISSN: 1755-4349, p. 517, figure 4b | 1-3 |
| X | WO 2021119571 A1 (BIOTHERYX INC.) 17 June 2021 (2021-06-17) description, paragraphs [0267] and [0279] | 1-3 |
| X | CN 110898056 A (HUANG YONGHUA) 24 March 2020 (2020-03-24) claim 1 | 1-5 |
| X | BHOLE, R. P. et al. "A Stability Indicating HPTLC Method for Apremilast and Identification of Degradation Products Using MS/MS" *Journal of Pharmaceutical Sciences and Research,* Vol. 11, No. 5, 31 May 2019 (2019-05-31), ISSN: 0975-1459, p. 1866, compounds DP-6 and DP-7 in figures 13 and 14 | 1-4 |
| A | CN 1802353 A (CELGENE CORP.) 12 July 2006 (2006-07-12) entire document | 1-9 |

**INTERNATIONAL SEARCH REPORT**

International application No.
**PCT/CN2022/088776**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **10**
because they relate to subject matter not required to be searched by this Authority, namely:

[1] Claim 10 sets forth a method for controlling an intracellular cAMP level. The method relates to a method for treating a human or animal body, belonging to the cases listed in PCT Rule 39.1(iv) for which no international search is required.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2022/088776**

| Patent document cited in search report | Publication date (day/month/year) | Patent family member(s) | Publication date (day/month/year) |
|---|---|---|---|
| CN 113527179 A | 22 October 2021 | None | |
| WO 2018157779 A1 | 07 September 2018 | CA 3052516 A1 | 07 September 2018 |
| | | AU 2018228541 A1 | 05 September 2019 |
| | | CN 110291065 A | 27 September 2019 |
| | | KR 20190122678 A | 30 October 2019 |
| | | EP 3590924 A1 | 08 January 2020 |
| | | EP 3590924 A4 | 19 February 2020 |
| | | US 2020061033 A1 | 27 February 2020 |
| | | JP 2020509017 A | 26 March 2020 |
| | | HK 40008858 A0 | 19 June 2020 |
| | | AU 2018228541 B2 | 16 July 2020 |
| | | RU 2728829 C1 | 31 July 2020 |
| | | ZA 201905098 A | 17 December 2020 |
| | | JP 6942380 B2 | 29 September 2021 |
| | | EP 3590924 B1 | 27 October 2021 |
| | | KR 102318401 B1 | 29 October 2021 |
| | | NZ 756231 A2 | 24 December 2021 |
| AU 2006200033 A1 | 02 February 2006 | US 6011050 A | 04 January 2000 |
| | | AU 1447200 A | 22 May 2000 |
| | | NO 20012021 A | 26 June 2001 |
| | | BR PI9915201 A | 30 October 2001 |
| | | AU 756308 B2 | 09 January 2003 |
| | | NZ 511253 A | 28 February 2003 |
| | | AU 2003203681 A1 | 03 July 2003 |
| | | NO 319790 B1 | 12 September 2005 |
| | | EP 1752148 A2 | 14 February 2007 |
| | | DE 69934708 D1 | 15 February 2007 |
| | | EP 1752148 A3 | 14 March 2007 |
| | | ES 2278467 T3 | 01 August 2007 |
| | | DE 69934708 T2 | 18 October 2007 |
| | | AU 2006200033 B2 | 14 August 2008 |
| | | AU 2006200033 B8 | 11 September 2008 |
| | | CA 2348993 C | 29 December 2009 |
| | | EP 1752148 B1 | 23 June 2010 |
| | | DE 69942532 D1 | 05 August 2010 |
| | | ES 2343744 T3 | 09 August 2010 |
| | | EP 2305248 A1 | 06 April 2011 |
| | | BR PI9915201 B1 | 22 February 2012 |
| | | EP 2305248 B1 | 08 May 2013 |
| | | ES 2421153 T3 | 29 August 2013 |
| | | AU 2003203681 B2 | 06 October 2005 |
| CN 111514135 A | 11 August 2020 | None | |
| WO 2021119571 A1 | 17 June 2021 | TW 202136273 A | 01 October 2021 |
| CN 110898056 A | 24 March 2020 | None | |
| CN 1802353 A | 12 July 2006 | WO 2004060313 A2 | 22 July 2004 |
| | | AU 2003303511 A1 | 29 July 2004 |
| | | TW 200418779 A | 01 October 2004 |
| | | US 2004204448 A1 | 14 October 2004 |
| | | MX 2005006998 A1 | 01 September 2005 |

Form PCT/ISA/210 (patent family annex) (January 2015)

**INTERNATIONAL SEARCH REPORT**
**Information on patent family members**

International application No.
**PCT/CN2022/088776**

| Patent document cited in search report | Publication date (day/month/year) | Patent family member(s) | | | Publication date (day/month/year) |
|---|---|---|---|---|---|
| | | KR | 20050090435 | A | 13 September 2005 |
| | | EP | 1587474 | A3 | 15 September 2005 |
| | | WO | 2004060313 | A3 | 15 September 2005 |
| | | EP | 1587474 | A2 | 26 October 2005 |
| | | BR | 0317885 | A | 06 December 2005 |
| | | JP | 2006515310 | A | 25 May 2006 |
| | | ZA | 200505308 | A | 25 October 2006 |
| | | EP | 1587474 | A4 | 22 November 2006 |
| | | US | 7173058 | B2 | 06 February 2007 |
| | | US | 2007072902 | A1 | 29 March 2007 |
| | | MX | 257566 | B | 02 June 2008 |
| | | NZ | 541487 | A | 28 November 2008 |
| | | US | 7504427 | B2 | 17 March 2009 |
| | | AU | 2003303511 | B2 | 04 June 2009 |
| | | EP | 1587474 | B1 | 18 November 2009 |
| | | DE | 60330187 | D1 | 31 December 2009 |
| | | ES | 2333220 | T3 | 18 February 2010 |
| | | US | 7893102 | B2 | 22 February 2011 |
| | | US | 2011124645 | A1 | 26 May 2011 |
| | | US | 8158672 | B2 | 17 April 2012 |
| | | CA | 2511843 | C | 24 April 2012 |
| | | JP | 2013151497 | A | 08 August 2013 |
| | | JP | 5269281 | B2 | 21 August 2013 |
| | | US | 2009143382 | A1 | 04 June 2009 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 6020358 A
- US 6962940 B
- WO 0134606 A1
- WO 0025777 A
- WO 2012083153 A
- WO 2018157779 A1
- WO 0134604 A
- WO 2016169533 A
- US 2003187052 A
- CN 1652772
- CN 1965823
- CN 101683334
- CN 03811093


### Non-patent literature cited in the description


- **LOWE ; CHENG.** *Drugs of the Future,* 1992, vol. 17 (9), 799-807
- **BEAVO ; REITSNYDER.** *Trends in Pharm.,* 1990, vol. 11, 150-155
- **VERGHES et al.** *Journal of Pharmacology and Experimental Therapeutics,* 1995, vol. 272 (3), 1313-1320
- **TRACEY et al.** *Nature,* 1987, vol. 330, 662-664
- **HINSHAW et al.** *Circle. Shock,* 1990, vol. 30, 279-292
- **MILLAR et al.** *Lancet,* 1989, vol. 2, 712-714
- **FERRAI-BALIVIERA et al.** *Arch Surg.,* 1989, vol. 124, 1400-1405
- **BERTOLINI et al.** *Nature,* 1986, vol. 319, 516-518
- **PIGNET et al.** *Nature,* 1990, vol. 344, 245-247
- **BISSONNETTE et al.** *Inflammation,* 1989, vol. 13, 329-339
- **BAUGHMAN et al.** *J Lab. Lin. Med.,* 1990, vol. 115, 36-42
- **ELLIOT et al.** *Int. J. Pharmac.,* 1995, vol. 17, 141-145
- **VON DULLEMEN et al.** *Gastroenterology,* 1995, vol. 109, 129-135
- **LOWE.** *Exp. Opin. Ther. Patents,* 1998, vol. 8, 1309-1332
- **JACQUES, J. et al.** Enantiomers, Racemates and Resolutions. Wiley-Interscience, 1981
- **WILEN, S.H. et al.** *Tetrahedron,* 1977, vol. 33, 2725
- **ELIEL, E.L.** Stereochemistry of Carbon Compounds. McGraw-Hill, 1962
- **WILEN, S.H. ; ELIEL E.L.** Tables of Resolving Agents and Optical Resolutions. Notre Dame University Press, 1972, 268
- Remington's Pharmaceutical Sciences. Mack, 1990